# EUROPEAN PATENT APPLICATION

(11) **EP 4 455 266 A1**
(43) Date of publication of application: **30.10.2024**
(21) Application number: 22910094.6
(22) Date of filing: 21.12.2022
(51) Int. Cl.: C12N 5/02, A61K 39/395, C07K 16/30, C07K 16/46, C07H 21/04, G01N 33/574

(54) **FULLY HUMAN ANTIBODY TARGETING GPRC5D AND CHIMERIC ANTIGEN RECEPTOR (CAR) AND USE THEREOF**

(30) Priority: 21.12.2021 WO PCT/CN2021/140153; 15.03.2022 WO PCT/CN2022/080836
(71) Applicant: Shanghai Iaso Biotechnology Co., Ltd., Shanghai 201203 (CN)
(72) Inventor: TAN, Taochao, Shanghai 201203 (CN); LUO, Qian, Shanghai 201203 (CN); DAI, Zhenyu, Shanghai 201203 (CN); WEI, Qiaoe, Shanghai 201203 (CN); ZHAO, Ya, Shanghai 201203 (CN); ZHANG, Yanying, Shanghai 201203 (CN); LIU, Jianwei, Shanghai 201203 (CN); JIA, Xiangyin, Shanghai 201203 (CN); ZHANG, Qianqian, Shanghai 201203 (CN); XIE, Meng, Shanghai 201203 (CN)
(74) Representative: Lavoix
(86) International application number: PCT/CN2022/140769
(87) International publication number: WO 2023/116782

(57) **Abstract**

A fully human antibody targeting GPRC5D and a chimeric antigen receptor (CAR) comprising the fully human antibody. A host cell expressing the CAR, such as a CAR-T cell. A use of the fully human antibody, the CAR, and the CAR-T cell in treatment of tumors (such as multiple myeloma).

## Description

The present application claims the priority to the PCT patent application PCT/CN2021/140153 submitted to the Chinese Patent Office on December 21, 2021, and the PCT patent application PCT/CN2022/080836 submitted to the Chinese Patent Office on March 15, 2022, the entire contents of which are incorporated herein by reference.

### TECHNICAL FIELD

The present application relates to the field of biomedicine, and specifically to a fully humanized antibody and a CAR targeting GPRC5D, and preparation methods and uses thereof.

### BACKGROUND

Multiple myeloma is a plasma cell tumor that occurs in the bone marrow. The tumor may cause hypercalcemia, anemia, renal dysfunction, osteonecrosis, bone marrow failure, etc. Currently, multiple myeloma is the second most common hematological tumor ^{[1]}. As shown in the multiple myeloma incidence statistics released by the World Health Organization (WHO) in 2020, the morbidity and mortality rates in the Asian population accounted for 36% and 42% respectively, ranking first among all continents. The morbidity and mortality ratio of multiple myeloma is 1.8:1.1 in the world, and is 1.1:0.76 in Asia. It can be seen that the survival rate of the population affected with multiple myeloma is relatively low. The main reason is that first, the median age of the population affected is relatively high, around 66 years old, and the incidence rate in the population under 40 years old is around 2%. Second, multiple myeloma is almost incurable with commonly used chemotherapy, immunomodulators, and monoclonal antibody therapies. Even if remission is achieved, it is accompanied by an extremely high relapse rate, i.e., relapsed/refractory multiple myeloma (RRMM), with a five-year survival rate of only 51% ^{[1], [2]}.

In recent years, for the treatment of multiple myeloma, breakthrough progress has been made in T cell bispecific antibody therapy (bispecific T cell engagers (BiTEs)) and adoptive T cell therapy (ACT). The main targets of targeted therapy products for multiple myeloma include BCMA, CD38, CD138, GPRC5D, etc. ^{[2]}. CD38 and CD138 are expressed on cells in normal tissues and hematopoietic stem cells. Elimination of them using targeted therapies has significant side effects and often causes damage to normal organs or impairs the autoimmune system. BCMA and GPRC5D are mainly expressed on plasma cells or plasma cells in myeloma, and plasma cells can be compensated by the continuous regeneration of the body's own B cells. Taking T cell bispecific antibody products as an example, the products targeting BCMA mainly include REGN5458 developed by Regeneron Pharmaceuticals, Inc. (REGN) and Teclistamab developed by Johnson & Johnson ^{[2][3]}. REGN5458 is currently in Phase I clinical trials. The 2022 ASH conference reported that 54 patients received intravenous drug injections, with a response rate of 71.4% and a maximal remission rate of 57.1%; and 51 patients received subcutaneous drug injections, with a response rate of 60.4% and a maximal remission rate of 39.6% ^{[4]}. Teclistamab was on the market in October 2022, with an overall response rate of 61.8%, and 28.2% of patients achieved complete remission or stringent remission ^{[5]}. The representative product of the bispecific antibody targeting GPRC5D is Talqulemab, which has submitted a BLA application for marketing approval in December 2022. The completed Phase 1/2 clinical trial showed that at a dose of 0.4 mg/kg, the overall response rate was 73%, the maximal remission rate was 58%, and the complete remission rate was greater than 29% ^{[6]}. It has entered Phase III clinical trials.

A CAR-T product developed by Bristol-Myers Squibb (BMS) targeting the BCMA target for multiple myeloma, bb2121, was approved by the FDA in May 2021 as the first CAR-T product to be marketed for multiple myeloma. The overall response rate (ORR) of this product was 73%, and 33% of patients achieved complete remission (CR) ^{[7]}. Nanjing Legend's BCMACAR-T, which was on the market in 2022, has improved efficacy compared to BMS. The product has an overall response rate (ORR) of 98%, 80% of patients can achieve complete remission (CR), and the median disease progression-free survival is 22.8 months ^{[8]}. The two-year survival rates of both marketed products are less than 50%. It can be seen that the existing BCMACAR-T treatment products have a higher recurrence rate in the later stages. In addition, since this therapy eliminates all plasma cells, there are still side effects caused by cytopenia and decreased immunoglobulins. GPRC5D expression is more specific relative to BCMA. It is only expressed in plasma cells of myeloma patients and almost not expressed in normal tissues. Only significant RNA and protein expression can be detected in hair follicle tissues ^{[9], [10]}. Further findings showed that there was no correlation between BCMA expression and GPRC5D. Although both were expressed in plasma cells, their expression profiles were relatively independent ^{[11]}. Targeting GPRC5D may be used for treatment of patients with lower BCMA expression and those who have failed BCMA CAR-T therapy.

The main GPRC5D-targeted CAR-T products currently under development include MCARH109 developed by Eureka Corporation, which is in Phase I clinical trials. Data reported at the 2022 ASH Annual Meeting showed that among 17 patients who received GPRC5D CAR-T injection, the overall response rate was 71% (12/17), and the maximal remission rate was 59% (10/17) ^{[12]}. Another CAR-T therapy for GPRC5D was developed by Oricell Therapeutics and has been approved for IND. Therefore, the medical market still needs new GPRC5D CAR-T products with stronger functions and low immunogenicity.

### SUMMARY OF THE INVENTION

In one aspect, the present invention provides a GPRC5D binding peptide, including a heavy chain variable region of an antibody molecule, where HCDR1, HCDR2 and HCDR3 in the heavy chain variable region are selected from one of the following combinations:
1) HCDR1 is GGSFSGYY (SEQ ID NO:1);
   HCDR2 is INHSGST (SEQ ID NO:2);
   the sequence of HCDR3 is ARARRYGGRTRFDP (SEQ ID NO:3);
2) HCDR1 is GFIFSSYG (SEQ ID NO:4);
   the sequence of HCDR2 is ISSSGDYT (SEQ ID NO: 5);
   the sequence of HCDR3 is ARMSFRRYDH (SEQ ID NO:6); and
3) HCDR1 is GFSFSGYI (SEQ ID NO:7);
   the sequence of HCDR2 is TSSSGTET (SEQ ID NO: 8);
   the sequence of HCDR3 is ARYYSKYGRSYHVDS (SEQ ID NO: 9).

In some embodiments, the GPRC5D binding peptide is an antibody molecule or an antigen binding fragment thereof.

In some embodiments, the heavy chain variable region comprises an amino acid sequence as shown in SEQ ID NO: 10, 11 or 12; or the heavy chain variable region comprises an amino acid sequence having at least 90%, 91%, 92%, 93%, 94%, 95%, 96%, 97%, 98%, or 99% sequence identity to the sequence as shown in SEQ ID NO: 10, 11 or 12 and is capable of specifically binding to GPRC5D.

In some embodiments, the GPRC5D binding peptide is a single domain antibody molecule.

In some embodiments, the GPRC5D binding peptide is a fully humanized antibody molecule.

In some embodiments, the ability of the GPRC5D binding peptide to bind to cells expressing GPRC5D on the surface thereof is such that the EC50 value detected by FACS is not higher than 10 nM, 9 nM, 8 nM, 7 nM, 6 nM, or 5 nM.

In another aspect, the present invention provides a fusion protein, comprising the above-mentioned GPRC5D binding peptide.

In some embodiments, the fusion protein comprises at least two GPRC5D binding peptides.

In some embodiments, the HCDR1, HCDR2 and HCDR3 of the two GPRC5D binding peptides are selected from different combinations listed above, respectively.

In some embodiments, in the fusion protein, one of the two GPRC5D binding peptides comprises an amino acid sequence as shown in SEQ ID NO: 10, or comprises an amino acid sequence having at least 90%, 91%, 92%, 93%, 94%, 95%, 96%, 97%, 98%, or 99% sequence identity to the sequence as shown in SEQ ID NO: 10 and is capable of specifically binding to GPRC5D, and the other comprises an amino acid sequence as shown in SEQ ID NO: 11, or comprises an amino acid sequence having at least 90%, 91%, 92%, 93%, 94%, 95%, 96%, 97%, 98%, or 99% sequence identity to the sequence as shown in SEQ ID NO: 11 and is capable of specifically binding to GPRC5D; one of the two GPRC5D binding peptides comprises an amino acid sequence as shown in SEQ ID NO: 10, or comprises an amino acid sequence having at least 90%, 91%, 92%, 93%, 94%, 95%, 96%, 97%, 98%, or 99% sequence identity to the sequence as shown in SEQ ID NO: 10 and is capable of specifically binding to GPRC5D, and the other includes an amino acid sequence as shown in SEQ ID NO:12, or includes an amino acid sequence having at least 90%, 91%, 92%, 93%, 94%, 95%, 96%, 97%, 98%, or 99% sequence identity to the sequence as shown in SEQ ID NO:12 and is capable of specifically binding to GPRC5D; or one of the two GPRC5D binding peptides comprises an amino acid sequence as shown in SEQ ID NO: 11, or comprises an amino acid sequence having at least 90%, 91%, 92%, 93%, 94%, 95%, 96%, 97%, 98%, or 99% sequence identity to the sequence as shown in SEQ ID NO: 11 and is capable of specifically binding to GPRC5D, and the other comprises an amino acid sequence as shown in SEQ ID NO: 12, or comprises an amino acid sequence having at least 90%, 91%, 92%, 93%, 94%, 95%, 96%, 97%, 98%, or 99% sequence identity to the sequence as shown in SEQ ID NO: 12 and is capable of specifically binding to GPRC5D.

In some embodiments, the fusion protein comprises at least three GPRC5D binding peptides, and the HCDR1, HCDR2 and HCDR3 of the three GPRC5D binding peptides are three combinations listed above, respectively.

In some embodiments, the first of the three GPRC5D binding peptides of the fusion protein comprises an amino acid sequence as shown in SEQ ID NO: 10, or comprises an amino acid sequence having at least 90%, 91%, 92%, 93%, 94%, 95%, 96%, 97%, 98%, or 99% sequence identity to the sequence as shown in SEQ ID NO: 10 and is capable of specifically binding to GPRC5D; the second comprises an amino acid sequence as shown in SEQ ID NO: 11, or comprises an amino acid sequence having at least 90%, 91%, 92%, 93%, 94%, 95%, 96%, 97%, 98%, or 99% sequence identity to the sequence as shown in SEQ ID NO: 11 and is capable of specifically binding to GPRC5D; and the third comprises an amino acid sequence as shown in SEQ ID NO: 12, or comprises an amino acid sequence having at least 90%, 91%, 92%, 93%, 94%, 95%, 96%, 97%, 98%, or 99% sequence identity to the sequence as shown in SEQ ID NO: 12 and is capable of specifically binding to GPRC5D.

In some embodiments, the fusion protein further comprises an Fc fragment.

In some embodiments, the fusion protein further comprises another binding peptide capable of specifically binding to an antigen different from GPRC5D.

In some embodiments, the another binding peptide is a single domain antibody or a single chain antibody.

In some embodiments, the fusion protein further includes a detectable tag or a purification tag.

In some embodiments, the detectable tag has enzymatic activity.

In another aspect, the present invention provides a nucleic acid molecule encoding the above-mentioned GPRC5D binding peptide or the above-mentioned fusion protein.

In some embodiments, the nucleic acid molecule comprises a nucleotide sequence as shown in SEQ ID NO: 13, 14 or 15.

In another aspect, the present invention provides an expression vector comprising the above-mentioned nucleic acid molecule.

In another aspect, the present invention provides a host cell comprising the above-mentioned nucleic acid molecule or the above-mentioned expression vector.

In another aspect, the present invention provides a multispecific antibody molecule, including at least a first functional portion and a second functional portion, wherein the first functional portion comprises the above-mentioned GPRC5D binding peptide; and the second functional portion has binding specificity different from that of the first functional portion.

In some embodiments, the second functional portion has binding specificity for an immune cell.

In some embodiments, the second functional portion has binding specificity for T cell.

In some embodiments, the second functional portion has binding specificity for CD3.

In another aspect, the present invention provides an immunoconjugate, comprising the above-mentioned GPRC5D binding peptide linked to a therapeutic agent.

In some embodiments, the therapeutic agent is a drug.

In some embodiments, the therapeutic agent is a cytotoxin.

In some embodiments, the therapeutic agent is a radioactive isotope.

In another aspect, the present invention provides a pharmaceutical composition, comprising: 1) the above-mentioned GPRC5D binding peptide; the above-mentioned fusion protein; the above-mentioned multispecific antibody molecule; or the above-mentioned immunoconjugate; and 2) a pharmaceutically acceptable carrier. The above-mentioned GPRC5D binding peptide, fusion protein, multispecific antibody molecule, immunoconjugate, or pharmaceutical composition can be used for treating GPRCSD-related disorders.

In another aspect, the present invention provides use of the above-mentioned GPRC5D binding peptide, fusion protein, multispecific antibody molecule, or immunoconjugate in the preparation of drugs for treating GPRC5D-related disorders.

In some embodiments, the above-mentioned GPRCSD-related disorder is a cancer or an autoimmune disease, and the cancer is preferably a plasma cell malignancy disease, such as multiple myeloma, or a B cell malignant disease, such as Hodgkin's lymphoma or non-Hodgkin's lymphoma.

In another aspect, the present invention provides a method for treating GPRCSD-related disorders, including: administering an effective amount of the above-mentioned GPRC5D binding peptide, fusion protein, multispecific antibody molecule, immunoconjugate, or pharmaceutical composition to a subject in need thereof. In some embodiments, the above-mentioned GPRCSD-related disorder is a cancer or an autoimmune disease, and the cancer is preferably a plasma cell malignancy disease, such as multiple myeloma, or a B cell malignant disease, such as Hodgkin's lymphoma or non-Hodgkin's lymphoma.

In another aspect, the present invention provides a method for detecting the content of GPRC5D in a biological sample, including:
1) contacting the biological sample with the above-mentioned GPRC5D binding peptide, fusion protein, or multispecific antibody molecule, so as to form a binding complex with the GPRC5D; and
2) determining the content of GPRC5D in the biological sample based on the amount of the binding complex generated in step 1).

In another aspect, the present invention provides a method for detecting the content of GPRC5D in a biological sample, including:
1) coupling the above-mentioned GPRC5D binding peptide to a detectable tag;
2) contacting the GPRC5D binding peptide carrying the detectable tag with the biological sample; and
3) determining the content of GPRC5D in the biological sample by detecting the amount of the detectable tag.

In another aspect, the present invention provides a detection kit, comprising the above-mentioned GPRC5D binding peptide, fusion protein, or multispecific antibody molecule.

In another aspect, the present invention provides a drug kit, comprising the above-mentioned GPRC5D binding peptide, fusion protein, or multispecific antibody molecule.

In another aspect, the present invention provides a method for identifying GPRC5D-related disorders in a subject, including:
1) using the above-mentioned detection kit to determine the content of GPRC5D in a biological sample from the subject; and
2) comparing with the normal content of GPRC5D in a population to determine the presence or status of the GPRC5D-related disorder.

In some embodiments, the GPRCSD-related disorder is a cancer or an autoimmune disease, and the cancer is preferably a plasma cell malignancy disease, such as multiple myeloma, or a B cell malignant disease, such as Hodgkin's lymphoma or non-Hodgkin's lymphoma.

In another aspect, the present invention provides a method for determining the therapeutic effect of a drug for treating GPRC5D-related disorders in a subject, including:
1) using the above-mentioned method to identify the status of GPRCSD-related disorder in the subject;
2) treating the subject with the drug; and
3) repeating step 1) to determine the change in the status of GPRCSD-related disorder in the subject, and determining the therapeutic effect of the drug based on the change.

In some embodiments, the GPRCSD-related disorder is a cancer or an autoimmune disease, and the cancer is preferably a plasma cell malignancy disease, such as multiple myeloma, or a B cell malignant disease, such as Hodgkin's lymphoma or non-Hodgkin's lymphoma.

In another aspect, the present invention provides a chimeric antigen receptor (CAR), whose extracellular antigen binding domain includes one or more antibody molecules or antigen binding fragments thereof targeting GPRC5D, where the HCDR1, HCDR2 and HCDR3 of the heavy chain variable region of the antibody molecule are selected from one of the following combinations:
1) the sequence of HCDR1 is GGSFSGYY (SEQ ID NO:1);
   the sequence of HCDR2 is INHSGST (SEQ ID NO: 2);
   the sequence of HCDR3 is ARARRYGGRTRFDP (SEQ ID NO:3);
2) the sequence of HCDR1 is GFIFSSYG (SEQ ID NO:4);
   the sequence of HCDR2 is ISSSGDYT (SEQ ID NO: 5);
   the sequence of HCDR3 is ARMSFRRYDH (SEQ ID NO:6); and
3) the sequence of HCDR1 is GFSFSGYI (SEQ ID NO:7);
   the sequence of HCDR2 is TSSSGTET (SEQ ID NO: 8);
   the sequence of HCDR3 is ARYYSKYGRSYHVDS (SEQ ID NO: 9).

In some embodiments, the extracellular antigen binding domain includes a plurality of (such as two) antibody molecules or antigen binding fragments thereof connected in series, and preferably, the antibody molecules or antigen binding fragments thereof are connected by a linker peptide.

In some embodiments, the two antibody molecules or antigen binding fragments thereof are the same or different.

In some embodiments, the antibody molecule is a single domain antibody.

In some embodiments, the antibody molecule is a fully humanized single domain antibody.

In some embodiments, the heavy chain variable region (or the single domain antibody) comprises an amino acid sequence as shown in SEQ ID NO: 10, 11 or 12; or the heavy chain variable region comprises an amino acid sequence having at least 90%, 91%, 92%, 93%, 94%, 95%, 96%, 97%, 98%, or 99% sequence identity to the sequence as shown in SEQ ID NO: 10, 11 or 12 and is capable of specifically binding to GPRC5D.

In some embodiments, the CAR includes the extracellular antigen binding domain, a transmembrane domain and an intracellular signaling domain sequentially from an N-terminus to a C-terminus.

In some embodiments, the CAR further includes a signal peptide at the N-terminus.

In some embodiments, the signal peptide comprises an amino acid sequence as shown in SEQ ID NO: 17.

In some embodiments, the CAR further includes a hinge region between the extracellular antigen binding domain and the transmembrane domain.

In some embodiments, the hinge region comprises an amino acid sequence as shown in SEQ ID NO: 19.

In some embodiments, the transmembrane domain comprises an amino acid sequence as shown in SEQ ID NO: 21.

In some embodiments, the intracellular signaling domain includes a 4-1BB intracellular domain and a CD3ζ intracellular domain.

In some embodiments, the 4-1BB intracellular domain comprises an amino acid sequence as shown in SEQ ID NO: 23.

In some embodiments, the CD3ζ intracellular domain comprises an amino acid sequence as shown in SEQ ID NO: 25.

In some embodiments, the end of the CAR is also linked to a safety switch for CAR-T cell clearance.

In some embodiments, a safety switch is linked to the C-terminus of the CAR.

In some embodiments, the safety switch includes a tEGFR or a fusion protein with a suicide capability.

In some embodiments, the C-terminus of the CAR is linked to an EGFR molecule (tEGFR) in a truncated form. In some embodiments, a CSF2RA signal peptide is further linked to the N-terminus of the tEGFR.

In some embodiments, the tEGFR comprises an amino acid sequence as shown in SEQ ID NO: 31.

In some embodiments, the CSF2RA signal peptide comprises an amino acid sequence as shown in SEQ ID NO: 29.

In some embodiments, the safety switch is linked to the CAR via a self-cleaving polypeptide.

In some embodiments, the self-cleaving polypeptide comprises an amino acid sequence as shown in SEQ ID NO: 27 or 33.

In some embodiments, the CAR has an amino acid sequence as shown in SEQ ID NO: 35, 37 or 39; or the amino acid sequence of the CAR has at least 90%, 91%, 92%, 93%, 94%, 95%, 96%, 97%, 98%, or 99% sequence identity to the sequence as shown in SEQ ID NO: 35, 37 or 39.

In another aspect, the present invention provides a bispecific chimeric antigen receptor (CAR), whose extracellular antigen binding domain includes (i) an antibody molecule or an antigen binding fragment thereof targeting GPRC5D and (ii) an antibody molecule or an antigen binding fragment thereof targeting a second target, where the single domain antibody heavy chain variable region (V_{HH}) and the full anti-scFv (variable region) of the antibody molecule targeting GPRC5D are described above, and the second antibody molecule is selected from CD3, BCMA or a combination thereof.

In another aspect, the present invention further provides an isolated nucleic acid molecule encoding the above-mentioned CAR.

In some embodiments, the nucleic acid molecule comprises a nucleotide sequence as shown in any one of SEQ ID NO: 13, 14, 15, 16, 18, 20, 22, 24, 26, 28, 30, 32, 34, 36, and 38.

In another aspect, the present invention further provides an expression vector, comprising the above-mentioned nucleic acid molecule.

In some embodiments, the expression vector is selected from a plasmid, a retroviral vector and a lentiviral vector.

In another aspect, the present invention further provides a host cell, comprising the above-mentioned CAR, nucleic acid molecule, or expression vector.

In some embodiments, the host cell is an immune cell.

In some embodiments, the host cell includes T cell or NK cell, preferably 293T cell.

In another aspect, the present invention further provides an engineered immune cell expressing the above-mentioned CAR.

In some embodiments, the engineered immune cell includes an immune cell, preferably T cell or NK cell.

In some embodiments, the engineered immune cell includes an autologous cell or an allogeneic cell.

In another aspect, the present invention further provides a pharmaceutical composition, comprising the above-mentioned cell and a pharmaceutically acceptable carrier.

In another aspect, the present invention further provides use of the above-mentioned CAR, nucleic acid molecule, expression vector, host cell or engineered immune cell in the preparation of drugs for preventing or treating GPRC5D-related diseases.

In some embodiments, the GPRCSD-related disease is a cancer or an autoimmune disease, and the cancer is preferably a plasma cell malignancy disease, such as multiple myeloma, or a B cell malignant disease, such as Hodgkin's lymphoma or non-Hodgkin's lymphoma.

In another aspect, the present invention further provides a method for preventing or treating GPRCSD-related diseases, including: administering a therapeutically effective amount of the above-mentioned engineered immune cell or pharmaceutical composition to a subject in need thereof.

In some embodiments, the method further includes administering an EGFR antibody to the subject to inhibit the activity of the engineered immune cell or the pharmaceutical composition.

In some embodiments, the GPRCSD-related disease is a cancer or an autoimmune disease, and the cancer is preferably a plasma cell malignancy disease, such as multiple myeloma, or a B cell malignant disease, such as Hodgkin's lymphoma or non-Hodgkin's lymphoma.

The present application is based on an extremely specific expression spectrum of GPRC5D, its potential safety and the good therapeutic effect of BCMA therapy. A CAR-T product targeting GPRC5D has been developed for the treatment of refractory relapsed multiple myeloma. Its GPRC5D binding domain is developed from the screening of a humanized single domain antibody library, has a relatively simple structure, and has low immunogenicity.

### DESCRIPTION OF DRAWINGS

FIG. 1 shows the results of enzyme-linked immunosorbent assay (ELISA) of some panned phage monoclones with a target antigen and a control antigen.

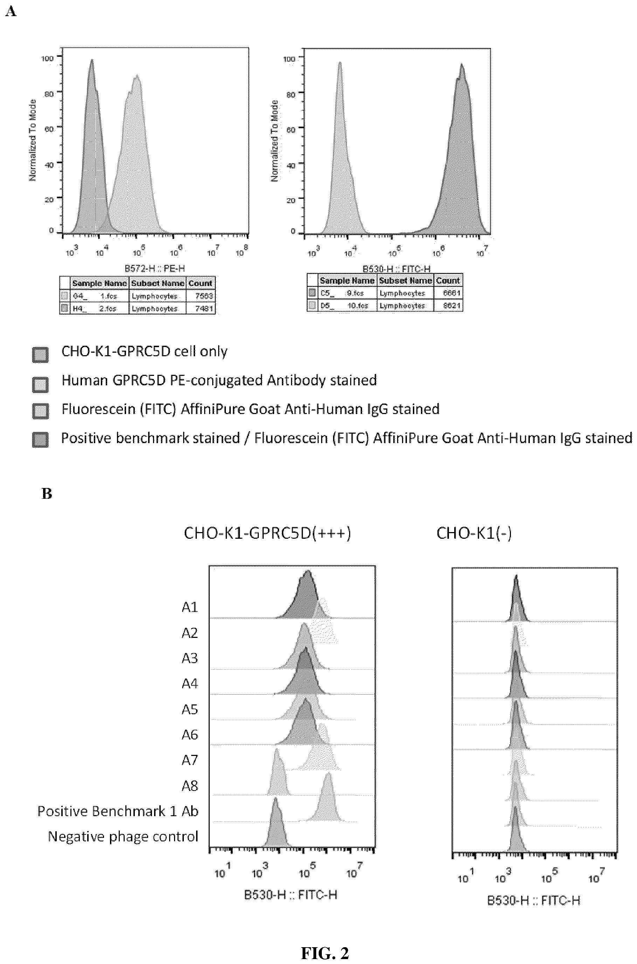

FIG. 2A shows the results of flow cytometric analysis of a CHO-K1-GPRC5D cell line stained with different antibodies; Human GPRC5D PE-conjugated Antibody stained is the result of staining using commercial flow cytometric antibodies, Fluorescein (FITC) AffiniPure Goat Anti-Human IgG stained is the negative control of staining using only fluorescently labeled antibodies; Positive benchmark stained/Fluorescein (FITC) AffiniPure Goat Anti-Human IgG stained is the result of staining using positive control antibodies and fluorescently labeled antibodies; and FIG. 2B shows the results of flow cytometric analysis of some phage monoclones binding to CHO-K1-GPRC5D and CHO-K1 cells.

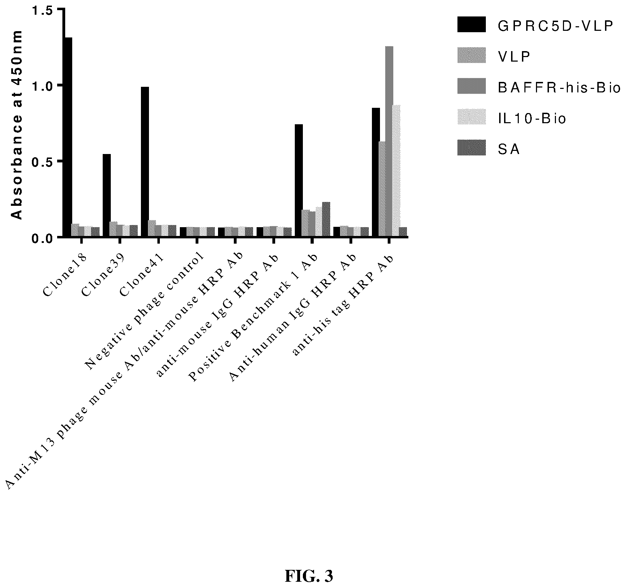

FIG. 3 shows the results of enzyme-linked immunosorbent assay (ELISA) analysis of the screened phage monoclones at the phage level with a variety of non-related antigens. Negative phage control is a negative control phage antibody clone, Anti-M13 phage mouse/anti-mouse HRP Ab is the negative antibody control with only primary and secondary antibodies added, anti-mouse HRP Ab is the negative antibody control with only secondary antibodies added, anti-human IgG HRP Ab is the negative antibody control with only secondary antibodies added, anti-his tag HRP Ab is the positive antibody control for detecting antigen tags, and Positive Benchmark1 is the positive antibody control for target antigens.
FIG. 4 shows the results of flow cytometric analysis of the screened phage monoclones at the phage level binding to a variety of different GPRC5D-positive and negative cell lines. Negative phage Control is a negative control phage antibody clone, Positive Benchmark1 Ab is a positive antibody for target antigens, and FITC anti-human IgG Ab is the negative control with only secondary antibodies added.
FIG. 5 shows the results of enzyme-linked immunosorbent assay (ELISA) analysis of the screened phage monoclones at the protein level with a variety of non-related antigens. Anti-human IgG HRP Ab is the negative antibody control with only secondary antibodies added, anti-his tag HRP Ab is the positive antibody control for detecting antigen tags, and Positive Benchmark1 is the positive antibody control for target antigens.
FIG. 6 shows the results of flow cytometric analysis of the screened phage monoclones at the protein level binding to a variety of different GPRC5D-positive and negative cell lines. Positive Benchmark1 Ab is a positive antibody for target antigens, and FITC anti-human IgG Ab is the negative control with only secondary antibodies added.
FIG. 7 shows the Experimental results of FACS detection of monoclones binding to CHO-K1-GPRC5D cells that highly express target antigens.
FIG. 8 shows the Experimental results of FACS detection of corresponding monoclonal antibodies respectively binding to monkey-derived and murine-derived cells highly-expressing GPRC5D (i.e., CHOS-macaqaueGPRC5D (FIG. 8A) and CHOS-mouseGPRC5D (FIG. 8B)).
FIG. 9 shows schematic diagrams of structures of GPRC5D CAR vector construction. CAR includes an extracellular signal peptide (SP), a ScFv (VL-linker-VH) or Single domain (V_{HH}) that binds to a target antigen GPRC5D, a hinge region and a transmembrane region (CD8a hinge+TM) between a cell membrane and an extracellular binding region, a 4-1BB co-stimulatory molecule, a CD3ζ intracellular domain, a T2A self-cleaving peptide, and a truncated tEGFR.
FIG. 10 shows the expression of tEGFR in each group of CAR-T cells.
FIG. 11 shows the killing results of each group of CAR-T cells against multiple target cells (8226-luc, U266-luc, MM1.s-luc, H929-luc, CCRF-luc, Raji-luc).
FIG. 12 shows in vitro proliferation conditions of various CAR-T cells after multiple target cell (8226) stimulations.
FIG. 13 shows the results of a CD107a degranulation effect of tumor cells derived from different tissue on CAR-T cells in each group.
FIG. 14 shows an anti-tumor efficacy of CAR-T cells corresponding to different clones in H929-luc tumor-bearing NCG mice. FIG. A shows the change of photon number over time after CAR-T cell reinfusion, FIG. B shows the survival rate of mice after CAR-T cell reinfusion, and FIG. C shows the change of the T cell to mouse lymphocyte ratio after CAR-T cell reinfusion.

### DETAILED DESCRIPTION

Unless otherwise defined, all technical and scientific terms used herein have the meanings as commonly understood by those of ordinary skill in the art.

Antibodies refer to immunoglobulins secreted by plasma cells (effector B cells)and used by the body's immune system to neutralize foreign substances (peptides, viruses, bacteria, etc.). The foreign substance is correspondingly called an antigen. The basic structure of a classical antibody molecule is a 4-mer consisting of 2 identical heavy chains and 2 identical light chains. According to the conservative differences in amino acid sequences, the heavy and light chains are divided into a variable region (V) at the amino terminus and a constant region (C) at the carboxy terminus. The variable regions of one heavy chain and one light chain interact to form an antigen binding site (Fv). In the variable region, the composition and arrangement of amino acid residues in certain regions are more variable than other regions (framework regions, FRs) in the variable region. These regions are called hypervariable regions (HVRs) which are actually the key sites for binding of antibodies to antigens. Since these hypervariable regions have their sequences complementary to antigenic determinants, they are also called complementarity-determining regions (CDRs). Both heavy and light chains have three complementarity-determining regions, called HCDR1, HCDR2 and HCDR3, and LCDR1, LCDR2 and LCDR3, respectively.

The "antigen binding fragment" of an antibody molecule refers to an amino acid fragment involved in antigen-specific binding in the antibody molecule, for example, Fab, Fab', and (Fab')₂. The single chain antibody and single domain antibody with an antigen binding capacity are single peptide chain antibody molecules, which can be regarded as "antigen binding fragments" of classical antibody molecules.

The "Fc fragment" refers to a stem region of a Y-shaped antibody molecule, that is, a fragment crystallizable (Fc) includes second and third constant domains (CH2 and CH3 domains) of heavy chains. An antibody Fc region can be obtained by hydrolyzing antibody molecules with a proteolytic enzyme (such as papain). In some examples, the Fc region can comprise a hinge, CH2, and CH3. When the Fc region comprises a hinge, it can mediate dimerization between two Fc-containing polypeptides. The Fc fragment can be derived from IgG, IgM, IgD, IgE or IgA. In some examples, the Fc region is derived from IgG1, IgG2, IgG3, or IgG4. The "Fc fragment" also includes variant Fc fragments derived from natural Fc fragments that have been modified but still retain their effector functions. The "variant Fc fragment" comprises an amino acid sequence having at least one amino acid change in the amino acid sequence of a natural Fc fragment. In some examples, the variant Fc fragment has at least one amino acid substitution compared to a parent Fc fragment (natural Fc fragment), for example, about 1 to about 10 amino acids are substituted in the parent Fc fragment, and preferably, about 1 to about 5 amino acids are substituted. In some examples, the variant Fc fragment/Fc region has at least about 80% sequence identity, at least about 90% sequence identity, at least about 95%, at least about 96%, at least about 97%, at least about 98% or at least about 99% sequence identity to the parent Fc fragment. The effector functions of the "Fc fragment" may include binding to Fc receptors, Clq binding and complement-dependent cytotoxicity (CDC), antibody-dependent cell-mediated cytotoxicity (ADCC), mediating phagocytosis, etc.

The single chain fragment variable (scFv) is composed of antibody heavy and light chain variable regions linked by a short peptide into a peptide chain. Through correct folding, the variable regions from the heavy chain and light chain interact through non-covalent bonds to form the Fv segment, so that scFv can better retain its affinity activity for antigens.

The "single domain antibody (sdAb)", or also called a "V_{HH} antibody", refers to an antibody molecule which has an antigen binding ability and includes a heavy chain variable region without a light chain. From a structural point of view, the single domain antibody may also be considered as an antigen binding fragment of an antibody molecule. It was first discovered in camelids, and subsequently, researchers discovered more single domain antibodies with an antigen binding ability through screening of antibody libraries (such as phage display libraries). Single domain antibodies have some advantages compared to ordinary antibody molecules(for example, classical tetrameric antibody molecules)or their antigen binding fragments, for example including but not limited to: smaller molecular weight, which makes it easy for ordinary antibody molecules to reach tissues or parts that are difficult to reach when used in the human body, or makes it being capable of contacting antigenic epitopes in proteins or polypeptides that are difficult for ordinary antibody molecules to contact; and being more stable, and able to withstand changes in temperature and pH, as well as the effects of denaturants and proteases. The "fully humanized antibodies" refer to antibodies derived from a human germline immunoglobulin sequence in variable and constant regions (if any). The fully humanized antibodies can be obtained through a variety of technologies, including a phage antibody library technology, a single B cell cloning technology, a transgenic mouse technology (for example, using transgenic mice that have introduced human germline immunoglobulin genes and removed the mouse's own germline immunoglobulin genes), etc. Compared with animal-derived antibodies (such as murine-derived antibodies), fully humanized antibodies have the advantages of low immunogenicity and high safety when used in human patients.

The "bispecific antibody" refers to an antibody molecule that has two different binding sites and can respectively recognize and bind to two different antigens. For example, one binding site of a bispecific antibody can be used to bind to immune cells (such as T cells), and the other binding site is used to bind to tumor cells, thereby enhancing the killing effect of immune cells on tumor cells while reducing side effects such as off-target toxicity. This bifunctional antibody is usually more effective than a monoclonal antibody as a drug for treating tumors. Similarly, an antibody molecule may be modified to include a plurality of different binding sites to produce a "trispecific antibody", a "tetraspecific antibody", etc. The "multi-specific antibody" as used herein covers these bispecific antibodies, trispecific antibodies, tetraspecific antibodies, etc.

The "immunoconjugates" refer to GPRC5D binding peptides conjugated with therapeutic agents as disclosed herein. The therapeutic agent is, for example, a cytotoxin, a drug (e.g., an immunosuppressant), or a radiotoxin.

The "fusion protein" refers to artificially produced (for example, by a genetic engineering technology) protein molecules consisting of at least two different peptide segments. These peptide segments do not exist in nature or do not exist in the same protein molecule. Examples of the common fusion proteins including antibody fragments include antibody-cytokine fusion proteins, antibody-cytotoxin fusion proteins (also known as immunotoxins), enzyme-labeled antibodies for immunoassays, chimeric antigen receptors (CARs), etc.

"Targeting" or "specific binding" refer to that one molecule (e.g., antibody or antigen binding fragment thereof) has a higher binding affinity for another molecule(e.g., tumor cell surface antigen) relative to other molecules that co-exist in the environment. The "targeting" or "specific binding" does not rule out that the molecule may have a binding affinity for more than one molecule, for example, bispecific antibodies may have a high affinity for two different antigens.

The "binding complex" refers to a complex formed between a molecule and its binding object, including both. Examples of the binding complexes may include antigen-antibody complexes, ligand-receptor complexes, protein dimers, etc. The forces that form the binding complex mainly include non-covalent bond forces such as hydrogen bonds, van der Waals forces, and ionic bonds.

GPRC5D is a G protein-coupled receptor C5 family subtype D, belonging to an orphan receptor, which is a 7transmembrane protein. An orphan receptor refers to a receptor which has a structure that is significantly similar to those of other confirmed receptors but whose endogenous ligand has not yet been discovered. GPRC5D is highly expressed on the surface of primary multiple myeloma cells, while its expression in normal tissues is limited to the hair follicle area. Studies have shown that65%of multiple myeloma patients have an expression threshold of GPRC5D exceeding 50%. With this feature, GPRC5D has become a potential target for the treatment of multiple myeloma (MM).

The "disorder status" refers to the presence, severity, stage, type, and progression of the disorder.

The GPRC5D binding peptide refers to a polypeptide that targets or specifically binds to GPRC5D. For example, the GPRC5D binding peptide is a single domain antibody targeting GPRC5D.

The "peptide segment" refers to a polypeptide fragment, which is a short amino acid sequence, for example, about 2-20 amino acids in length, and is usually part of a polypeptide or protein.

EC50 (concentration for 50% of maximal effect) refers to the concentration that causes 50%of the maximal effect. In FACS, when used to indicate the ability of an antibody molecule to bind to the corresponding antigen on a cell, it refers to the concentration of the antibody molecule that produces half of the maximal fluorescence intensity. The lower the EC50 value, the greater the binding affinity to the antigen on the cell.

The "purification tag" refers to an amino acid sequence expressed together with a protein or polypeptide of interest in the form of a fusion protein for the purification of the protein or polypeptide of interest, including but not limited to an His6 tag, a Flag tag, an MBP (maltose binding protein) tag, a GST (glutathione mercaptotransferase) tag, an SUMO (small ubiquitin related modifier), etc. These tags can be removed by enzymatic cleavage after purification, or can be used with tags without affecting the normal functions of the protein or polypeptide of interest (such as His6 tag).

The "detectable tag" refers to an amino acid sequence or other chemical groups linked to a protein or polypeptide, used to indicate the presence or content of the protein or polypeptide in a sample, or used to track information on the location of the protein or polypeptide in a subject's body or cells. Examples of the detectable tags include various enzymes that can be used in immunoassays, such as horseradish peroxidase (HRP), and alkaline phosphatase (ALP); fluorescent groups (such as FAM and FITC) or fluorescent proteins (such as GFP); and radioactive isotopes (such as ³H, ¹⁴C, ³⁵S). When the detectable tag is an enzyme, the presence or amount of the protein or polypeptide linked to the enzyme can be determined by the enzymatic activity of the enzyme.

The terms "polypeptide" and "protein" are interchangeably used and refer to polymers of amino acid residues. Such polymers of amino acid residues may contain natural or non-natural amino acid residues and include, but are not limited to, peptides, oligopeptides, dimers, trimers and multimers composed of amino acid residues. Both full-length proteins and fragments thereof are encompassed by the definition. The term also includes post-expression modifications of the polypeptide, such as glycosylation, sialylation, acetylation, phosphorylation, and the like modifications. In addition, for the purposes of the present invention, the "polypeptide" refers to a protein that includes modifications of natural sequences, such as deletions, additions, and substitutions (actually generally conserved), as long as the protein retains the desired activity. These modifications may be deliberate, such as through site-directed mutagenesis, or may be accidental, such as through mutations in the host that produces the protein or errors due to PCR amplification.

Herein, the terms "nucleic acid molecule", "nucleic acid" and "polynucleotide" are interchangeably used and refer to nucleotide polymers. Such nucleotide polymers may contain natural and/ or non-natural nucleotides and include, but are not limited to, DNA, RNA, and PNA. The "nucleic acid sequence" refers to a linear sequence of nucleotides contained in a nucleic acid molecule or polynucleotide.

The term "vector" refers to a nucleic acid molecule that can be engineered to contain a polynucleotide of interest (e.g., a coding sequence of a polypeptide of interest), or a nucleic acid molecule that can be replicated in a host cell (e.g., a nucleic acid, plasmid, or virus, etc.). The vector may include one or more of the following components: an origin of replication, one or more regulatory sequences that regulate the expression of the polynucleotide of interest (such as a promoter and/or an enhancer), and/or one or more selectable marker genes (such as antibiotic resistance genes and genes that can be used in colorimetric analysis, e.g., β-galactose). The term "expression vector" refers to a vector used to express a polypeptide of interest in a host cell.

The "host cell" refers to cells which may be or have been vectors or receptors of isolated polynucleotides. The host cell may be a prokaryotic cell or a eukaryotic cell. Exemplary eukaryotic cells include mammalian cells, such as primate or non-primate cells; fungal cells, such as yeast; plant cells; and insect cells. Non-limiting exemplary mammalian cells include (but are not limited to) NSO cells, 293 and CHO cells, and derived cells thereof, such as 293-6E, CHO-DG44, CHO-K1, CHO-S and CHO-DS cells. Host cells include the progeny of a single host cell, and the progeny may not necessarily be completely identical to the original parent cell (in morphology or genomic DNA complementarity) due to natural, accidental, or deliberate mutation. Host cells also include cells transfected in vivo with the nucleic acid molecules or expression vectors provided herein.

The "treatment" refers to the treatment on a subject to obtain beneficial or desired clinical results. The "treatment" as used herein covers a variety of treatment means, including the administration to the subject of any possible drug, surgery, radiation, etc. For the purposes of the present invention, the beneficial or desired clinical results include, but are not limited to, any one or more of the following: alleviation of one or more symptoms, reduction in disease severity, prevention or delay of disease spread (e.g., metastasis, such as to the lungs or lymph nodes), prevention or delay of disease recurrence, delay or slowing of disease progression, improvement of disease status, inhibition of disease or disease progression, arrest of its development, and remission (whether partial or complete). The methods provided herein encompass any one or more of these therapeutic aspects. As stated above, the "treatment" does not require complete removal of all symptoms of the disorder or disease or complete remission.

The term "therapeutically effective amount" refers to an amount of an active compound sufficient to elicit the biological or medical response desired by a clinician in a subject. The "therapeutically effective amount" of the fusion protein of the present invention can be determined by those skilled in the art according to the administration route, the subject's body weight, age or condition, and other factors. For example, a typical daily dose may range from 0.01 mg to 100 mg or more of active ingredient per kg of body weight.

When referring to pharmaceutical compositions, the term "pharmaceutically acceptable carrier" used refers to substances such as solid or liquid diluents, fillers, antioxidants, and stabilizers and the like, which can be safe for administration, and which are suitable for administration to humans and/or animals without undue adverse side effects, while being suitable for maintaining the activity of the drug or active agent therein. Depending on the administration route, various different carriers well known in the art can be used, including, but not limited to, saccharides, starch, cellulose and derivatives thereof, maltose, gelatin, talc, calcium sulfate, vegetable oil, synthetic oil, polyol, alginic acid, phosphate buffers, emulsifiers, isotonic saline, and/or pyrogen-free water, etc.

The pharmaceutical composition provided herein may be prepared into clinically acceptable dosage forms such as powders and injections. The pharmaceutical composition of the present invention may be administered to a subject in any appropriate route, for example, orally, by intravenous infusion, by intramuscular injection, by subcutaneous injection, subperitoneally, rectally, sublingually, or by inhalation, transdermally, and the like.

The "subject" refers to animals, such as mammals, including but not limited to humans, rodents, apes, feline, canine, equine, bovine, swine, sheep, goats, mammalian laboratory animals, mammalian livestock, mammalian sports animals, and mammalian pets. The subject may be male or female and may be of any appropriate age, including infants, young children, adolescents, adults, and elderly subjects. In some examples, the subject refers to an individual in need of treatment for a disease or disorder. In some examples, the subject receiving treatment may be a patient who suffers from, or is at risk of developing, a disorder associated with the treatment. In certain examples, the subject is a human, such as a human patient. The term is often used interchangeably with a "patient", a "detection object", a "treatment object", etc.

The "population" as used herein usually refers to the healthy population. In the specific analysis of certain disease, the "population" may also refer to an individual who does not suffer from the disease but suffers from other diseases. In addition, some individuals may be designated as "population" according to age, smoking, alcohol abuse, personal health status and the like characteristics. The "normal GPRC5D content" in the population can be obtained by determining a sufficient number of individuals.

The "biological sample" means a certain amount of substances from living being or prior living being. The substances include (but are not limited to) blood (such as whole blood), plasma, serum, urine, amniotic fluid, synovial fluid, endothelial cells, leukocytes, monocytes, other cells, organs, tissues, bone marrow, lymph nodes and spleen.

The term "sequence identity" (also known as sequence consistency) when referring to amino acid or nucleotide sequences refers to the degree of identity between two amino acid or nucleotide sequences (e.g., a query sequence and a reference sequence), usually expressed as a percentage. Typically, before calculating the identity percentage between two amino acid or nucleotide sequences, the sequence alignment is first performed, and a gap (if any) is introduced. If at a certain alignment position, the amino acid residues or bases in the two sequences are the same, the two sequences are considered to be identical or matched at the position; and if the amino acid residues or bases in the two sequences are different, they are considered to be non-identical or mismatched at the position. In some algorithms, the number of matched positions is divided by the total number of positions in the alignment window to obtain sequence identity. In other algorithms, the number of gaps and/or the gap length are also taken into account. For the purposes of the present invention, the published alignment software BLAST (available at ncbi.nlm.nih.gov) can be employed to obtain optimal sequence alignments by using default settings, and calculate the sequence identity between two amino acid or nucleotide sequences.

The "chimeric antigen receptor (CAR)", also known as a chimeric T cell receptor or a chimeric immunoreceptor, is an engineered protein receptor molecule that can confer a desired specificity to immune effector cells, such as the ability to bind to specific tumor antigens. The chimeric antigen receptor generally consists of an extracellular antigen binding domain, a transmembrane domain, and an intracellular signaling domain. In most cases, the antigen binding domain is a segment of scFv sequence responsible for recognizing and binding to a specific antigen. The intracellular signaling domain usually includes an immunoreceptor tyrosine-based activation motif (ITAM), such as a signaling domain derived from the CD3ζ molecule, which is responsible for activating immune effector cells and producing a killing effect. In addition, the chimeric antigen receptor may also include a signal peptide responsible for intracellular localization of the nascent protein at the amino terminus, and a hinge region between the antigen binding domain and the transmembrane domain. In addition to signaling domains, intracellular signaling domains may also include co-stimulatory domains derived from, for example, 4-1BB molecules.

The "CAR-T cells" refer to T cells expressing CARs, which are usually obtained by transducing T cells with an expression vector encoding CARs. Commonly used expression vectors are viral vectors, such as lentiviral expression vectors. Chimeric antigen receptor-modified T cells (CAR-T) are not restricted by the major histocompatibility complex and have specific targeted killing activity and the ability to proliferate persistently. In addition to T cells, other lymphocytes such as NK cells may also be transformed with an expression vector encoding a CAR to obtain targeted killer cells expressing the CAR.

"Self-cleaving peptides" refer to short peptides that can be achieve the function of cleaving proteins by ribosome skipping rather than protease hydrolysis, which may include T2A, F2A, and P2A.

The "CSF2RA signal peptide", i.e., the colony stimulating factor 2 receptor subunit alpha signal peptide, can induce the expression of newly synthesized proteins such as EGFRt on the surface of CAR-T cells.

"EGFRt" or "tEGFR" may be used interchangeably herein to refer to a gene encoding a truncated human epidermal growth factor receptor polypeptide or an encoded product thereof, which lacks a distal membrane EGF binding domain and cytoplasmic signaling tails but retains an extracellular epitope recognized by an anti-EGFR antibody. EGFRt can be used as a non-immunogenic selection tool and tracking marker with genetically modified cell functions. Herein, it can be used as a marker molecule for CAR-T cells on the one hand, and on the other hand, it can also be used to eliminate CAR-T cells in the body when necessary, for example, through the ADCC pathway mediated by EGFR antibodies (e.g., cetuximab) (see US8802374B2), that is, used as a safety switch during clinical transformation.

In addition to using tEGFR, the CAR-T cells provided herein can also be considered for use in combination with safety switches of other mechanisms. For example, the CAR provided herein can be processed so that it can utilize a chemical inducer of dimerization (CID) to control the activity of the CAR. In one example, the complete CAR provided herein can be expressed as a separate fusion protein (e.g., the CD3ζ intracellular domain is expressed separately from other portions), and the complete CAR can be reconstituted in the cell using a dimerization mechanism based on the FKBP/FRB binding domain and rapamycin (or a rapamycin analog), thereby controlling the integrity and activity of the CAR by the presence or absence of rapamycin (e.g., see the description in U.S. Pat. No. 11,084,880). In addition, when a self-cleaving peptide is used to enable the simultaneous expression of a fusion protein with a suicide capability, the activity of CAR-T cells can be controlled by degrading CAR molecules or causing the death of CAR-T cells. Examples in this aspect include the use of a PROTAC technology to degrade CAR molecules and the use of proteins with the ability to induce apoptosis, such as caspase-9 ^{[8]}.

### Brief description of amino acid and nucleotide sequences

The following provides descriptions of amino acid and coding nucleic acid sequences of some antibodies or antigen binding fragments (such as single domain antibodies) and CARs and related polypeptides used herein (especially in Examples).
SEQ ID NO:1 is an amino acid sequence of CDR1 of clone #18, i.e., CDR1 of an extracellular antigen binding domain of GPRC5D CAR 2290.
SEQ ID NO: 2 is an amino acid sequence of CDR2 of clone #18, i.e., CDR2 of the extracellular antigen binding domain of GPRC5D CAR 2290.
SEQ ID NO: 3 is an amino acid sequence of CDR3 of clone #18, i.e., CDR3 of the extracellular antigen binding domain of GPRC5D CAR 2290.
SEQ ID NO: 4 is an amino acid sequence of CDR1 of clone #39, i.e., CDR1 of an extracellular antigen binding domain of GPRC5D CAR 2436.
SEQ ID NO: 5 is an amino acid sequence of CDR2 of clone #39, i.e., CDR2 of the extracellular antigen binding domain of GPRC5D CAR 2436.
SEQ ID NO: 6 is an amino acid sequence of CDR3 of clone #39, i.e., CDR3 of the extracellular antigen binding domain of GPRC5D CAR 2436.
SEQ ID NO: 7 is an amino acid sequence of CDR1 of clone #41, i.e., CDR1 of an extracellular antigen binding domain of GPRC5D CAR 2438.
SEQ ID NO: 8 is an amino acid sequence of CDR2 of clone #41, i.e., CDR2 of the extracellular antigen binding domain of GPRC5D CAR 2438.
SEQ ID NO: 9 is an amino acid sequence of CDR3 of clone #41, i.e., CDR3 of the extracellular antigen binding domain of GPRC5D CAR 2438.
SEQ ID NO: 10 is an amino acid sequence of a heavy chain variable region of clone #18, i.e., a sequence of the extracellular antigen binding domain of GPRC5D CAR 2290.
SEQ ID NO: 11 is an amino acid sequence of a heavy chain variable region of clone #39, i.e., a sequence of the extracellular antigen binding domain of GPRC5D CAR 2436.
SEQ ID NO: 12 is an amino acid sequence of a heavy chain variable region of clone #41, i.e., a sequence of the extracellular antigen binding domain of GPRC5D CAR 2438.
SEQ ID NO: 13 is a coding sequence of the heavy chain variable region of clone #18, i.e., a coding sequence of the extracellular antigen binding domain of GPRC5D CAR 2290.
SEQ ID NO: 14 is a coding sequence of the heavy chain variable region of clone #39, i.e., a coding sequence of the extracellular antigen binding domain of GPRC5D CAR 2436.
SEQ ID NO: 15 is a coding sequence of the heavy chain variable region of clone #41, i.e., a coding sequence of the extracellular antigen binding domain of GPRC5D CAR 2438.
SEQ ID NO: 16 is a coding nucleic acid sequence of a CD8α signal peptide. The CD8α signal peptide can be used to guide a newly synthesized CAR to a cell surface. The signal peptide may be cleaved off in a mature CAR and thus may not be included.
SEQ ID NO: 17 is an amino acid sequence of the CD8α signal peptide.
SEQ ID NO: 18 is a coding nucleic acid sequence of a CD8α hinge region. The CD8α hinge region can be used to link the extracellular antigen binding domain and the transmembrane domain in the CAR.
SEQ ID NO: 19 is an amino acid sequence of the CD8α hinge region.
SEQ ID NO: 20 is a coding nucleic acid sequence of a CD8α transmembrane region.
SEQ ID NO: 21 is an amino acid sequence of the CD8α transmembrane region.
SEQ ID NO: 22 is a coding nucleic acid sequence of a 4-1BB intracellular domain.
SEQ ID NO: 23 is an amino acid sequence of the 4-1BB intracellular domain.
SEQ ID NO: 24 is a coding nucleic acid sequence of a CD3ζ intracellular signaling domain.
SEQ ID NO: 25 is an amino acid sequence of the CD3ζ intracellular signaling domain.
SEQ ID NO: 26 is a coding nucleic acid sequence of a self-cleaving peptide T2A.
SEQ ID NO: 27 is an amino acid sequence of the self-cleaving peptide T2A. Due to the presence of the self-cleaving peptide, the proteins on both sides of the self-cleaving peptide (CAR and EGFRt) are separated from each other during the translation process. For the convenience of description, it can be considered that the CAR including multiple elements as a fusion protein may also include T2A and EGFRt.
SEQ ID NO: 28 is a coding nucleic acid sequence of a CSF2RA signal peptide.
SEQ ID NO: 29 is an amino acid sequence of the CSF2RA signal peptide. It can be located upstream of EGFRt to guide a newly synthesized EGFRt to a cell surface. The CSF2RA signal peptide can be cleaved off from EGFRt.
SEQ ID NO: 30 is a coding nucleic acid sequence of EGFRt.
SEQ ID NO: 31 is an amino acid sequence of EGFRt.
SEQ ID NO: 32 is a coding nucleic acid sequence of a self-cleaving peptide P2A. The self-cleaving peptide P2A can also be selected to replace T2A as a linker peptide between CAR and EGFRt.
SEQ ID NO: 33 is an amino acid sequence of the self-cleaving peptide P2A.
SEQ ID NO: 34 is a coding sequence of GPRC5D CAR 2290(#18).
SEQ ID NO: 35 is an amino acid sequence of GPRC5D CAR 2290(#18).
SEQ ID NO: 36 is a coding sequence of GPRC5D CAR 2436 (#39).
SEQ ID NO: 37 is an amino acid sequence of GPRC5D CAR 2436(#39).
SEQ ID NO: 38 is a coding sequence of GPRC5D CAR 2438(#41).
SEQ ID NO: 39 is an amino acid sequence of GPRC5D CAR 2438(#41).

Among them, the sequences as shown in SEQ ID NOS: 34-39 are only the coding or amino acid sequences of the CAR structure and do not contain the EGFRt element.

In addition, the above-mentioned clones #18, #39 and #41 are examples of some specific single domain antibodies targeting GPRC5D for CAR construction provided in the present application.

### GPRC5D binding peptides

The present invention provides a GPRC5D binding peptide that specifically binds to GPRC5D. The GPRC5D binding peptide binds to the GPRC5D molecule with a relatively high binding affinity. The GPRC5D may be a fusion protein linked to other polypeptides, such as GPRC5D-VLP, or may be a GPRC5D membrane protein expressed on the cell surface. For example, as described in Examples below, the ability of the GPRC5D binding peptide to bind to GPRC5D can be measured by determination methods such as enzyme-linked immunosorbent assay (ELISA) and fluorescence activated cell sorting (FACS) technology. In addition, it can also be determined by other protein interaction determination methods known in the art, such as a surface plasmon resonance (SPR) technology and a bio-layer interferometry (BLI) technology.

In some embodiments, the GPRC5D binding peptide is an antibody molecule or an antigen binding fragment thereof. Preferably, the GPRC5D binding peptide is a single domain antibody.

As described in Examples herein, the GPRC5D binding peptide provided herein is derived from the screening of a humanized phage antibody library and is a fully humanized single domain antibody.

A single domain antibody is an antibody including only a heavy chain variable region, the examples of which may include, but are not limited to, a heavy chain antibody, an antibody naturally lacking a light chain, a single domain antibody derived from a classical four-chain antibody, and an engineered antibody. Single domain antibodies may be derived from any species, including, but not limited to, mouse, human, camel, llama, alpaca, vicuna, guanaco, shark, goat, rabbit, and/or cow.

The present invention provides three CDR sequences of GPRC5D binding peptides, which are shown as SEQ ID NOS: 1-3, 4-6, and 7-9, respectively.

Based on the CDR sequences of the GPRC5D binding peptide (single domain antibody) provided herein, those skilled in the art can construct various polypeptide constructs with a binding ability for GPRC5D, including combining framework regions (FRs) from different antibody molecules with these CDR sequences. These framework regions include natural framework region sequences from human antibodies or animal (e.g., mouse, rat, sheep, camel, etc.) antibodies. These framework regions may also include framework region sequence variants generated by altering the natural framework region sequence. A polypeptide construct that specifically binds to GPRC5D can be easily obtained by combining the CDR sequences provided herein with different framework region sequences to form a heavy chain variable region and detecting its ability to bind to GPRC5D. In some embodiments, the heavy chain variable region of the GPRC5D binding peptide (single domain antibody) provided by the present invention comprises an amino acid sequence having at least 90% sequence identity (e.g., at least 95%, at least 98%, at least 99% or even 100% sequence identity) to the sequence as shown in SEQ ID NO: 10.

In some embodiments, the heavy chain variable region of the GPRC5D binding peptide (single domain antibody) provided by the present invention comprises an amino acid sequence having at least 90% sequence identity (e.g., at least 95%, at least 98%, at least 99% or even 100% sequence identity) to the sequence as shown in SEQ ID NO: 11.

In some embodiments, the heavy chain variable region of the GPRC5D binding peptide (single domain antibody) provided by the present invention comprises an amino acid sequence having at least 90% sequence identity (e.g., at least 95%, at least 98%, at least 99% or even 100% sequence identity) to the sequence as shown in SEQ ID NO: 12.

Those skilled in the art can understand that, on the basis of the specific sequences provided herein, the corresponding variants of the antibody molecule targeting GPRC5D provided by the present invention can be obtained by replacing, deleting or adding a few amino acids, and verifying or screening the resultant product for its binding ability with the corresponding antigen GPRC5D or its biological activity, and these variants should also be included within the scope of the present invention. For example, the antibody molecule or single domain antibody provided herein may have a change of at least 1 and no more than 10, such as no more than 5, 4, 3, 2or 1, amino acids in its full length or variable region sequence or CDR sequence.

It is expected that the GPRC5D binding peptide described herein may comprise a conservative amino acid substitution. The conservative amino acid substitution can generally be described as a substitution of one amino acid residue with another amino acid residue of a similar chemical structure, and has little or substantially no effect on the function, activity or other biological properties of the polypeptide. The conservative amino acid substitution is well known in the art. The conservative substitution may, for example, be a substitution of one amino acid from the following groups (a) to (e) by another amino acid from the same group: (a) small aliphatic nonpolar or weakly polar residues: Ala, Ser, Thr, Pro and Gly; (b) polar negatively charged residues and (uncharged) amides thereof: Asp, Asn, Glu and Gln; (c) polar positively charged residues: His, Arg and Lys; (d) large aliphatic nonpolar residues: Met, Leu, Ile, Val and Cys; and (e) aromatic residues: Phe, Tyr and Trp.

The polypeptide construct may also include more than one GPRC5D binding peptides which are directly linked in series or linked in series via a linker sequence. In some embodiments, the polypeptide construct includes two GPRC5D binding peptides, and the two GPRC5D binding peptides are identical. In some embodiments, the polypeptide construct includes two GPRC5D binding peptides, and the two GPRC5D binding peptides differ in amino acid sequence by at least one amino acid. In some embodiments, the polypeptide construct includes at least one GPRC5D binding peptide provided herein and at least one other GPRC5D binding peptide. In some embodiments, the polypeptide construct includes two different GPRC5D binding peptides provided herein. In some embodiments, the polypeptide construct includes three different GPRC5D binding peptides provided herein.

The linker sequence may be a naturally occurring linker, a synthetic linker, or a combination of both. Particularly suitable linker sequences mainly include amino acid residues selected from glycine (Gly), serine (Ser), alanine (Ala) and threonine (Thr). For example, the linker may contain at least 75% (calculated based on the total number of residues present in the peptide linker), such as at least 80%, at least 85% or at least 90% of amino acid residues selected from Gly, Ser, Ala and Thr. The linker may also consist of only Gly, Ser, Ala and/or Thr residues. In some embodiments, the linker contains 1 to 25 glycine residues, 5 to 20 glycine residues, 5 to 15 glycine residues, or 8 to 12 glycine residues. In some aspects, suitable peptide linkers typically contain at least 50% glycine residues, such as at least 75% glycine residues. In some embodiments, the peptide linker comprises only glycine residues. In some examples, the peptide linker comprises only glycine and serine residues, for example, in the form of (GS) ₙ, where n is, for example, an integer from 1 to 20.

### Fusion protein comprising GPRC5D binding peptide

The above-mentioned polypeptide constructs including a plurality of GPRC5D binding peptides belong to fusion proteins including GPRC5D binding peptides. In addition, the GPRC5D binding peptide can also form a fusion protein with other polypeptides.

In some embodiments, the GPRC5D binding peptide can be linked to an Fc fragment to form a fusion protein. The Fc fragment may be located at the C-terminus and N-terminus of the GPRC5D binding peptide. Preferably, the Fc fragment may be located at the C-terminus of the GPRC5D binding peptide. The fusion protein formed by the GPRC5D binding peptide and the Fc fragment has the ability to specifically bind to GPRC5D, and also has the effector function of the Fc fragment, such as mediating complement-dependent cytotoxicity (CDC), antibody-dependent cell-mediated cytotoxicity (ADCC), mediating phagocytosis, etc. In addition, fusion with the Fc fragment can increase the half-life of the GPRC5D binding peptide in vivo, so as to increase the dosing interval when the GPRC5D binding peptide is used as a therapeutic drug. Non-limiting fusion protein constructs containing GPRC5D binding peptides may include, but are not limited to, the following forms: VHH1-Fc, VHH1-VHH2-Fc, and VHH1-VHH2-VHH3-Fc, where VHH refers to a GPRC5D binding peptide (single domain antibody), and VHH1, VHH2, and VHH3 may be the same or different.

In some embodiments, GPRC5D binding peptides may be linked to protein tags to form fusion proteins. Protein tags may include purification tags and detectable tags. Purification tags include, but are not limited to, His6 tags, Flag tags, MBP tags, GST tags, SUMO tags, etc. The detectable tag can be used to indicate the presence or content of the GPRC5D binding peptide in a sample, or to track the location information of the GPRC5D binding peptide in a subject or cell. Examples of detectable tags include various enzymes that can be used in immunoassays, such as horseradish peroxidase (HRP), alkaline phosphatase (ALP) and the like; fluorescent proteins such as GFP. Due to the ability of the GPRC5D binding peptide for specifically binding to GPRC5D, the content of the GPRC5D binding peptide can be determined by the amount of the detectable tag linked to the GPRC5D binding peptide, and thus the content of GPRC5D in the sample can be determined.

In other embodiments, GPRC5D binding peptides may be linked to cytokines or therapeutic proteins to form fusion proteins. In this case, the ability of the GPRC5D binding peptide for specifically binding to GPRC5D can be used to purposefully deliver cytokines or therapeutic proteins to specific tissues or cells (such as tumor tissues expressing GPRC5D) to achieve the therapeutic effects of cytokines or therapeutic proteins.

### Multispecific antibodies including GPRC5D binding peptides

In some embodiments, the present invention provides a multispecific binding peptide including at least one GPRC5D-binding domain (or functional unit) and one or more additional binding domains. The one or more additional binding domains may bind to a second antigen or protein other than GPRC5D.

In some embodiments, the present invention provides a multispecific binding peptide including at least two GPRC5D-binding domains. The two GPRC5D-binding domains differ in amino acid sequence and bind to different antigenic epitopes on GPRC5D, respectively.

In some embodiments, provided herein is a multispecific binding peptide including at least two GPRC5D-binding domains and one or more additional binding domains. The two GPRC5D-binding domains differ in amino acid sequence and respectively bind to different antigenic epitopes on GPRC5D; and the one or more additional binding domains may bind to a second antigen or protein other than GPRC5D.

In some embodiments, the GPRC5D-binding domain is a single domain antibody provided herein; and the one or more additional binding domains may be single domain antibodies, single chain antibodies, or other antigen binding fragments.

In some embodiments, the second antigen is a tumor-associated antigen (TAA) or a tumor microenvironment-associated antigen (TMEAA). In some embodiments, the second antigen is an immunomodulatory antigen, where the antigen is associated with enhancing or inhibiting a signaling pathway in an immune cell. In some embodiments, the second antigen is a T cell surface molecule, such as a component of a T cell receptor complex, e.g., CD3 (including y, δ, ε, ζ and η chains).

Preferably, the multispecific binding peptide is a bispecific antibody molecule.

In some embodiments, the multispecific binding peptide further includes an Fc fragment. The presence of the Fc fragment may facilitate the multimerization of binding domains and may provide associated effector functions.

### Immunoconjugate (or antibody conjugate) comprising GPRC5D binding peptide

The present invention provides a conjugate containing at least one GPRC5D binding peptide provided herein that specifically binds to GPRC5D and one or more other functional portions. The other portions may be a chemical group, for example, a therapeutic agent, such as a cytotoxic agent, or may be a tracer. In some examples, the portion may be a targeting portion, a small molecule drug (e.g., a non-polypeptide drug of less than 500 Da), a toxin, a cytostatic agent, a cytotoxic agent, an immunosuppressant, a radioactive agent suitable for diagnostic purposes, a radioactive metal ion for therapeutic purposes, etc.

In some embodiments, the immunoconjugate is an antibody drug conjugate (ADC) containing one or more GPRC5Dbinding peptides provided herein and a therapeutic agent, which has cytotoxicity, inhibits cell growth, or provides some therapeutic benefits. In some embodiments, the cytotoxic agent is a chemotherapeutic agent, a drug, a growth inhibitor, a toxin (e.g., an enzymatically active toxin of a bacterial, fungal, plant, or animal origin, or a fragment thereof), or a radioactive isotope (i.e., a radioconjugate). In some examples, the antibody drug conjugate provided herein allows for targeted delivery of the drug portion to a tumor. In some cases, this may result in targeted killing of tumor cells.

In some examples, therapeutic agents include, for example, daunomycin, doxorubicin, methotrexate, vindesine, maytansinoid, etc. In some examples, the therapeutic agent has intracellular activity. In some examples, the immunoconjugate that binds to GPRC5D is internalized, and the therapeutic agent has the ability to block cellular protein synthesis activity, block nucleic acid synthesis activity, and cause cell growth arrest or death.

In some embodiments, the immunoconjugate contains one or more GPRC5D binding peptides provided herein and a tracer. The immunoconjugate can be used for research or diagnostic purposes, such as for in vivo detection of cancers. The tracer can produce a detectable signal directly or indirectly. For example, the tracer may be a radioactive isotope, such as ³H, ¹⁴C, ³²P, ³⁵S, or ¹²³I; a fluorescent (fluorophore) or chemiluminescent (chromophore) compound, such as fluorescent isothiocyanate, rhodamine, or fluorescein; an imaging agent; or a metal ion. In some embodiments, the tracer is a radioactive atom such as ⁹⁹Tc or¹²³I for scintigraphic studies, or a spin label such as ⁸⁹Zr, ¹²³I, ¹⁹F, ¹³C, ¹⁵N, or ¹⁷O for nuclear magnetic resonance (NMR) imaging (also known as magnetic resonance imaging, MRI). ⁸⁹Zr can be complexed with a variety of metal chelators and bound to antibodies, for example, for PET imaging.

The linkage between the GPRC5D binding peptide and other functional portions may be covalent or non-covalent. Examples of non-covalent linkage may include via a Biotin-Avidin system. Examples of covalent linkage may include various chemical linkers, including peptide linkers, cleavable linkers, or non-cleavable linkers. Exemplary linker components include 6-maleimidocaproyl (MC), maleimidopropionyl (MP), valine-citrulline (val-cit), alanine-phenylalanine (ala-phe), p-aminobenzyloxycarbonyl (PAB), N-succinimidyl4-(2-pyridylthio) pentanoate (SPP), N-succinimidyl4-(N-maleimidomethyl) cyclohexane-1-carboxylate (SMCC), and N-succinimidyl (4-iodo-acetyl) aminobenzoate (SIAB).

In some embodiments, the linker may comprise amino acid residues. Exemplary amino acid linker components include a dipeptide, a tripeptide, a tetrapeptide, or a pentapeptide. Exemplary dipeptides include: valine-citrulline (vc or val-cit), and alanine-phenylalanine (afa-phe). Exemplary tripeptides include: glycine-valine-citrulline (gly-val-cit) and glycine-glycine-glycine (gly-gly-gly). The amino acid residues containing the amino acid linker component include naturally occurring residues as well as non-naturally occurring amino acid analogs, such as citrulline. The amino acid linker component can be designed and optimized in terms of its selectivity for enzymatic cleavage by specific enzymes, such as tumor-associated proteases, cathepsins B, C and D, and plasmin proteases.

Conjugates of GPRC5D binding peptides with cytotoxic agents can be made using a variety of bifunctional protein coupling agents, such as N-succinimidyl-3-(2-pyridyldithiol)propionate (SPDP), iminothiolane (IT), bifunctional derivatives of imidoesters (such as dimethyl adipimidate HCl), active esters (such as disuccinimidyl substrates), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene2,6-diisocyanate), and bis-active fluorine compounds (such as 1,5-difluoro-2,4-dinitrobenzene).

### Pharmaceutical composition comprising GPRC5D binding peptide, and therapeutic method

The GPRC5D binding peptide disclosed herein and the fusion protein, multispecific antibody molecule, and immunoconjugate comprising the GPRC5D binding peptide (which can be formulated as a pharmaceutical composition together with a pharmaceutically acceptable carrier) can be used for administration to a subject for tumor prevention or treatment. Effective amounts for this use may depend on the severity of the disease and the overall status of the patient's own immune system. The administration scheme will also vary with the disorder and the status of the subject, and will generally range from a single bolus administration or continuous infusion to multiple administrations per day (e.g., every 4-6 hours). A clinician skilled in the art can readily determine whether a subject is a candidate for such treatment, for example, by utilizing clinical trials, physical examination, and the subject's family history.

In some implementations, the GPRC5D binding peptide disclosed herein and the fusion protein, multispecific antibody molecule, and immunoconjugate including the GPRC5D binding peptide can be administered in combination with one or more other drugs (e.g., antineoplastic agents).

In some embodiments, the disease or disorder treated is a tumor, preferably multiple myeloma. The GPRC5D binding peptide disclosed herein (as well as the fusion protein, multispecific antibody molecule, and immunoconjugate including the GPRC5D binding peptide) may be used by any suitable method or approach, and optionally in combination with other antineoplastic agents. Administration routes include, for example, oral, intravenous, intraperitoneal, subcutaneous or intramuscular administration.

The nucleic acid molecule encoding the GPRC5D binding peptide, the expression vector including the nucleic acid molecule, and the host cell transfected with the nucleic acid molecule or the expression vector alone provided herein may also be used for the above-mentioned therapeutic purposes in various ways. For example, the expression vector can be introduced into the subject through gene therapy methods known in the art to express the protein or polypeptide (GPRC5D binding peptide) of interest, thereby achieving the therapeutic purposes.

### Detection kit, diagnostic kit or therapeutic kit including GPRC5D binding peptide

The GPRC5D binding peptide provided herein and other forms of molecules (e.g., fusion proteins or bispecific antibody molecules) including the GPRC5D binding peptide can specifically bind to GPRC5D in a sample. The content (or presence or absence) of GPRC5D in a sample can be conveniently determined by detecting the amount of the formed GPRC5D binding peptide-GPRC5D complex, or the amount of the GPRC5D binding peptide in the formed GPRC5D binding peptide-GPRC5D complex.

As described above, for this purpose, the GPRC5D binding peptide provided herein can be linked to various detection labels to facilitate detection by various means, including but not limited to bioluminescence, fluorescence, radioactive labeling, enzymatic reaction product production, etc. After detecting the content of GPRC5D in the sample, it can be compared with the normal GPRC5D content in the normal population to determine the disease condition or severity of the subject providing the sample. By repeatedly detecting changes in content of GPRC5D over time during the treatment of the subject, it can also be used to determine whether the treatment is effective, thereby providing a basis for changes in the treatment regimen.

The GPRC5D binding peptide provided herein and other forms of molecules including the GPRC5D binding peptide (e.g., fusion proteins or bispecific antibody molecules) can be placed in containers to form a detection kit, diagnostic kit or therapeutic kit. These containers may be in the form of boxes, ampoules, vials, tubes, bags or any suitable containers known in the art. These containers may be made of plastic, glass, laminated paper, metal foil, or other materials suitable for storing drugs. If desired, instructions for use are also provided with the container. The instructions may generally include information about how to use the GPRC5D binding peptide or a composition comprising the GPRC5D binding peptide for treating or preventing tumors (e.g., multiple myeloma), and may include, for example, descriptions of a therapeutic agent (e.g., GPRC5D binding peptide); a dosage regimen for treating or preventing neoplasia (e.g., multiple myeloma); precautions; warnings; indications; contraindications; adverse reactions; animal pharmacology; clinical studies; and/or reference materials. The instructions may be printed directly on the container (if present), or as a label affixed to the container, or as a separate paper, booklet, card, or folded printed matter provided in or with the container.

The GPRC5D binding peptide provided herein (or called the antibody molecule targeting GPRC5D) is a fully humanized single domain antibody, and its binding affinity for GPRC5D is close to that of a control antibody, or better than that of a control antibody.

### Chimeric antigen receptors (CARs)

The chimeric antigen receptor (CAR) provided herein is an artificially constructed chimeric protein, including an extracellular antigen binding domain that specifically binds to GPRC5D, a transmembrane domain, and one or more intracellular signaling domains. These features of the CAR include their ability to redirect T cell specifically and reactively to cells expressing GPRC5D in an MHC-independent manner. MHC-independent GPRC5Drecognition enables T cells or NK cells expressing the disclosed CAR to recognize antigens independent of antigen processing.

The intracellular signaling domain may include a T cell receptor signaling domain, a T cell co-stimulatory signaling domain, or both. The T cell receptor signaling domain may include an intracellular domain of a T cell receptor, such as an intracellular portion of a CD3ζ protein. The co-stimulatory signaling domain is an intracellular domain of a co-stimulatory molecule. Co-stimulatory molecules are cell surface molecules other than antigen receptors or ligands thereof that are required for lymphocytes to effectively respond to antigens.

### 1. Extracellular antigen binding domains

In some embodiments, the CAR provided herein includes an extracellular antigen binding domain that specifically binds to GPRC5D. For example, the antigen binding domain may be scFv, which is a heavy chain variable region and a light chain variable region of any antibody or antigen binding fragment thereof linked by a peptide linker that can specifically bind to GPRC5D. As another example, for constructing a CAR using a single domain antibody that specifically binds to GPRC5D, the extracellular antigen binding domain may include a heavy chain variable region from the single domain antibody, but does not include any light chain variable region.

In some embodiments, the extracellular antigen binding domain of the CAR provided herein includes a heavy chain variable region from clone #18, and the sequences of HCDR1, HCDR2, and HCDR3 of the #18 heavy chain variable region are as follows:
the sequence of HCDR1 is GGSFSGYY (SEQ ID NO:1);
the sequence of HCDR2 is INHSGST (SEQ ID NO: 2); and
the sequence of HCDR3 is ARARRYGGRTRFDP (SEQ ID NO:3).

In some embodiments, the extracellular antigen binding domain of the CAR provided herein comprises a heavy chain variable region from clone #39, and the sequences of HCDR1, HCDR2, and HCDR3 of the above-mentioned #39 heavy chain variable region are as follows:
the sequence of HCDR1 is GFIFSSYG (SEQ ID NO:4);
the sequence of HCDR2 is ISSSGDYT (SEQ ID NO: 5); and
the sequence of HCDR3 is ARMSFRRYDH (SEQ ID NO:6).

In some embodiments, the extracellular antigen binding domain of the CAR provided herein comprises a heavy chain variable region from clone #18, and the sequences of HCDR1, HCDR2, and HCDR3 of the above-mentioned #41 heavy chain variable region are as follows:
the sequence of HCDR1 is GFSFSGYI (SEQ ID NO: 7);
the sequence of HCDR2 is TSSSGTET (SEQ ID NO: 8); and
the sequence of HCDR3 is ARYYSKYGRSYHVDS (SEQ ID NO: 9).

In some embodiments, the extracellular antigen binding domain of the CAR provided herein comprises a heavy chain variable region (SEQ ID NO: 10) from clone #18 or any functional variant thereof.

In other embodiments, the extracellular antigen binding domain of the CAR provided herein comprises a heavy chain variable region (SEQ ID NO: 11) from clone #39 or any functional variant thereof.

In other embodiments, the extracellular antigen binding domain of the CAR provided herein comprises a heavy chain variable region (SEQ ID NO: 12) from clone #41 or any functional variant thereof.

The functional variant herein may include insertion, deletion or replacement of several amino acid residues compared to its parent sequence (the VH amino acid sequence provided herein), and substantially retain the ability to specifically bind to GPRC5D.

In some embodiments, the functional variant may include insertion, deletion or replacement of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more amino acid residues compared to its parent sequence, and also retains at least 50%, 60%, 70%, 80% or 90% of the binding affinity of the parent sequence for GPRC5D, or even greater than that of the parent sequence. It is known to those skilled in the art that there is considerable room for variation in a VH framework region sequence as long as the spatial position of a corresponding CDR sequence is maintained. For example, a framework region sequence in a VH amino acid sequence can be changed, for example, by replacing framework regions of antibody molecules of different species with each other, so as to obtain a functional variant that substantially retains the antigen binding ability. In addition, functional variants that still have the antigen binding ability can be obtained by changing a few residues in the CDR sequences, such as 1, 2 or 3 amino acid residues, and detecting the binding ability of the modified antibody molecule to the corresponding antigen.

Conservative amino acid replacements for amino acids with similar functions are well known to those of ordinary skill in the art. The following six groups are examples of amino acids that are considered conservative replacements for each other:
1) alanine (A), serine (S), and threonine (T);
2) aspartic acid (D), and glutamic acid (E);
3) asparagine (N), and glutamine (Q);
4) arginine (R), and lysine (K);
5) isoleucine (I), leucine (L), methionine (M), and valine (V); and
6) phenylalanine (F), tyrosine (Y), and tryptophan (W).

The extracellular antigen binding domain of the CAR provided herein may include two or more V_{HH} fragments described above that have a GPRC5D binding ability. They may be linked in series directly or via a peptide linker. Using two or more V_{HH} fragments linked in series as the extracellular antigen binding domain or part thereof helps to promote the recognition and binding of the constructed CAR to a target molecule (e.g., GPRC5D). Preferably, these VH fragments respectively bind to different epitopes on GPRC5D.

The CAR may comprise a signal peptide sequence, for example, located at the N-terminus of the antigen binding domain. Any suitable signal peptide sequence may be used. In one embodiment, the signal peptide sequence is a CD8αsignal peptide Although the signal peptide sequence can promote the expression of the CAR on the cell surface, the presence of the signal peptide sequence in the expressed CAR is not essential for the function of the CAR. After the CAR is expressed on the cell surface, the signal peptide sequence may be cleaved from the CAR. Thus, in some embodiments, the CAR lacks a signal peptide sequence.

Between the antigen binding domain and the transmembrane domain of the CAR, there may be a hinge region (or spacer domain) that includes a polypeptide sequence. The hinge region may comprise up to 300 amino acids, preferably 10 to 100 amino acids. In one embodiment, the CAR provided herein includes a CD8αprotein hinge region.

### 2. Transmembrane domains

The CAR may include a transmembrane domain fused to the extracellular domain of the CAR. In one embodiment, a transmembrane domain that is naturally associated with other elements in the CAR (such as a cross-linking region or an intracellular signaling domain) is used.

The transmembrane domain may be derived from natural or synthetic sources. In a case that the source is natural, the domain may be derived from any membrane-bound or transmembrane protein. Exemplary transmembrane domains for use in the disclosed CAR may include at least transmembrane domains of a α, β or ζ chain of a T cell receptor, CD28, CD3ε, CD45, CD4, CD5, CDS, CD9, CD16, CD22, CD33, CD37, CD64, CD80, CD86, CD134, CD137, and CD154. Alternatively, the transmembrane domain may be synthetic, in which case it will mainly comprise hydrophobic residues such as leucine and valine. In some embodiments, a triplet of phenylalanine, tryptophan, and valine will appear at each end of the synthetic transmembrane domain. Optionally, a short oligo- or polypeptide linker, preferably 2 to 10 amino acids in length, can form a linkage between the transmembrane domain and the intracellular T cell signaling domain and/or T cell co-stimulatory domain of the CAR. The exemplary linker sequence includes one or more glycine-serine doublets.

In some embodiments, the transmembrane domain includes a transmembrane domain of a T cell receptor, such as a CD8α transmembrane domain (SEQ ID NO:21).

### 3. Intracellular domains

An intracellular region of the CAR includes one or more intracellular T cell signaling domains responsible for activating at least one normal effector function of the T cell expressing the CAR. Exemplary T cell signaling domains are provided herein and are known to those of ordinary skill in the art.

Although the entire intracellular T cell signaling domain can be used in the CAR, in many cases, it is not necessary to use the entire chain. To the extent that a truncated portion of an intracellular T cell signaling domain is used, the truncated portion may be used in place of the intact chain as long as it transduces relevant T cell effector function signals.

Examples of intracellular T cell signaling domains used in the CAR include the cytoplasmic sequences of the T cell receptor (TCR) and co-stimulatory molecules that act synergistically to initiate signal transduction following antigen receptor binding, as well as any derivatives or variants of these sequences and any synthetic sequences having the same function.

The T cell receptor signaling domain regulates the activation of the T cell receptor complex in a stimulatory or inhibitory manner. The CAR disclosed herein may comprise a cytoplasmic signaling sequence that acts in a stimulatory manner, which may contain a signaling motif known as an immunoreceptor tyrosine-based activation motif or ITAM. Examples of primary cytoplasmic ITAM-containing signaling sequences that may be comprised in the disclosed CAR include intracellular domains from CD3ζ, FcRy, FcRβ, CD3γ, CD3δ, CD3ε, CDS, CD22, CD79a, CD79b, and CD66d proteins. In some embodiments, the cytoplasmic signaling molecule in the CAR includes an intracellular T cell signaling domain from CD3ζ.

The intracellular domain of the CAR provided herein may include a CD3ζ chain portion and an intracellular co-stimulatory signaling domain. The co-stimulatory signaling domain may include an intracellular domain of a co-stimulatory molecule. Co-stimulatory molecules are cell surface molecules other than antigen receptors or ligands thereof that are required for lymphocytes to effectively respond to antigens. Examples of such molecules include CD27, CD28, 4-1BB (CD137), OX40 (CD134), CD30, CD40, PD-1, ICOS, lymphocyte function-associated antigen 1 (LFA-1), CD2, CD7, LIGHT, NKG2C, and B7-H3.

In some embodiments, the CAR may include a CD3ζ signaling domain, a CD8 signaling domain, a CD28 signaling domain, a 4-1BB signaling domain, or a combination of two or more thereof. In one embodiment, an intracellular domain includes a signaling domain of CD3-ζ and a signaling domain of CD28. In another embodiment, a cytoplasmic domain includes a signaling domain of CD3ζ and a signaling domain of 4-1BB. In still another embodiment, a cytoplasmic domain includes a signaling domain of CD3-ζ and signaling domains of CD28 and CD137. One of ordinary skill in the art can change the order of one or more T cell signaling domains on the CAR as needed. Cytoplasmic signaling sequences within a cytoplasmic signaling portion of the CAR of the present invention may be linked to each other in a random or specified order. Optionally, a short polypeptide linker, preferably between 2 and 10 amino acids in length, may form the linkage. Glycine-serine doublets provide particularly suitable linkers. In addition, between the signaling domain and the transmembrane domain of the CAR, there may be a spacer domain comprising a polypeptide sequence. The spacer domain may comprise up to 300 amino acids, preferably 10 to 100 amino acids, most preferably 25 to 50amino acids.

### 4. Additional description of CARs

Based on the CAR provided herein (as a parent CAR), those skilled in the art can modify it, for example, by fusing it with other polypeptides, or retaining only a fragment of the CAR provided herein, and these fusion molecules or fragments have at least part of the biological activity of the parent CAR, such as identifying target cells (e.g., tumor cells expressing GPRC5D) or detecting, treating or preventing disease activity.

The CAR provided herein may also comprise additional amino acids at the amino or carboxyl terminus or at both termini, and there additional amino acids are not present in the amino acid sequence of the parent CAR. Ideally, the additional amino acids do not interfere with the biological function of the CAR or the functional portion, such as recognizing target cells, detecting cancers, or treating or preventing cancers. More ideally, the additional amino acids enhance the biological activity as compared to the biological activity of the parent CAR.

Also provided is a functional variant of the CAR described herein, which has substantial or significant sequence identity or similarity to the parent CAR. The functional variant retains the biological activity as a variant of the CAR. Functional variants encompass those variants of, for example, the CAR described herein (parent CAR) that retain the ability to recognize target cells to a similar degree, the same degree, or a higher degree than the parent CAR. With reference to the parent CAR, the functional variant may, for example, have at least about 30%, about 50%, about 75%, about 80%, about 85%, about 90%, about 91%, about 92%, about 93%, about 94%, about 95%, about 96%, about 97%, about 98%, about 99% or higher identity in the amino acid sequence to the parent CAR. The functional variant may, for example, comprise an amino acid sequence of a parent CAR with at least one conservative amino acid replacement. Alternatively or additionally, the functional variant may comprise an amino acid sequence of a parent CAR with at least one non-conservative amino acid replacement. In this case, it is preferred that the non-conservative amino acid replacement does not interfere with or inhibit the biological activity of the functional variant. The non-conservative amino acid replacement can enhance the biological activity of the functional variant, such that the biological activity of the functional variant is increased compared to that of the parent CAR.

The CAR provided herein may comprise synthetic amino acids to replace one or more naturally occurring amino acids. Such synthetic amino acids are known in the art and include, for example, aminocyclohexanecarboxylic acid, norleucine, a-amino n-decanoic acid, homoserine, S-acetylaminomethyl-cysteine, trans-3- and trans-4-hydroxyproline, 4-aminophenylalanine, 4-nitrophenylalanine, 4-chlorophenylalanine, 4-carboxyphenylalanine, β-phenylserine, β-hydroxyphenylalanine, phenylglycine, α-naphthylalanine, cyclohexylalanine, cyclohexylglycine, dihydroindole-2-carboxylic acid, 1,2,3,4-tetrahydroisoquinoline-3-carboxylic acid, aminomalonic acid, aminomalonic acid monoamide, N'-benzyl-N'-methyl-lysine, N',N'-dibenzyl-lysine, 6-hydroxylysine, ornithine, α-aminocyclopentanecarboxylic acid, α-aminocyclohexanecarboxylic acid, oc-aminocycloheptanecarboxylic acid, -(2-amino-2-norbornane)-carboxylic acid, γ-diaminobutyric acid, α,β-diaminopropionic acid, homophenylalanine and α-tert-butylglycine, etc.

The CAR provided herein can be glycosylated, amidated, carboxylated, phosphorylated, esterified, N-acylated, cyclized via, for example, a disulfide bond, or converted into an acid addition salt and/or optionally dimerized or multimerized, or conjugated.

Methods for generating chimeric antigen receptors, T cells including such receptors, and uses thereof (e.g., for treating cancers) are known in the art. For example, a nucleic acid molecule encoding the disclosed chimeric antigen binding receptor may be included in an expression vector (e.g., a lentiviral vector) for expression in a host cell, such as a T cell, so as to prepare the disclosed CAR. In some embodiments, a method of using a chimeric antigen receptor includes isolating T cells from a subject, transforming the T cells with an expression vector (e.g., a lentiviral vector) encoding the chimeric antigen receptor, and administering the engineered T cells expressing the chimeric antigen receptor to the subject for treatment, such as treatment of tumors in the subject.

### Nucleic acid molecules and expression

The present invention provides coding nucleic acid sequences encoding various elements for constructing a CAR. These nucleic acid sequences can be prepared by any suitable method including, for example, cloning, amplification, or direct chemical synthesis. Chemical synthesis produces single-stranded oligonucleotides. This can be converted into double-stranded DNA by hybridization with a complementary sequence or by polymerization with a DNA polymerase using a single strand as a template. Technicians will recognize that although chemical synthesis of DNA is generally limited to sequences of about 100 bases, longer sequences can be obtained by linking shorter sequences.

Exemplary nucleic acids can be prepared by cloning techniques. Examples of suitable cloning and sequencing techniques as well as instructions sufficient to guide technicians through many cloning exercises are known. Nucleic acids can also be prepared by amplification methods. Amplification methods include a polymerase chain reaction (PCR), a ligase chain reaction (LCR), a transcription-based amplification system (TAS), and a self-sustained sequence replication system (3SR). A variety of cloning methods, host cells, and in vitro amplification methods are well known to those skilled in the art.

In some embodiments, the nucleic acid molecule encodes the CAR provided herein of T cells for expression in a T cell to generate a chimeric antigen receptor. Nucleic acid molecules encoding chimeric antigen binding receptors can be contained in a vector (e.g., a lentiviral vector) for expression in a host cell, such as a T cell. Exemplary cells include T cells, natural killer (NK) cells, cytotoxic T lymphocytes (CTLs), and regulatory T cells.

Nucleic acid molecules can be expressed in recombinant engineered cells such as bacterial, plant, yeast, insect and mammalian cells. Antibodies and antigen binding fragments can be expressed as V_{HH} alone (for single domain antibodies), or V_{H} and V_{L} chains, or can be expressed as fusion proteins.

To produce scFv, DNA fragments encoding V_{H} and V_{L} can be operably linked to another fragment encoding a flexible linker, for example, a fragment encoding an amino acid sequence (Gly₄-Ser)₃, so that V_{H} and V_{L} sequences can be expressed as a continuous single-chain protein with V_{L} and V_{H} domains linked via the flexible linker.

Those skilled in the art are familiar with numerous expression systems that can be used to express proteins, including Escherichia coli (*E. coli*), other bacterial hosts, yeast, and various higher eukaryotic cells, such as COS, CHO, HeLa, and myeloma cell lines.

To produce the CAR, the host cell is preferably a mammalian cell, such as a human cell. In some embodiments, the host cell may be a peripheral blood lymphocyte (PBL) or a peripheral blood mononuclear cell (PBMC), or a T cell. The T cell may be any T cell, such as a cultured T cell, such as a primary T cell, or a T cell from a cultured T cell line, such as Jurkat or SupTl, or a T cell obtained from a mammal. If obtained from mammals, T cells may be obtained from a variety of sources including, but not limited to, blood, bone marrow, lymph nodes, thymuses, or other tissues or body fluids. T cells may also be enriched or purified. T cells may be human T cells. T cells may be T cells isolated from humans. T cells may be any type of T cells and may be at any developmental stage, including but not limited to CD4⁺/CD8⁺ double positive T cells, CD4⁺ helper T cells, such as Th₁ and Th₂ cells, CD8⁺T cells (e.g., cytotoxic T cells), tumor infiltrating cells, memory T cells, naive T cells, etc. T cells may be CD8⁺ T cells or CD4⁺ T cells.

The nucleic acids encoding the CAR described herein or elements thereof can be modified without reducing their biological activity. For example, some modifications may be made to facilitate cloning or expression. Such modifications are well known to those skilled in the art and include, for example, a stop codon, a methionine added at the amino terminus to provide a start site, and additional amino acids (such as poly-histidine) at any terminus to create conveniently located restriction sites to facilitate the purification step. In addition to a recombinant method, the CAR of the present disclosure or elements thereof can also be constructed in whole or in part using standard peptide synthesis well known in the art.

### Pharmaceutical compositions and medical applications

The CAR provided herein or the host cell expressing the CAR (such as a T cell or an NK cell) can specifically recognize and bind to a target cell expressing GPRC5D, such as a tumor cell.

Therefore, in some embodiments, the present invention provides a pharmaceutical composition for preventing or treating diseases associated with GPRC5D expression, comprising a host cell expressing the CAR provided herein, especially a CAR-T cell, and a pharmaceutically acceptable carrier. These pharmaceutical compositions can be formulated into dosage forms suitable for administration mode, such as injections.

In other embodiments, provided herein is a method for treating diseases associated with GPRC5D expression, including administering an effective amount of CAR-T cells provided herein or the pharmaceutical composition comprising CAR-T cells to a subject in need thereof. In some embodiments, the administration mode is intravenous administration, such as intravenous injection; intramuscular administration, such as intramuscular injection; or in situ administration to the tumor site.

In other embodiments, provided herein is a use of the above-mentioned CAR-T cells in the preparation of drugs for treating diseases associated with GPRC5D expression.

In some embodiments, the disease associated with GPRC5D expression is a tumor, such as a plasma cell malignancy disease (e.g., multiple myeloma), or a B cell malignant disease (e.g., Hodgkin's lymphoma or non-Hodgkin's lymphoma); or an autoimmune disease.

In some embodiments, the CAR-T cells or pharmaceutical compositions herein can be administered to a subject to prevent or treat tumors. In these applications, a therapeutically effective amount of CAR-T cells or pharmaceutical compositions targeting target cells expressing GPRC5D are administered to a subject, thereby slowing or inhibiting tumor growth or metastasis, reducing tumor volume, or inhibiting signs or symptoms of cancers. The therapeutically effective amount will depend on the severity of a disease and the general health condition of a patient. The therapeutically effective amount is an amount which provides either subjective relief of symptoms or an objectively identifiable improvement as noted by clinicians or other qualified observers. In one embodiment, the therapeutically effective amount is an amount necessary to inhibit tumor growth, inhibit metastasis and reduce tumor volume, or an amount effective to reduce signs or symptoms of tumors. The therapeutically effective amount of the agent administered may vary depending on the desired effect and the subject to be treated. In some examples, the therapeutic amount is an amount that eliminates or reduces the patient's tumor burden, or prevents or reduces proliferation of metastatic cells, or alleviates tumor symptoms. Any administration method can be used for the disclosed CAR-T cells or pharmaceutical compositions, including local and systemic administration. For example, topical, oral, intravascular (e.g., intravenous), intramuscular, intraperitoneal, intranasal, intradermal, intrathecal, and subcutaneous administration can be used. The specific administration mode and administration scheme will be selected by the attending clinician, taking into account the specific circumstances of a case (e.g., the subject, the disease, the disease status involved, and whether the treatment is prophylactic).

Administration of the CAR-T cells or pharmaceutical compositions provided herein can be accompanied by administration of other anti-cancer or anti-angiogenic agents or therapeutic treatments (e.g., surgical resection or radiotherapy of tumors). For example, a subject can receive one or more additional therapies or therapeutic agents before or after administration of a therapeutically effective amount of CAR-T cells. For example, the additional therapy may include, but is not limited to, the use of chemotherapeutic agents, anti-angiogenic agents, or a combination thereof. In another example, prior to administering an effective amount of CAR-T cells or pharmaceutical compositions provided herein, at least a part of the tumor is removed or the size or volume of the tumor is reduced by surgeries or other methods.

Specific examples of additional therapeutic agents that can be used include microtubule binding agents, DNA intercalators or cross-linking agents, DNA synthesis inhibitors, DNA and RNA transcription inhibitors, antibodies, enzymes, enzyme inhibitors, gene modulators, and angiogenesis inhibitors. These agents (administered in therapeutically effective amounts) and treatments may be used alone or in combination. For example, any suitable anti-cancer agent or anti-angiogenic agent can be administered in combination with the CAR-T cells or pharmaceutical compositions disclosed herein.

### Research overview

We used a large-capacity phage antibody library to screen fully humanized GPRC5D-specific antibodies, and assessed the specificity of these antibodies at the phage level and protein level through enzyme linked immunosorbent assay (ELISA) and fluorescence activated cell sorting (FACS) experiments. We then screened the affinity of the antibody binding to cells through the FACS assay, and constructed these antibody molecules into second-generation CAR lentiviral expression vectors, transfected T cells, and preliminarily evaluated the killing functions of these antibody molecules through in vitro cell killing assays. Finally, we obtained three fully humanized antibody clones with good specificity and strong killing functions.

We used different antibody libraries and picked 768 monoclones in total for enzyme linked immunosorbent assay (ELISA) and flow cytometry (FACS) preliminary screening after recombinant GPRC5D protein panning and GPRC5D protein/cell (CHO-K1-GPRC5D) panning, where 216 clones specifically bound to GPRC5D-VLP protein and GPRC5D-expressing positive cells CHO-K1-GPRC5D, but did not bind to control protein VLP and GPRC5D-expressing negative cells CHO-K1. After sequencing, 35different monoclonal sequences were obtained. Subsequently, we identified these 35 antibodies by flow cytometric analysis (FACS) with various GPRC5D-positive (CHO-K1-GPRC5D, MM1S) and negative cell lines (CHO-K1, Jurkat), and by enzyme linked immunosorbent assay (ELISA) with various non-related antigens (VLP, BAFFR-his-Bio, IL10-Bio, SA) and GPRC5D protein (GPRC5D-VLP), where 3 clones showed good binding and specificity on multiple cell lines and multiple protein antigens. The acquisition of these clones lays a foundation for the subsequent development of fully humanized GPRC5D CAR-T products or antibody drugs.

The present invention uses a fully humanized phage library to perform antibody screening to directly obtain fully humanized monoclonal antibodies. Compared with the traditional hybridoma technology, the difficult step of humanizing murine-derived antibodies is omitted, and fully humanized antibodies have lower immunogenicity than humanized murine-derived antibodies, and have better potential in the development of antibody drugs or CAR-T.

Antibody clones that specifically bind to the cell surface GPRC5D antigen (including single domain antibodies #18, #39, #41, etc.) were obtained through the fully humanized phage library. These clones and the control antibody benchmark 1 (the variable region sequence comes from GC5B596 of Chinese patent application publication CN 109715667 A) were then constructed onto the second-generation CAR structure, packaged with a lentivirus, and transfected into T cells. At the CAR-T cell level, fully humanized GPRC5D antibody clones and candidate CAR-T molecules with stronger functions than the control benchmark 1 were screened from the perspectives of target cell activation and killing, target cell stimulation and proliferation, etc.

In the development process, through phage-level antibody screening/specificity identification, specific antibody clones are screened quickly and efficiently, and then directly linked to CAR-T functional testing to select the best candidate antibodies, thereby optimizing the antibody screening process for the purpose of CAR-T development, and improving the research and development efficiency while ensuring the research quality.

The following table lists the amino acid sequences and coding sequences of single domain antibody heavy chain variable region sequences that were specifically studied and other elements used for CAR construction.

| Content | Sequence | SEQ ID NO: |
|---|---|---|
| #18 CDR1 | GGSFSGYY | 1 |
| #18 CDR2 | INHSGST | 2 |
| #18 CDR3 | ARARRYGGRTRFDP | 3 |
| #39 CDR1 | GFIFSSYG | 4 |
| #39 CDR2 | ISSSGDYT | 5 |
| #39 CDR3 | ARMSFRRYDH | 6 |
| #41 CDR1 | GFSFSGYI | 7 |
| #41 CDR2 | TSSSGTET | 8 |
| #41 CDR3 | ARYYSKYGRSYHVDS | 9 |
| #18 Heavy chain variable region Amino acid sequence | QVQLQQWGAGLLKPSETLSLTCAVYGGSFSGYYWSWIR QPPGKGLEWIGEINHSGSTNYNPSLKSRVTISVDTSKNQF SLKLSSVTAADTAVYYCARARRYGGRTRFDPWGQGTL VTVSS | 10 |
| #39 Heavy chain variable region Amino acid sequence | EVQLLESGGGLVQPGGSLRLSCAASGFIFSSYGMGWVR QAPGKGLEWVSSISSSGDYTYYADSVKGRFTISRDNSKN TLYLQMNSLRAEDTAVYYCARMSFRRYDHWGQGTLVT VSS | 11 |
| #41 Heavy chain variable region Amino acid sequence | EVQLLESGGGLVQPGGSLRLSCAASGFSFSGYIMGWVR QAPGKGLEWVSSTSSSGTETYYADSVKGRFTISRDNSKN TLYLQMNSLRAEDTAVYYCARYYSKYGRSYHVDSWGQ GTLVTVSS | 12 |
| #18 Heavy chain variable region Coding sequence | CAGGTGCAGCTACAGCAGTGGGGCGCAGGACTGTTGA AGCCTTCGGAGACCCTGTCCCTCACCTGCGCTGTCTAT GGTGGGTCCTTCAGTGGTTACTACTGGAGCTGGATCCG CCAGCCCCCAGGGAAGGGGCTGGAGTGGATTGGGGAA ATCAATCATAGTGGAAGCACCAACTACAACCCGTCCC TCAAGAGTCGAGTCACCATATCAGTAGACACGTCCAA GAACCAGTTCTCCCTGAAGCTGAGCTCTGTGACCGCCG CGGACACGGCTGTGTATTACTGTGCGAGGGCCCGACG CTACGGTGGCCGAACCAGGTTCGACCCCTGGGGCCAG | 13 |
| | GGAACCCTGGTCACCGTCTCCTCA | |
| #39 Heavy chain variable region Coding sequence | GAAGTTCAGCTGCTGGAAAGCGGTGGTGGTCTGGTTC AGCCTGGTGGTAGCCTGCGTCTGAGCTGTGCAGCAAG CGGTTTTATCTTTAGCAGCTATGGCATGGGTTGGGTTC GTCAGGCACCTGGTAAAGGTCTGGAATGGGTTAGCAG CATCAGCAGCAGCGGCGATTATACCTATTATGCAGAT AGCGTTAAAGGTCGTTTTACCATTAGCCGTGATAACAG CAAAAATACCCTGTACCTGCAGATGAATAGTCTGCGT GCAGAGGATACCGCAGTGTATTATTGTGCGCGCATGT CTTTCCGTCGTTACGATCATTGGGGTCAAGGTACTCTG GTGACCGTCTCCTCA | 14 |
| #41 Heavy chain variable region Coding sequence | GAAGTTCAGCTGCTGGAAAGCGGTGGTGGTCTGGTTC AGCCTGGTGGTAGCCTGCGTCTGAGCTGTGCAGCAAG CGGTTTTAGCTTTAGCGGCTATATCATGGGTTGGGTTC GTCAGGCACCTGGTAAAGGTCTGGAATGGGTTAGCAG CACCAGCAGCAGCGGCACCGAAACCTATTATGCAGAT AGCGTTAAAGGTCGTTTTACCATTAGCCGTGATAACAG CAAAAATACCCTGTACCTGCAGATGAATAGTCTGCGT GCAGAGGATACCGCAGTGTATTATTGTGCGCGCTACT ACTCTAAATACGGTCGTTCTTACCATGTTGATTCTTGG GGTCAAGGTACTCTGGTGACCGTCTCCTCA | 15 |
| CD8α Signal peptide Coding nucleic acid sequence 63 bp | ATGGCCCTGCCTGTGACAGCTCTGCTCCTCCCTCTGGC CCTGCTGCTCCATGCCGCCAGACCC | 16 |
| CD8α Signal peptide Protein sequence 21 aa | MALPVTALLLPLALLLHAARP | 17 |
| CD8α Hinge region Nucleic acid sequence 165 bp | TTCGTGCCCGTGTTCCTGCCCGCCAAACCTACTACTAC CCCTGCACCTAGGCCTCCCACCCCAGCCCCAACAATCG CCAGCCAGCCTCTGTCTCTGCGGCCCGAAGCCTGTAGA CCTGCTGCCGGCGGAGCCGTGCACACCAGAGGCCTGG ACTTCGCCTGCGAC | 18 |
| CD8α Hinge region Protein sequence 55 aa | FVPVFLPAKPTTTPAPRPPTPAPTIASQPLSLRPEACRPAA GGAVHTRGLDFACD | 19 |
| CD8α Transmembrane region Nucleic acid sequence 84 bp | ATCTACATCTGGGCCCCTCTGGCCGGCACCTGTGGCGT GCTGCTGCTGAGCCTGGTGATCACCCTGTACTGCAACC ACCGGAAC | 20 |
| CD8α Transmembrane region Protein sequence 28 aa | IYIWAPLAGTCGVLLLSLVITLYCNHRN | 21 |
| 4-1BB Intracellular domain Nucleic acid | AAACGGGGCAGAAAGAAACTCCTGTATATATTCAAAC AACCATTTATGAGACCAGTACAAACTACTCAAGAGGA | 22 |
| sequence 126 bp | AGATGGCTGTAGCTGCCGATTTCCAGAAGAAGAAGAA GGAGGATGTGAACTG | |
| 4-1BB Intracellular domain Protein sequence 42 aa | KRGRKKLLYIFKQPFMRPVQTTQEEDGCSCRFPEEEEGG CEL | 23 |
| CD3ζ Intracellular signaling domain Nucleic acid sequence 336 bp | AGAGTGAAGTTCAGCAGATCCGCCGACGCCCCTGCCT ACCAGCAGGGACAGAACCAGCTGTACAACGAGCTGAA CCTGGGCAGACGGGAAGAGTACGACGTGCTGGACAAG CGGAGAGGCCGGGACCCCGAGATGGGCGGAAAGCCC AGACGGAAGAACCCCCAGGAAGGCCTGTATAACGAAC TGCAGAAAGACAAGATGGCCGAGGCCTACAGCGAGAT CGGCATGAAGGGCGAGCGGAGGCGCGGCAAGGGCCA CGATGGCCTGTACCAGGGCCTGAGCACCGCCACCAAG GACACCTACGACGCCCTGCACATGCAGGCCCTGCCCC CCAGA | 24 |
| CD3ζ Intracellular signaling domain Protein sequence 112 aa | RVKFSRSADAPAYQQGQNQLYNELNLGRREEYDVLDKR RGRDPEMGGKPRRKNPQEGLYNELQKDKMAEAYSEIG MKGERRRGKGHDGLYQGLSTATKDTYDALHMQALPPR | 25 |
| Cleaving peptide T2A Nucleic acid sequence 54 bp | GAGGGAAGGGGCAGCTTATTAACATGTGGCGATGTGG AAGAGAACCCCGGTCCC | 26 |
| Cleaving peptide T2A Protein sequence 18 aa | EGRGSLLTCGDVEENPGP | 27 |
| CSF2RA signal Nucleic acid sequence 66 bp | ATGCTGCTGCTCGTGACCTCTTTACTGTTATGTGAGCT GCCCCACCCCGCTTTTTTACTGATCCCT | 28 |
| CSF2RA signal Protein sequence 22 aa | MLLLVTSLLLCELPHPAFLLIP | 29 |
| EGFRt Nucleic acid sequence 1005 bp | CGTAAGGTGTGTAACGGAATCGGCATTGGCGAGTTCA AGGACTCTTTAAGCATCAACGCCACAAACATCAAGCA CTTCAAGAATTGTACCTCCATCAGCGGCGATTTACACA TTCTCCCCGTGGCTTTTCGTGGCGATTCCTTCACCCAC ACCCCCCCTCTGGACCCCCAAGAGCTGGACATTTTAAA AACCGTGAAGGAGATCACCGGCTTTCTGCTGATCCAA GCTTGGCCCGAGAATCGTACCGACCTCCACGCCTTCGA GAATTTAGAGATTATTCGTGGAAGGACCAAGCAGCAC GGCCAGTTCTCTTTAGCCGTCGTGTCTTTAAACATTAC CAGCCTCGGTTTAAGGTCTTTAAAGGAGATTAGCGAC GGCGACGTGATCATCTCCGGCAACAAGAACCTCTGCT ACGCCAACACCATCAACTGGAAGAAGCTGTTCGGAAC CAGCGGCCAAAAGACCAAGATCATCAGCAATCGTGGA GAGAACTCTTGTAAGGCCACTGGTCAAGTTTGCCACG | 30 |
| | CCCTCTGTAGCCCCGAAGGATGTTGGGGCCCCGAGCC TAGGGACTGTGTTAGCTGCAGAAACGTGAGCAGAGGC AGAGAGTGTGTGGACAAATGCAATTTACTGGAAGGAG AGCCTAGGGAGTTCGTGGAGAACAGCGAATGTATCCA GTGCCACCCCGAGTGTTTACCTCAAGCCATGAACATCA CTTGTACCGGAAGGGGCCCCGATAACTGCATCCAATG CGCCCACTACATCGACGGACCCCACTGCGTGAAAACT TGTCCCGCCGGAGTGATGGGAGAGAATAACACTTTAG TGTGGAAGTACGCCGACGCTGGCCACGTCTGCCATCT GTGCCACCCCAACTGTACCTACGGCTGCACTGGTCCCG GTTTAGAGGGATGTCCTACCAACGGCCCCAAGATCCC CTCCATCGCCACCGGAATGGTGGGCGCTCTGTTATTAC TGCTGGTGGTGGCTCTGGGCATCGGTTTATTCATCATG | |
| EGFRt Protein sequence 335 aa | RKVCNGIGIGEFKDSLSINATNIKHFKNCTSISGDLHILPV AFRGDSFTHTPPLDPQELDILKTVKEITGFLLIQAWPENRT DLHAFENLEIIRGRTKQHGQFSLAVVSLNITSLGLRSLKEI SDGDVIISGNKNLCYANTINWKKLFGTSGQKTKIISNRGE NSCKATGQVCHALCSPEGCWGPEPRDCVSCRNVSRGRE CVDKCNLLEGEPREFVENSECIQCHPECLPQAMNITCTGR GPDNCIQCAHYIDGPHCVKTCPAGVMGENNTLVWKYA DAGHVCHLCHPNCTYGCTGPGLEGCPTNGPKIPSIATGM VGALLLLLVVALGIGLFM | 31 |
| P2A Nucleic acid sequence 57 bp | gctactaacttcagcctgctgaagcaggctggtgacgtcgaggagaatcctggcccc | 32 |
| P2A Protein sequence 19 aa | ATNFSLLKQAGDVEENPGP | 33 |
| GPRC5D CAR 2290(#18) Coding nucleic acid sequence 1134 bp | ATGGCCCTGCCTGTGACAGCTCTGCTCCTCCCTCTGGC CCTGCTGCTCCATGCCGCCAGACCCCAGGTGCAGCTAC AGCAGTGGGGCGCAGGACTGTTGAAGCCTTCGGAGAC CCTGTCCCTCACCTGCGCTGTCTATGGTGGGTCCTTCA GTGGTTACTACTGGAGCTGGATCCGCCAGCCCCCAGG GAAGGGGCTGGAGTGGATTGGGGAAATCAATCATAGT GGAAGCACCAACTACAACCCGTCCCTCAAGAGTCGAG TCACCATATCAGTAGACACGTCCAAGAACCAGTTCTCC CTGAAGCTGAGCTCTGTGACCGCCGCGGACACGGCTG TGTATTACTGTGCGAGGGCCCGACGCTACGGTGGCCG AACCAGGTTCGACCCCTGGGGCCAGGGAACCCTGGTC ACCGTCTCCTCATTCGTGCCCGTGTTCCTGCCCGCCAA ACCTACTACTACCCCTGCACCTAGGCCTCCCACCCCAG CCCCAACAATCGCCAGCCAGCCTCTGTCTCTGCGGCCC GAAGCCTGTAGACCTGCTGCCGGCGGAGCCGTGCACA CCAGAGGCCTGGACTTCGCCTGCGACATCTACATCTGG | 34 |
| | GCCCCTCTGGCCGGCACCTGTGGCGTGCTGCTGCTGAG CCTGGTGATCACCCTGTACTGCAACCACCGGAACAAA CGGGGCAGAAAGAAACTCCTGTATATATTCAAACAAC CATTTATGAGACCAGTACAAACTACTCAAGAGGAAGA TGGCTGTAGCTGCCGATTTCCAGAAGAAGAAGAAGGA GGATGTGAACTGAGAGTGAAGTTCAGCAGATCCGCCG ACGCCCCTGCCTACCAGCAGGGACAGAACCAGCTGTA CAACGAGCTGAACCTGGGCAGACGGGAAGAGTACGA CGTGCTGGACAAGCGGAGAGGCCGGGACCCCGAGATG GGCGGAAAGCCCAGACGGAAGAACCCCCAGGAAGGC CTGTATAACGAACTGCAGAAAGACAAGATGGCCGAGG CCTACAGCGAGATCGGCATGAAGGGCGAGCGGAGGC GCGGCAAGGGCCACGATGGCCTGTACCAGGGCCTGAG CACCGCCACCAAGGACACCTACGACGCCCTGCACATG CAGGCCCTGCCCCCCAGA | |
| GPRC5D CAR 2290 (#18) Amino acid sequence 378aa | MALPVTALLLPLALLLHAARPQVQLQQWGAGLLKPSET LSLTCAVYGGSFSGYYWSWIRQPPGKGLEWIGEINHSGS TNYNPSLKSRVTISVDTSKNQFSLKLSSVTAADTAVYYC ARARRYGGRTRFDPWGQGTLVTVSSFVPVFLPAKPTTTP APRPPTPAPTIASQPLSLRPEACRPAAGGAVHTRGLDFAC DIYIWAPLAGTCGVLLLSLVITLYCNHRNKRGRKKLLYIF KQPFMRPVQTTQEEDGCSCRFPEEEEGGCELRVKFSRSA DAPAYQQGQNQLYNELNLGRREEYDVLDKRRGRDPEM GGKPRRKNPQEGLYNELQKDKMAEAYSEIGMKGERRRG KGHDGLYQGLSTATKDTYDALHMQALPPR | 35 |
| GPRC5D CAR 2436 (#39) Coding nucleic acid sequence 1125 bp | ATGGCCCTGCCTGTGACAGCTCTGCTCCTCCCTCTGGC CCTGCTGCTCCATGCCGCCAGACCCGAAGTTCAGCTGC TGGAAAGCGGTGGTGGTCTGGTTCAGCCTGGTGGTAG CCTGCGTCTGAGCTGTGCAGCAAGCGGTTTTATCTTTA GCAGCTATGGCATGGGTTGGGTTCGTCAGGCACCTGG TAAAGGTCTGGAATGGGTTAGCAGCATCAGCAGCAGC GGCGATTATACCTATTATGCAGATAGCGTTAAAGGTC GTTTTACCATTAGCCGTGATAACAGCAAAAATACCCTG TACCTGCAGATGAATAGTCTGCGTGCAGAGGATACCG CAGTGTATTATTGTGCGCGCATGTCTTTCCGTCGTTAC GATCATTGGGGTCAAGGTACTCTGGTGACCGTCTCCTC ATTCGTGCCCGTGTTCCTGCCCGCCAAACCTACTACTA CCCCTGCACCTAGGCCTCCCACCCCAGCCCCAACAATC GCCAGCCAGCCTCTGTCTCTGCGGCCCGAAGCCTGTAG ACCTGCTGCCGGCGGAGCCGTGCACACCAGAGGCCTG GACTTCGCCTGCGACATCTACATCTGGGCCCCTCTGGC CGGCACCTGTGGCGTGCTGCTGCTGAGCCTGGTGATCA | 36 |
| | CCCTGTACTGCAACCACCGGAACAAACGGGGCAGAAA GAAACTCCTGTATATATTCAAACAACCATTTATGAGAC CAGTACAAACTACTCAAGAGGAAGATGGCTGTAGCTG CCGATTTCCAGAAGAAGAAGAAGGAGGATGTGAACTG AGAGTGAAGTTCAGCAGATCCGCCGACGCCCCTGCCT ACCAGCAGGGACAGAACCAGCTGTACAACGAGCTGAA CCTGGGCAGACGGGAAGAGTACGACGTGCTGGACAAG CGGAGAGGCCGGGACCCCGAGATGGGCGGAAAGCCC AGACGGAAGAACCCCCAGGAAGGCCTGTATAACGAAC TGCAGAAAGACAAGATGGCCGAGGCCTACAGCGAGAT CGGCATGAAGGGCGAGCGGAGGCGCGGCAAGGGCCA CGATGGCCTGTACCAGGGCCTGAGCACCGCCACCAAG GACACCTACGACGCCCTGCACATGCAGGCCCTGCCCC CCAGA | |
| GPRC5D CAR 2436 (#39) Amino acid sequence 375aa | MALPVTALLLPLALLLHAARPEVQLLESGGGLVQPGGSL RLSCAASGFIFSSYGMGWVRQAPGKGLEWVSSISSSGDY TYYADSVKGRFTISRDNSKNTLYLQMNSLRAEDTAVYY CARMSFRRYDHWGQGTLVTVSSFVPVFLPAKPTTTPAPR PPTPAPTIASQPLSLRPEACRPAAGGAVHTRGLDFACDIYI WAPLAGTCGVLLLSLVITLYCNHRNKRGRKKLLYIFKQP FMRPVQTTQEEDGCSCRFPEEEEGGCELRVKFSRSADAP AYQQGQNQLYNELNLGRREEYDVLDKRRGRDPEMGGK PRRKNPQEGLYNELQKDKMAEAYSEIGMKGERRRGKGH DGLYQGLSTATKDTYDALHMQALPPR | 37 |
| GPRC5D CAR 2438(#41) Coding nucleic acid sequence 1140bp | ATGGCCCTGCCTGTGACAGCTCTGCTCCTCCCTCTGGC CCTGCTGCTCCATGCCGCCAGACCCGAAGTTCAGCTGC TGGAAAGCGGTGGTGGTCTGGTTCAGCCTGGTGGTAG CCTGCGTCTGAGCTGTGCAGCAAGCGGTTTTAGCTTTA GCGGCTATATCATGGGTTGGGTTCGTCAGGCACCTGGT AAAGGTCTGGAATGGGTTAGCAGCACCAGCAGCAGCG GCACCGAAACCTATTATGCAGATAGCGTTAAAGGTCG TTTTACCATTAGCCGTGATAACAGCAAAAATACCCTGT ACCTGCAGATGAATAGTCTGCGTGCAGAGGATACCGC AGTGTATTATTGTGCGCGCTACTACTCTAAATACGGTC GTTCTTACCATGTTGATTCTTGGGGTCAAGGTACTCTG GTGACCGTCTCCTCATTCGTGCCCGTGTTCCTGCCCGC CAAACCTACTACTACCCCTGCACCTAGGCCTCCCACCC CAGCCCCAACAATCGCCAGCCAGCCTCTGTCTCTGCGG CCCGAAGCCTGTAGACCTGCTGCCGGCGGAGCCGTGC ACACCAGAGGCCTGGACTTCGCCTGCGACATCTACAT CTGGGCCCCTCTGGCCGGCACCTGTGGCGTGCTGCTGC TGAGCCTGGTGATCACCCTGTACTGCAACCACCGGAA | 38 |
| | CAAACGGGGCAGAAAGAAACTCCTGTATATATTCAAA CAACCATTTATGAGACCAGTACAAACTACTCAAGAGG AAGATGGCTGTAGCTGCCGATTTCCAGAAGAAGAAGA AGGAGGATGTGAACTGAGAGTGAAGTTCAGCAGATCC GCCGACGCCCCTGCCTACCAGCAGGGACAGAACCAGC TGTACAACGAGCTGAACCTGGGCAGACGGGAAGAGTA CGACGTGCTGGACAAGCGGAGAGGCCGGGACCCCGA GATGGGCGGAAAGCCCAGACGGAAGAACCCCCAGGA AGGCCTGTATAACGAACTGCAGAAAGACAAGATGGCC GAGGCCTACAGCGAGATCGGCATGAAGGGCGAGCGG AGGCGCGGCAAGGGCCACGATGGCCTGTACCAGGGCC TGAGCACCGCCACCAAGGACACCTACGACGCCCTGCA CATGCAGGCCCTGCCCCCCAGA | |
| GPRC5D CAR 2438 (#41) Amino acid sequence 380aa | MALPVTALLLPLALLLHAARPEVQLLESGGGLVQPGGSL RLSCAASGFSFSGYIMGWVRQAPGKGLEWVSSTSSSGTE TYYADSVKGRFTISRDNSKNTLYLQMNSLRAEDTAVYY CARYYSKYGRSYHVDSWGQGTLVTVSSFVPVFLPAKPT TTPAPRPPTPAPTIASQPLSLRPEACRPAAGGAVHTRGLD FACDIYIWAPLAGTCGVLLLSLVITLYCNHRNKRGRKKL LYIFKQPFMRPVQTTQEEDGCSCRFPEEEEGGCELRVKFS RSADAPAYQQGQNQLYNELNLGRREEYDVLDKRRGRDP EMGGKPRRKNPQEGLYNELQKDKMAEAYSEIGMKGER RRGKGHDGLYQGLSTATKDTYDALHMQALPPR | 39 |

### In vitro function verification of CAR-T cells

Experimental purpose and principle: Through affinity panning, specific antibody clones targeting GPRC5D protein were enriched from a phage antibody library, and clones that bind to GPRC5D-positive target cells were obtained after screening and identification. However, after these clones were constructed onto the second-generation CAR, their functions on CAR-T cells need to be further confirmed. To this end, we prepared lentiviral vectors of these clones and transduced T cells to prepare CAR-T cells. Then, the in vitro biological efficacy of CAR-T cells was evaluated by CD107a degranulation assay and in vitro cytotoxicity assay, and the function of the CAR constructed with the published antibody sequence (Benchmark 1) was simultaneously assessed. Through these functional verifications of CAR-T-level, we finally selected candidate single-chain antibody clones with ideal efficacy and safety for downstream CAR-T product development.

### Example

### Example 1. Enrichment of specific antibody clones targeting GPRC5D protein from phage antibody library by affinity panning

Appropriate negative and positive panning strategies were used to enrich the specific antibody clones we needed from a phage antibody library.

### Construction of phage antibody library

The phage antibody libraries we constructed include natural libraries, semi-synthetic libraries and single domain libraries. The semi-synthetic phage antibody library, used together with the natural library, solves the problem that the natural library may lack GPRC5D high affinity antibody clones. The single domain phage antibody libraries are antibody libraries composed only of variable region amino acids of heavy chain antibodies, which have the molecular weight of only 12-15 kDa, but have similar or higher specificity and affinity than traditional antibodies. In addition, single domain antibodies have attracted much attention due to their stable physicochemical properties, high affinity, easy recombinant expression and preparation, and easy combination with other target or epitope antibodies.

### GPRC5D protein panning

Multiple rounds of panning were performed using GPRC5D-VLP as a positive panning protein and VLP protein as a negative panning protein to obtain a phage pool enriched with an antibody clone of interest. Experimental steps were briefly described as follows:
1) Antigen coating: Dilute the antigen GPRC5D-VLP to 10 µg/mL with a clean coating buffer (PBS), add 100 µL of working solution to each well, select 6 wells for each panning, and bind overnight at 4°C; and
   dilute the antigen VLP protein to 10 µg/mL with a clean coating buffer (PBS), add 100 µL of working solution to each well, select 6 wells for each panning, and bind overnight at 4°C, where negative panning antigens were marked as negative plates, and positive panning antigens were marked as positive plates;
2) Blocking: Turn the antigen upside down, pat off the residual liquid in the well with absorbent paper, add 250 µL 3% BSA-PBS for blocking, and block at a room temperature for 2 h;
3) Add a phage library (containing 5×10¹² phage particles), and incubate with the control antigen VLP to remove the phage antibody clones that non-specifically bind to VLP;
4) After incubation, transfer the supernatant to the positive plate bound to the target antigen GPRC5D-VLP to continue incubation to allow the phage to bind to the target antigen;
5) Wash magnetic beads with a washing solution to remove unbound phages;
6) Elute off the positive phage from the target antigen with an eluent, and add a neutralization solution for neutralization;
7) Re-infect the host bacteria XL1-blue with the eluted phages, and amplify the recovered phages; and retain a small amount of sample for gradient dilution, infect with host bacteria, coat on Amp-resistant plates, and calculate the number of recovered phages; and
8) Repeat steps 1) to 6), and usually perform 3 rounds of panning until a significant increase in the phage recovery rate (number of eluted phages/number of input phages) was observed.

The enriched phage pool can be used for subsequent monoclone picking and ELISA/FACS screening.

Main materials and reagents:
Fully humanized phage antibody library, including natural library, semi-synthetic library and single domain library;
Helper phage KO7, Thermo/Invitrogen, 18311019;
Human GPRC5D Protein-VLP, Kactus, GPR-HM05P;
VLP protein, Kactus, order;
High binding ELSIA plate, Costar, #3590;
Blocking solution: PBS + 3% BSA;
Rinse solution: PBS + 0.1% Tween20;
Eluent: 0.2M Glycine, pH 2.2; and
Neutralization solution: 1M Tris, pH 9.1.
Experimental results:
Using different antibody libraries, three rounds of protein panning were performed, and a significant increase in the recovery rate was observed in each panning (Table 1), demonstrating that the antibody clones were effectively enriched.

**Table 1 Experimental results of protein panning**

| Antibody library | Rounds | Recovery rate | Enrichment factor |
|---|---|---|---|
| XL-V1 | 1st | 7.92E-06 | / |
| | 2nd | 1.40E-06 | 0.18 |
| | 3rd | 2.80E-04 | 200.00 |
| XL-V2 | 1st | 2.64E-06 | / |
| | 2nd | 9.20E-07 | 0.35 |
| | 3rd | 9.00E-05 | 97.83 |
| XL-SD-1 | 1st | 6.96E-06 | / |
| | 2nd | 1.12E-05 | 1.61 |
| | 3rd | 5.90E-04 | 52.68 |
| XL-NVH | 1st | 9.60E-06 | / |
| | 2nd | 4.40E-06 | 0.46 |
| | 3rd | 2.40E-04 | 54.55 |

It can be seen that after three rounds of panning, different antibody libraries have been enriched (the recovery rate in the third round was significantly higher than that in the previous round). However, this panning strategy screened out fewer specific binding clones, and in subsequent FACS experiments, they bound poorly to CHO-K1-GPRC5D cells that highly expressed the GPRC5D antigen, that is, they could not recognize the GPRC5D antigen in its natural status on the cell surface. Therefore, in subsequent experiments, we used the method of alternating protein and cell panning to enrich specific antibody clones that can simultaneously bind to GPRC5D protein and GPRC5D in its natural status on the cell surface from the phage antibody library. Table 2 shows the results of co-panning using recombinant GPRC5D protein and CHO-K1-GPRC5D/CHO-K1 cell line. Judging from the recovery rate, all four rounds of panning were enriched and can be used for the next step of picking monoclones.

**Table 2 Experimental results of protein+ cell panning**

| Antibody library | Rounds | Recovery rate | Enrichment factor |
|---|---|---|---|
| XL-V1 | 1st | 7.92E-06 | / |
| | 2nd | 1.40E-06 | 0.18 |
| | 3rd | 3.30E-05 | 23.57 |
| | 4th | 3.80E-03 | 115.15 |
| XL-V2 | 1st | 2.64E-06 | / |
| | 2nd | 9.20E-07 | 0.35 |
| | 3rd | 2.24E-05 | 24.35 |
| | 4th | 1.09E-02 | 486.61 |
| XL-SD-1 | 1st | 6.96E-06 | / |
| | 2nd | 1.12E-05 | 1.61 |
| | 3rd | 3.08E-04 | 27.5 |
| | 4th | 1.88E-02 | 61.04 |
| XL-NVH | 1st | 9.60E-06 | / |
| | 2nd | 4.40E-06 | 0.46 |
| | 3rd | 9.86E-05 | 22.41 |
| | 4th | 1.67E-02 | 169.37 |

### Example 2. Screening for specific clones from the enriched phage pool using ELISA and FACS

Purpose and principle: The phage pool enriched by affinity panning steps contains phage antibodies of various properties: specific clones, non-specific clones, and negative clones. In order to obtain specific clones, we need to isolate monoclones therefrom, package them into monoclonal phages, preliminarily screen a large number of monoclones by ELISA and FACS, and pick monoclones that specifically bind to both GPRC5-VLP protein and GPRC5D-positive cell line CHO-K1-GPRC5D therefrom. The specific monoclone is further confirmed by DNA sequencing to identify the contained unique antibody sequence therein.

In the ELISA preliminary screening, clones that only bound to GPRC5D-VLP but not to control antigen VLP were identified as specific clones. The FACS preliminary screening was performed using the positive cell line CHO-K1-GPRC5D with high GPRC5D expression and the GPRC5D-negative cell line CHO-K1. The clones that only bound to CHO-K1-GPRC5D cells and not to CHO-K1 cells were identified as specific clones. Through two kinds of preliminary screening of ELISA and FACS, we can obtain candidate antibodies that can bind to recombinant expressed GPRC5D protein and recognize the GPRC5D molecules in natural state on the cell surface for further screening subsequently.

Brief steps of ELISA screening experiment:
1) Cultivate and package monoclonal phages in deep-well 96-well plates;
2) Dilute GPRC5D-VLP to 10 µg/mL with PBS, add at 100 µL/well to a high binding ELISA plate, and coat overnight at 4°C;
3) Discard the coating solution, add 250 µL of blocking solution to each well, and block at a room temperature for 2 h;
4) Wash the plate with 250 µL of rinse solution twice;
5) Add 100 µL of the cultured phage supernatant from step 1) to the well coated with a target antigen, and allow to bind at a room temperature for 2 h;
6) Wash the plate with 250 µL of rinse solution four times;
7) Add 100 µL of 1:2000 diluted mouse anti M13 primary antibody per well, and incubate at a room temperature for 45 min;
8) Wash the plate with 250 µL of rinse solution four times;
9) Add 100 µL of 1:2000 diluted HRP Donkey anti-mouse IgG per well, and incubate at a room temperature for 45 min;
10) Wash the plate with 250 µL of rinse solution six times;
11) Add 100 µL of TMB chromogenic substrate, and develop for 10 min; and
12) Add 100 µL of 2M H₂SO₄ to terminate the reaction, and read the results on a microplate reader.

Brief steps of FACS preliminary screening experiment:
1) Cultivate and package monoclonal phages in deep-well 96-well plates;
2) Wash CHO-K1-GPRC5D and CHO-K1 cells with PBS twice, resuspend them in PBS to a concentration of 1×10⁷/mL, and dispense 50 µL into 96-well deep-well plates;
3) Add 50 µL of packaged monoclonal phage to each well, mix well, and allow to bind at 4°C for 2 h;
4) Wash with 200 µL of PBS twice;
5) Add 100 µL of 1:2000 diluted mouse anti M13 primary antibody per well, pipette to mix well, and then, incubate at a room temperature for 45 min;
6) Wash with 200 µL of PBS twice;
7) Add 100 µL of 1:300 diluted FITC horse anti mouse-IgG (H+L) per well, pipette to mix well, and then, incubate at a room temperature for 45 min;
8) Wash with 200 µL of PBS twice, and finally, resuspend the cells with 200 µL of PBS; and
9) Detect a sample FITC channel for the fluorescence intensity on a flow cytometer, and analyze results.

Main materials and reagents:
Helper phage KO7, Thermo/Invitrogen, 18311019;
Streptavidin, Pierce, 21125;
Human GPRC5D Protein-VLP, Kactus, GPR-HM05P;
Biotinylated Human IL-10 Protein, Kactus, IL1-HM410B;
Biotinylated Recombinant human BAFFR Protein, Kactus, BAF-HM40RB;
High binding ELSIA plate, Costar, #3590;
Corning 96 Well Clear Round Bottom TC-Treated Microplate, Costar, #3799;
Blocking solution: PBS + 3% BSA;
Rinse solution: PBS + 0.1% Tween20;
Soluble one-component TMB substrate solution, Tiangen, PA-107-02;
Anti-M13 Bacteriophage Coat Protein g8p antibody, abcam, ab9225;
HRP Goat anti-mouse IgG(minimal x-reactivity) Antibody, Biolegend, 405306;
HRP Donkey anti-human IgG (minimal x-reactivity) Antibody, Biolegend,410902;
HRP Conjugated Anti His-Tag Mouse Monoclonal Antibody, CWBIO, CW0285;
FITC horse anti mouse-IgG(H+L), Vector, FI2000;
H_GPRC5D CHO-K1 Cell Line Clone, Shanghai Jiman, GM-C03857;
Human GPRC5D PE-conjugated Antibody, RD, FAB6300RP; and
Fluorescein (FITC) AffiniPure Goat Anti-Human IgG, Fcy fragment specific, Jackson ImmunoReseach, 109-095-190.

### Experimental results:

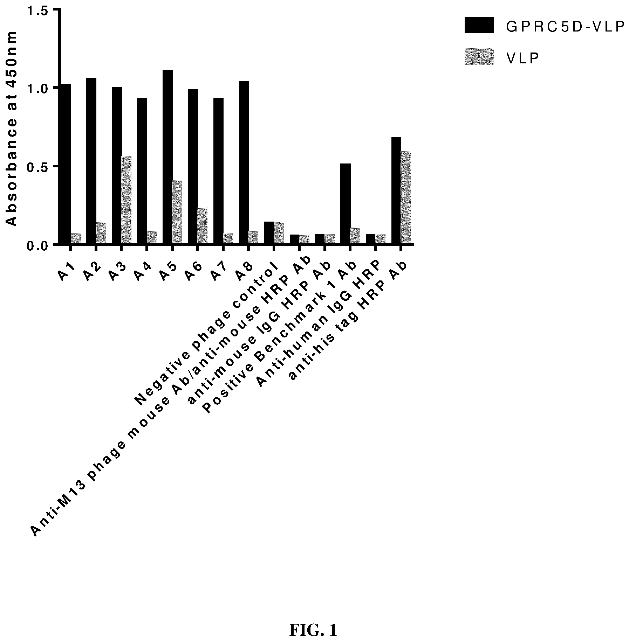

Monoclones were randomly picked from the enriched phage antibody pool and packaged into phages, and then, the binding of monoclonal phages to GPRC5D-VLP protein and control protein VLP was detected by phage ELISA to find GPRC5D-specific phage antibody clones. The ELISA results of some clones were as shown in FIG. 1. As can be seen from the figure, clones A1, A2, A4, A7 and A8 bind strongly to the target antigen GPRC5D (GPRC5D-VLP) and do not bind to the control antigen VLP, with good specificity. Clones A3, A5 and A6 bind to both the target antigen and the control antigen and do not meet specific binding requirements. Negative phage control is a negative control phage antibody clone, which does not bind to either the target antigen or the control antigen. Anti-M13 phage mouse/anti-mouse HRP Ab is a negative antibody control with only primary and secondary antibodies added. Anti-mouse HRP Ab is a negative antibody control with only secondary antibodies added. Anti-human IgG HRP Ab is a negative antibody control with only secondary antibodies added. They do not bind to either the target antigen or the control antigen. Anti-his tag HRP Ab is a positive antibody control for detecting antigen tags, which binds to the his-tagged antigen, indicating that the coated antigen has been bound to the plate. Positive Benchmark1 is a positive antibody for the target antigen, which binds to the target antigen but not to the control antigen.

The results of FACS preliminary screening of antibody clones corresponding to ELISA were as shown in FIG. 2. FIG. 2A shows results of staining the CHO-K1-GPRC5D cell line with commercial flow cytometric antibodies Human GPRC5D PE-conjugated Antibody and Positive benchmark1 (the variable region sequence comes from GC5B596 of Chinese patent application publication CN 109715667 A). The results show that the cells can bind to the GPRC5D antibody, indicating that CHO-K1-GPRC5D cells are GPRC5D-expressing positive cells. The clones A1, A2, A3, A4, A5, A6 and A7 in FIG. 2B bind to CHO-K1-GPRC5D but not to CHO-K1 cells, and are specific clones. The clone A8 does not bind to either of the two cells and is a negative clone. Negative phage control is a negative control phage antibody clone that does not bind to either the target antigen or the control antigen. Positive Benchmark1 Ab (GC5B596) is a positive antibody for the target antigen, which binds to the high-expressing cell line CHO-K1-GPRC5D, but does not bind to the control negative cell line CHO-K1.

Through ELISA detection and FACS preliminary screening, we obtained a total of 216 ELISA and FACS double-positive clones with good specificity. We then sequenced the obtained 216 double-positive clones with good specificity and obtained 35 different monoclonal sequences. These 35 monoclones with different sequences were then further identified by FACS of multiple cell lines and ELISA of multiple antigens to detect the binding specificity of the candidate clones. Three single domain antibody clones (clone numbers 18, 39 and 41) were selected for experiments in the following examples, and their HCDR sequences, heavy chain variable region (VH) sequences and VH encoding nucleic acid sequences were shown above.

### Example 3. Further identification of monoclonal specificity at phage level by ELISA and FACS

Experimental purpose and principle: Antibodies used for treatment must have very good target specificity, only binding to the target antigen and not to any unrelated antigens. On the other hand, the amino acid sequence of the same antigen on different cell lines may be different (isomers or mutants) or the bound ligands may be different, so it is also necessary to examine whether our antibodies can bind to positive cells for various target proteins. In order to further analyze the specificity and universality of these monoclones and find the best candidate clones, we further assessed the specificity of preliminary screening clones by enzyme linked immunosorbent assay (ELISA) and flow cytometry.

### Further identification of monoclonal specificity by ELISA using multiple unrelated antigens

In this experiment, we used the target antigen GPRC5D-VLP antigen and various GPRC5D-unrelated antigens to react with these monoclonal phage antibodies to analyze whether these clones have any non-specific binding to other GPRC5D-unrelated antigens. Through this experiment, we obtained several clones with excellent specificity.

Experimental method: same as ELISA preliminary screening.

Main samples and reagents:

| Abbreviation | Name | Manufacturer | Part number |
|---|---|---|---|
| VLP | VLP | Kactus | order |
| GPRC5D-VLP | Human GPRC5D Protein-VLP | Kactus | GPR-HM05P |
| BAFFR-his-Bio | Biotinylated Recombinant human BAFFR Protein | Kactus | BAF-HM40RB |
| IL10-Bio | Biotinylated Human IL-10 Protein | Kactus | IL1-HM410B |
| SA | Streptavidin, | Pierce | 21125 |

The remaining reagents are the same as those for the ELISA preliminary screening.

### Experimental results:

Antibodies used for treatment must have very good target specificity. In order to further analyze the specificity of these monoclonal antibodies, we identified the multiple clones obtained in Example 2 using enzyme-linked immunosorbent assay (ELISA) on various antigens. The results were as shown in FIG. 3. Negative phage control is a negative control phage antibody clone that does not bind to either the target antigen or the control antigen. Anti-M13 phage mouse/anti-mouse HRP Ab is a negative antibody control with only primary and secondary antibodies added. Anti-mouse HRP Ab is a negative antibody control with only secondary antibodies added. They do not bind to either the target antigen or the control antigen. Positive Benchmark1 Ab (GC5B596) is a positive antibody control for the target antigen (GPRC5D-VLP), which binds to the target antigen but not the control antigen. Anti-human IgG HRP Ab is a negative antibody control with only secondary antibodies added. Anti-his HRP Ab is a positive antibody control for detecting antigen tags, which binds to the his-tagged antigen, indicating that the coated antigen has been bound to the plate. Clones 18, 39, and 41 all bind to the GPRC5D antigen, but do not bind to four unrelated antigens, indicating that the clones 18, 39, and 41 can bind to the GPRC5D antigen with good specificity.

### Further identification of monoclonal specificity by FACS using multiple cell lines at phage level

In this experiment, we used various GPRC5D-positive cell lines and various GPRC5D-negative cell lines to react with these monoclonal phage antibodies to analyze whether these clones can bind to GPRC5D antigens on different cell lines and whether there is any non-specific binding to other cell lines that do not express GPRC5D. Through this experiment, we obtained several clones with excellent specificity.

### Experimental method: same as FACS preliminary screening;

Main samples and reagents:
CHO-K1-GPRC5D cell line, GPRC5D-positive cell line;
MM1S cell line, GPRC5D-positive cell line;
CHO-K1 cell line, GPRC5D-negative cell line; and
Jurkat cell line, GPRC5D-positive cell line.

The remaining reagents are the same as those for the FACS preliminary screening.

### Experimental results:

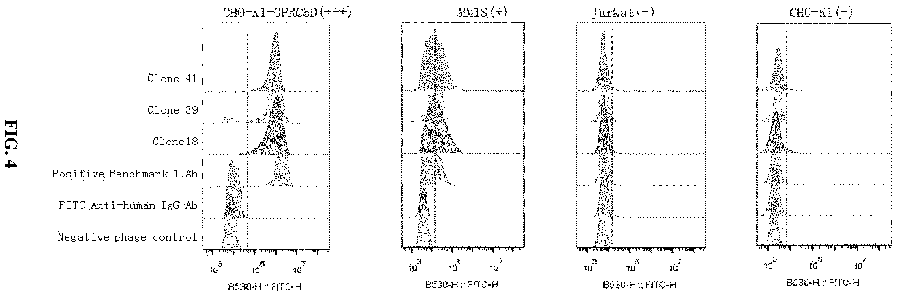

Antibodies used for treatment must have very good target specificity. In order to further analyze the specificity of these monoclonal antibodies, we identified the unique clone obtained in Example 2 using enzyme-linked immunosorbent assay and flow cytometry on various antigens and cell lines. The results were as shown in FIG. 4. Negative phage Control is a negative control phage antibody clone. Clones 18, 39, and 41 bind to both GPRC5D-positive cell lines, CHO-K1-GPRC5D and MM1S, but do not bind to both GPRC5D-negative cell lines, CHO-K1 and Jurkat, with good specificity. Positive Benchmark1 Ab (GC5B596) is a positive antibody for the target antigen, which binds to the high-expressing cell line CHO-K1-GPRC5D but not to the control negative cell CHO-K1. FITC anti-human IgG Ab is a negative control with only secondary antibodies added, which does not bind to both cell lines.

### Example 4. Further confirmation of binding specificity of monoclones by ELISA and FACS at protein level Experimental purpose and principle

By affinity panning, specific antibody clones targeting the GPRC5D antigen were enriched from the phage antibody library, and clones that bind to the GPRC5D antigen were obtained after screening and identification. However, after the antibody molecules expressed in the prokaryotic system were converted into IgG antibody molecules expressed in the eukaryotic system, their binding ability and specificity need to be further confirmed. To this end, we prepared IgG expression plasmids for these clones and expressed them by transient transfection of CHOS cells, and they were purified by Protein A into antibodies. Then, the binding specificity of monoclones is further confirmed by ELISA and FACS.

### Further confirmation of binding specificity of monoclones at antibody level by ELISA at protein level

Main samples and reagents:
same as ELISA reagents at phage level;

### Experimental results:

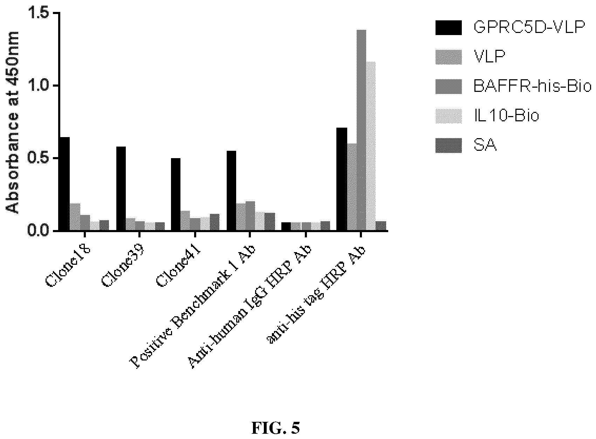

In order to further analyze whether these monoclonal antibodies still maintain the binding ability and specificity of the original antibodies after being expressed as IgG antibodies in the eukaryotic system, three clones obtained in Example 3 were expressed by CHOS, and the antibodies purified by Protein A into the IgG form were identified by enzyme-linked immunosorbent assay (ELISA) on multiple antigens. The results were as shown in FIG. 5. Positive Benchmark1 Ab is a positive antibody control for the target antigen (GPRC5D-VLP), which binds to the target antigen but not to the control antigen. Anti-human IgG HRP Ab is a negative antibody control with only secondary antibodies added. Anti-his HRP Ab is a positive antibody control for detecting antigen tags, which binds to the his-tagged antigen, indicating that the coated antigen has been bound to the plate. Clones 18, 39, and 41 all bind to the GPRC5D antigen, but do not bind to four unrelated antigens, indicating that the clones 18, 39, and 41 can bind to the GPRC5D antigen with good specificity.

### Further confirmation of binding specificity of monoclones at antibody level by FACS at protein level

### Main samples and reagents:

Fluorescein (FITC) AffiniPure Goat Anti-Human IgG, Fcy fragment specific, Jackson ImmunoReseach, 109-095-190

### Experimental results:

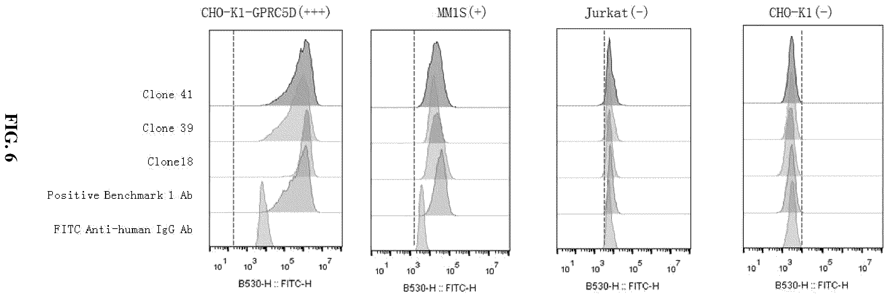

In order to further analyze whether these monoclonal antibodies still maintain the binding ability and specificity of the original antibodies after being expressed as IgG antibodies in the eukaryotic system, three clones obtained in Example 3 were expressed by CHOS, and the antibodies purified by Protein A into the IgG form were identified by enzyme-linked immunosorbent assay and flow cytometry on more antigens and cell lines. The results were as shown in FIG. 6. Clones 18, 39, and 41 bind to both GPRC5D-positive cell lines, CHO-K1-GPRC5D and MM1S, but do not bind to both GPRC5D-negative cell lines, CHO-K1 and Jurkat, with good specificity. Positive Benchmark1 Ab (GC5B596) is a positive antibody for the target antigen, which binds to the high-expressing cell line CHO-K1-GPRC5D but not to the control negative cell CHO-K1. FITC anti-human IgG Ab is a negative control with only secondary antibodies added, which does not bind to both cell lines.

### Example 5. Detection of binding ability of monoclonal antibody to high-expressing cell line by FACS

The affinity between the GPRC5D antibody molecule and the antigen may have an important impact on the killing effect and duration of CAR-T or antibody drugs in the patient's body. Since it is difficult to obtain purified antigens for the target GPRC5D, we used the FACS binding method to analyze the half effective concentration (Ec50) of the antibody molecule to provide important information for the research and development process.

Brief steps of FACS binding experiment:
1) Preparation of antibodies at different concentrations: Use PBS to dilute anti-GPRC5D IgG from 300 nM in 5-fold increments to 0.00384 nM, a total of 8 concentrations, to detect the binding ability of antibodies to CHO-K1-GPRC5D cells;
2) Wash CHO-K1-GPRC5D cells with PBS twice, resuspend them in PBS to a concentration of 1×10⁷/mL, and dispense 50 µL into 96-well deep-well plates;
3) Add 50 µL of diluted antibody to each well, mix well, and then, allow to bind at 4°C for 2 h;
4) Wash with 200 µL of PBS twice;
5) Add 100 µL of 1:300 diluted Alexa Fluor^{®} 647 AffiniPure Goat Anti-Human IgG per well, pipette to mix well, and then, incubate at a room temperature for 45 min;
6) Wash with 200 µL of PBS twice; and finally, resuspend the cells with 200 µL of PBS;
7) Detect a sample Alexa Fluor^{®} 647 channel for the fluorescence intensity on a flow cytometer; and
8) Analyze binding constants by using Graphpad Prism software.

### Main samples and reagents:

CHO-K1-GPRC5D cell line, GPRC5D-positive cell line; and

Alexa Fluor^{®} 647 AffiniPure Goat Anti-Human IgG, Fcy fragment specific, Jackson ImmunoReseach, 109-605-008.

### Experimental results

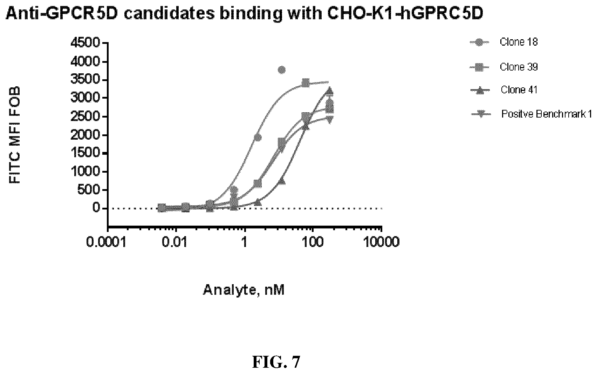

Affinity refers to the strength of binding of a single molecule to its ligand. The binding ability to positive cell lines can usually be detected by FACS to assess the strength of the interaction between two molecules and rank them. The smaller the Ec50 value, the greater the affinity of the antibody for its target. As shown in Table 3 and FIG. 7, Benchmark1, Clone 18, Clone 39 and Clone 41 can all bind to CHO-K1-GPRC5D cells in a GPRC5D overexpressing cell line, and the affinity of Clone 18 is slightly higher than that of Benchmark1 and higher than that of Clone 39 and Clone 41.

**Table 3 Affinity determination results**

| Antibody | Ec50 (nM) | TOP (MFI) |
|---|---|---|
| Clone 18 | 1.58 | 3462 |
| Clone 39 | 6.93 | 2795 |
| Clone 41 | 41.02 | 3690 |
| Benchmark 1 | 6.72 | 2525 |

### Example 6. FACS detection for monoclonal antibody cross-species

To research the species cross characteristics of three monoclonal antibodies obtained by identification, we detected the binding of the three antibodies to murine-derived and monkey-derived GPRC5D target antigens. Due to the complex structure of GPRC5D antigens, it is difficult to obtain purified antigens, we constructed monkey-derived and murine-derived overexpressing cell lines and used FACS analysis to confirm the cross species characteristics of candidate antibodies.

Brief steps of FACS binding experiment: same as Example 4.

Main samples and reagents:
CHOS-mouseGPRC5D cell line, GPRC5D overexpressing murine-derived positive cell line;
CHOS-macaqueGPRC5D cell line, GPRC5D overexpressing monkey-derived positive cell line;
Alexa Fluor^{®} 647 AffiniPure Goat Anti-Human IgG, Fcy fragment specific, Jackson ImmunoReseach, 109-605-008; and
Fluorescein (FITC) AffiniPure Goat Anti-Human IgG (H+L), Jackson ImmunoReseach, 109-095-008.

### Experimental results:

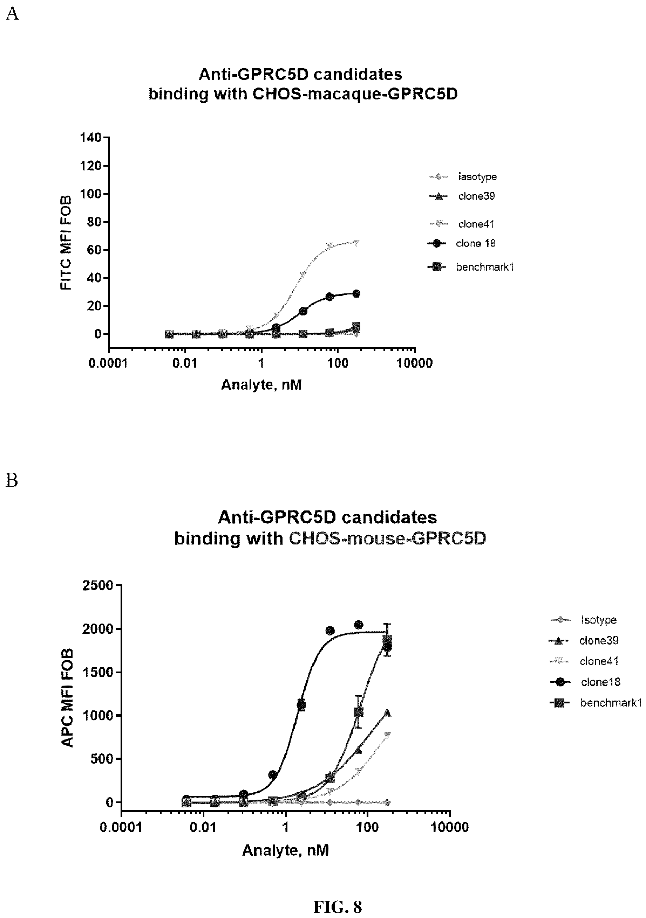

Due to the complex structure of GPRC5D antigens, it is difficult to obtain purified antigens. We overexpressed murine-derived GPRC5D proteins and monkey-derived GPRC5D proteins on CHOS cells respectively. Cross characteristics in monkeys and murine were confirmed by detecting candidate antibodies in combination with overexpressing cell lines. As shown in FIG. 8A, both Clones 18 and 41 can bind to CHOS-macaqueGPRC5D, i.e., cells in a GPRC5D monkey-derived positive cell line, while Clone 39 and Benchmark1 monkey cross-reactivity is weaker. As shown in FIG. 8B, Clones 18, 41, and 39 and benchmark1 can all bind to a CHOS-mouseGPRC5D cell line, i.e., a GPRC5D murine-derived positive cell line. The binding of Clone 18 and Benchmark1 to the murine-derived GPRC5D cell line more is stronger than that of Clone 39 and Clone 41.

### Example 7. Detection of EGFRt expression on surface of CAR-T cells

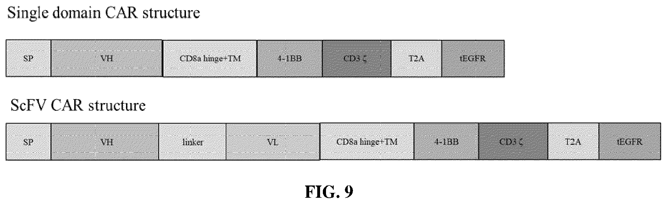

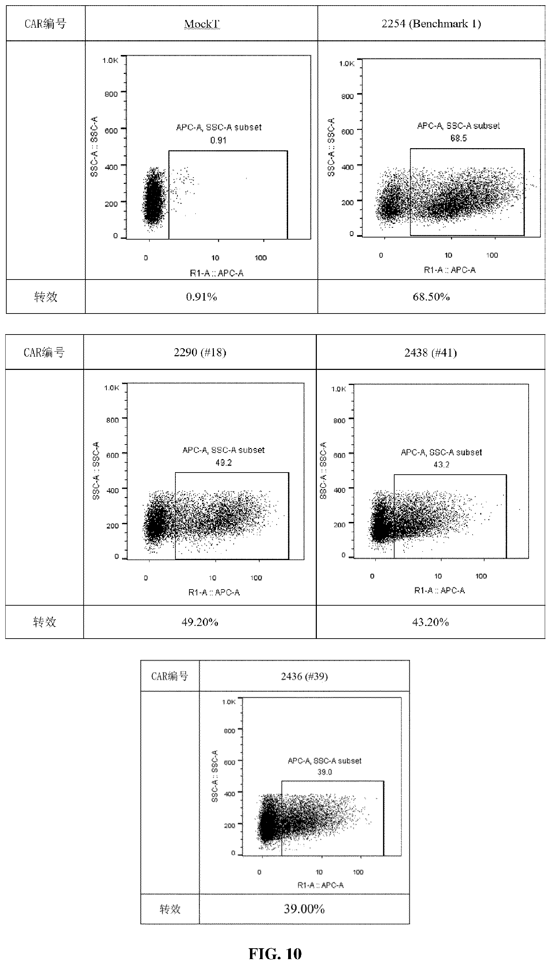

To verify the functions of candidate clones on CAR-T cells, based on a candidate antibody sequence, we constructed a CAR vector with the structure as shown in FIG. 9. The CAR structure contains a CD8α signal peptide, V_{HH} or scFv, a CD8α hinge region, a CD8α transmembrane region, a 4-1BB co-stimulatory molecule and CD3ζ, and a truncated EFGR molecule (EGFRt) was linked with T2A, which can be used as a safety switch in clinical transformation. The CAR vector was then packaged with a lentivirus, and the expression of EGFRt was detected 5 to 7 days after T cells were transfected with the CAR lentivirus (FIG. 10). Since EGFRt is co-expressed with CAR molecules, it can be used as an indirect detection indicator for the distribution of CAR molecules on the surface of T cells without affecting the structure and function of the CAR.

Brief experimental steps for CAR-T/EGFRt expression detection are as follows:
1) Take 1×10⁶ CAR-T/T cells per well, add PBS to wash once, centrifuge at 300 g for 5 min, and discard the supernatant.
2) Resuspend the cell pellet with 100 µL of PBS, add 5 µL of APC-CD5 antibody and 5 µL of APC-EGFR antibody respectively, and incubate at 4°C in the dark for 15 min.
3) Wash with PBS twice, and centrifuge at 300 g for 5 min.
4) Resuspend with 200 µL of PBS, and detect on flow cytometer.

Main materials and reagents:
APC anti-human EGFR Antibody, Clone AY13, BioLegend, Cat. No.352906; and
Fetal bovine serum (FBS), Gibco, Cat. No.10099141.

### Experimental results:

As shown in FIG. 10, detection was performed on the seventh day, and the expression of EGFR in Tcells in each group was ≥30%.

### Example 8. CD107a degranulation assay

### Experimental principle and purpose:

CD107a is a marker for intracellular microvesicles. CD107a on the cell membrane increases after granzyme-loaded microvesicles fuse with the cell membrane, and when its recovery is blocked by monesin (purchased from BioLegend), it can quantitatively reflect the strength of microvesicle release. When CAR-T cells are stimulated by target antigens on target cells, granzymes are released, and the activation of T cells can be determined by flow cytometric detection of the increase in CD107a.

Brief experimental steps of CD107a degranulation:
1) Centrifuge the CAR-T cells to be detected and target cells respectively at a room temperature and 300 g for 5 min, discard the supernatant, and then, resuspend the cells with a 1640 culture medium + 10%FBS to 2×10⁶ cells/mL;
2) Add 100 µL of the CAR-T cells to be detected and 100 µL of the target cells to 96-well plates respectively, and mix well;
3) Add 1.5 µL of PE/Cy7 anti-human CD107a antibody and 0.2 µL of monensin to each well of cells, then place in a cell incubator at 37°C, and incubate with 5% of CO₂ for 4 h;
4) After completion of incubation, centrifuge at 4°C and 300 g for 5 min, then discard the supernatant, and wash the cells with 200 µL of PBS twice;
5) Resuspend the cells with 100 µL of PBS, add 1.5 µL of BV421 anti-human CD8 and 1.5 µL of APC anti-human EGFR antibodies respectively, mix well, and then, incubate on ice in the dark for 20 min; and
6) After completion of incubation, wash the cells with 200 µL of PBS 3 times, resuspend the cells with 100 µL of PBS, and then, detect by a flow cytometer.

### Main samples and reagents:

Target cells U266-luc (GPRC5D+), 8226-luci (GPRC5D+), MM.1S-luci (GPRC5D+), Raji-luc (GPRC5D-), CCRF-luc (GPRC5D-), Molt4-luc (GPRC5D-), Jurkat-luc (GPRC5D-);
Fetal bovine serum (FBS), Gibco, Cat. No.10099141;
Monensin, BioLegend, Cat. No. 420701;
PE/Cy7 mouse anti-human CD107a, BD, Cat. No. 561348;
BV421 mouse anti-human CD8, BD, Cat. No. 301036; and
APC anti-human EGFR Antibody, Clone AY13, BioLegend, Cat. No.35290.
Experimental results:
   CAR-T cells were obtained by lentiviral transduction, and the CAR-T cells were cultured in vitro for 9-12 days before a CD107a degranulation assay. The CAR-T cells to be detected were incubated with target cells, monensin and CD107a antibodies for 4 h, and the cell density of the CAR-T cells and target cells was both2×10⁵ cells/mL. The samples were then labeled with CD8 antibodies and EGFR antibodies and then subjected to flow cytometric detection. By analysis in the Flowjo software, a live cell gate (P1) was selected in a scatter plot to remove cell debris; the cells in the P1 gate were analyzed, and a single scattered cell gate (P2)was selected; then, CD8-positive cells (P3) were further selected in the P2 gate; and finally, in the P3 gate, the proportion of CD107a-positive cells in EGFRantibody-stained positive cells (i.e., CAR-positive cells) was analyzed. The analysis results were as shown in Table 4. The degranulation level of the GPRC5D CAR-T was significantly increased only after co-incubation with GPRC5D-positive cells, while there was no significant increase in the degranulation level after co-incubation with negative cell lines. The degranulation levels of GPRC5D-positive cells of Clones 18, 39, and 41 were higher than that of Benchmark 1.

**Table 4 Results of CD107a degranulation of different target cells on CAR-T cells of different clones**

| **CD107a+/CAR+CD8 +%** | **MockT** | **2254 (Benchmark#1)** | **2290 (#18)** | **2436 (#39)** | **2438 (#41)** |
|---|---|---|---|---|---|
| U266 (GPRC5D+) | 5.43 | 9.68% | 80.50 | 78.80 | 56.00 |
| 8226 (GPRC5D+) | 5.38 | 45.00 | 83.20 | 76.50 | 56.50 |
| MM.1S (GPRC5D+) | 4.07 | 50.70 | 71.70 | 63.50 | 41.30 |
| Raji (GPRC5D-) | 6.41 | 30.50 | 11.70 | 13.20 | 5.45 |
| CCRF (GPRC5D-) | 5.29 | 4.05 | 10.30 | 12.80 | 4.44 |
| Molt4 (GPRC5D-) | 4.74 | 4.14 | 9.28 | 12.00 | 3.13 |
| Jurkat (GPRC5D-) | 4.49 | 4.79 | 11.70 | 10.30 | 3.87 |
| EFFECTOR | 7.96 | 5.49 | 15.20 | 18.00 | 7.68 |

### Example 9. In vitro cell killing assays

### Experimental purpose and principle:

In vitro cell killing assays used U266, MM1.s, 8226, and H929 cells as GPRC5D-positive target cells, and Raji and CCRF cells as GPRC5D-negative target cells to evaluate the antigen-specific killing ability of GPRC5D CAR-T cells. The above target cells were respectively transduced by lentiviral transduction to obtain target cells that stably express firefly luciferase, so the activity of luciferase in the sample can reflect the number of target cells. CAR-T cells and target cells were co-incubated and cultured. When target cells were killed by CAR-T cells, luciferase was released and quickly inactivated (firefly luciferase has a half-life of about 0.5 h). If target cells were not killed or inhibited by CAR-T cells, more luciferases will be produced with the proliferation of target cells and continuous expression of luciferase. Therefore, the killing of target cells by the CAR-T can be detected by the activity of luciferase.

### Brief experimental steps of in vitro cell killing:

1) Centrifuge the above-mentioned target cells at a room temperature and 500 g for 5 min respectively, discard the supernatant, then resuspend the cells with a 1640 + 10%FBS culture medium to 1×10⁵cells/mL, and add 100 µL of target cells to each well of 96-well plates respectively;
2) According to the CAR positive rate and effector-target ratio of the CAR-T sample to be detected, add 100 uL of CAR-T cells to each well of the 96-well plates respectively, and mix well with the target cells; and then, put the mixture in a carbon dioxide incubator to incubate and culture for 24h; and
3) Use a luciferase detection kit to respectively detect the luciferase activity in each well of sample.

### Main samples and reagents:

GPRC5D-positive target cells U266-luc, MM1.s-luc 8226-luc, H929-luc;
GPRC5D-negative target cells Raji-luc, CCRF-luc; and
Steady-Glo Luciferase Assay System, Promega, Cat. No. E2520.

### Experimental results:

CAR-T cell samples and a fixed number of target cells (1x104) were mixed at different effector-target ratios (E:T) and incubated together for 24 h, and then, the luciferase activity (RLU) in the samples was detected. Since the luciferase activity can reflect the number of target cells in a sample, the killing/inhibition ability of CAR-T cells on target cells can be obtained by changes in luciferase activity in the sample. The lower the luciferase activity readout (RLU), the more the target cells killed.

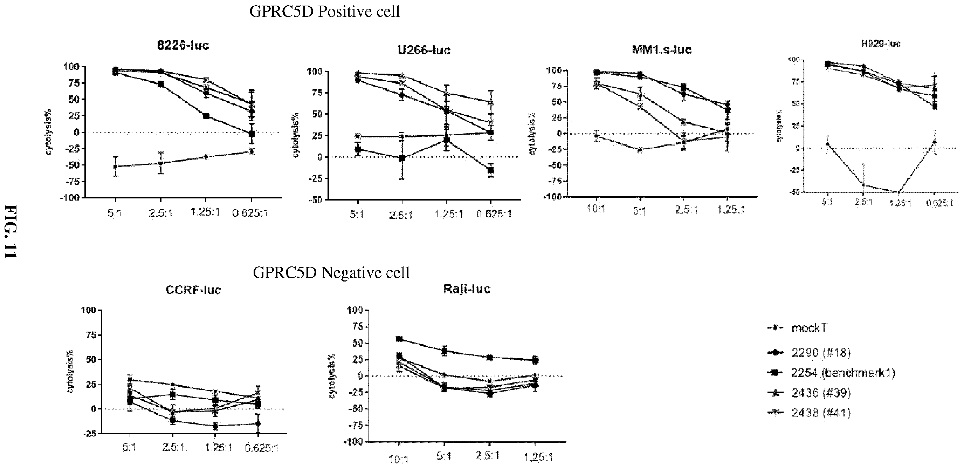

As shown in FIG. 11, all CAR-T cell samples can effectively kill GPRC5D-positive target cells. Among them, the killing of Clones 18, 39, and 41 were higher than that of Benchmark 1 in 8226-luc and U266-luc. Benchmark 1 has a certain degree of killing of negative cells Raji. Clones 18, 39, and 41 have no non-specific killing of negative cells.

Through CD107a degranulation assays and in vitro cell killing assays, it was found that Clones 18, 39, and 41 were superior to benchmark 1 in the in vitro function. The cytokines, in vitro proliferation abilities and the like of the CARs of these three clones were further verified and evaluated below.

### Example 10. Determination of cytokine expression on CAR cells

### Experimental purpose and principle

U266, MM1.s, 8226, and H929 cells were used as GPRC5D-positive target cells, and Raji and CCRF cells were used as GPRC5D-negative target cells. GPRC5D CAR-T cells were incubated with target cells, and the expression of IFN-r and TNFa inCAR cells was determined by flow cytometry.

Brief experimental steps:
1) Centrifuge the CAR-T cells to be detected and target cells respectively at a room temperature and 300 g for 5 min, discard the supernatant, and resuspend the cells with a 1640 culture medium + 10%FBS to 8×10⁶ cells/mL;
2) Add 50 µL of the CAR-T cells to be detected and 50 µL of the target cells to 96-well plates respectively, and mix well;
3) Add 50 ul of monensin and brefeldin A premixto each well of cells (equivalent to 0.2 ul/well), then place in a cell incubator at 37°C, and incubate with 5% CO2 for 4 h;
4) After completion of incubation, centrifuge at 4°C and 300 g for 5 min, then discard the supernatant, and wash the cells with 200 µL of PBS twice;
5) Resuspend the cells with 100 µL of PBS, add 1 µL of BV421 anti-human CD8 and 1 µL of APC anti-human EGFR antibodies respectively, mix well, then incubate on ice in the dark for 20 min, and wash with 200 ul of PBS twice;
6) Add 100 ul of fixation/permeabilization buffer to each well, incubate at 4°C for 20 min, and wash with 1× wash buffer twice; and
7) Add 50 ul of buffer of each 1.5 ul of PE-TNFα antibodies and PE-Cy7-INFy antibodies, incubate at 4°C for 20 min, and wash with 1x wash buffer twice. After resuspending with 100 ul of PBS, the cells were detected by a flow cytometer.

### Main samples and reagents:

Target cells U266-luc (GPRC5D+), 8226-luc (GPRC5D+), MM.1S-luc (GPRC5D+) H929-luc (GPRC5D+);
Raji-luc (GPRC5D-), CCRF-luc (GPRC5D-);
Fetal bovine serum (FBS), Gibco, Cat. No.10099141;
Monensin, BioLegend, Cat. No. 420701;
PE/Cy7 mouse anti-human CD107a, BD, Cat. No. 561348;
BV421 mouse anti-human CD8, BD, Cat. No. 301036;
APC anti-human EGFR Antibody, Clone AY13, BioLegend, Cat. No.35290;
PE anti-human TNF-a Biolegend, Cat. No.502909; and
PE-Cy7 anti-human IFN-r Biolegend, Cat. No. 506578.

### Experimental results

By analysis in the Flowjo software, a live cell gate (P1) was selected in a scatter plot to remove cell debris; the cells in the P1 gate were analyzed, and a single scattered cell gate (P2) was selected; then, CD8-positive cells (P3) were further selected in the P2 gate; and finally, in the P3 gate, the proportion of TNF-a/IFN-r positive cells in TNF-a antibody or IFN-r-stained positive cells (i.e., CAR-positive cells) was analyzed. The analysis results were as shown in Table 5. The expression levels of TNFa and IFN-r were significantly increased only after the GPRC5D CAR-T was co-incubated with GPRC5D-positive cells, while there was no significant increase in the expression levels of TNFa and IFN-r after the GPRC5D CAR-T was co-incubated with negative cell lines. Among them, the expression levels of TNFa and IFN-r of GPRC5D-positive cells of Clones 18, 39, and 41 were relatively high, and higher than that of Benchmark 1. At the same time, the expression of TNFa and IFN-r by these three clones on various positive target cells was relatively uniform, and Benchmark 1 has lower secretion of these two cytokines under U266 and H929 stimulation.

**Table 5 Percentage of cytokine IFNy-positive cells and TNFα cells in CAR-T cells after co-incubation of CAR-T cells with various target cells (8226-luc, U266-luc, MM1.s-luc, H929-luc, CCRF-luc, Raji-luc).**

| TNF-α (TNFα+/CAR+%) | MockT | 2290 (#18) | 2254 (benchmark1) | 2436 (#39) | 2438 (#41) |
|---|---|---|---|---|---|
| U266-luc (GPRC5D+) | 9.55 | 24.1 | 1.12 | 42.5 | 36.2 |
| 8226-luc (GPRC5D+) | 5.08 | 31.4% | 12 | 45 | 45.4 |
| H929-luc (GPRC5D+) | 4 | 29.1 | 5.21 | 51.6 | 44.8 |
| MM1.s-luc (GPRC5D+) | 5.73 | 26.5 | 17.7 | 38.5 | 39.2 |
| Raji-luc (GPRC5D-) | 11.3 | 6.07 | 2.66 | 4.45 | 2.2 |
| CCRF-luc (GPRC5D-) | 3.33 | 4.83 | 0.29 | 2.44 | 0.56 |
| Effector | 3.87 | 7.54 | 0.95 | 4.26 | 1.99 |

| IFN-γ (IFN+/CAR+%) | MockT | 2290 (#18) | 2254 (benchmark1) | 2436 (#39) | 2438 (#41) |
|---|---|---|---|---|---|
| U266-luc (GPRC5D+) | 2.51 | 35.3 | 5.25 | 49 | 29 |
| 8226-luc (GPRC5D+) | 2.03 | 41.4 | 25.1 | 54.8 | 39.6 |
| H929-luc (GPRC5D+) | 2 | 42 | 12.2 | 57.6 | 35.2 |
| MM1.s-luc (GPRC5D+) | 4.2 | 40 | 31.1 | 50.9 | 34.5 |
| Raji-luc (GPRC5D-) | 4.64 | 10.1 | 9.49 | 6.19 | 2.23 |
| CCRF-luc (GPRC5D-) | 0.83 | 9.49 | 0.45 | 4.3 | 0.97 |
| Effector | 1.94 | 14.3 | 1.4 | 6.36 | 1.5 |

### Example 11. Proliferation assay for repetitive stimulation

### Experimental purpose and principle:

Target cells 8226 treated with Mitomycin were mixed with different groups of GPRC5D CAR-T cells. After multiple stimulations, the CAR-T cells and target cells were co-incubated and cultured to determine the proliferation capacity of different CAR-T cells after multiple repetitive stimulations by target cells.

### Brief experimental steps:

1) Take 300 g of 8226 7×10⁶ cells, and centrifuge at a room temperature for 5 min.
2) Adjust the density to 0.2×10⁶ cells/mL with a complete medium, add 5 µL of Mitomycinstock solution (1 µg/µL), mix well, and then, culture for 24 h for later use.
3) Take 300 g of the 8226-Mitomycin cells after treatment for 24 h, centrifuge for changing the medium, wash with PBS 6 times, resuspend the 8226-Mitomycin cells with a CTS medium, count and adjust the density to 6×10⁶ cells/mL for later use.
4) Take 3×10⁵ CAR-T cells respectively, and transfer them into 24-well plates. Add 50 µL of 8226-Mitomycin cells to each well of CAR-T to make the effector-target ratio E:T = 1:1. Use a CTS complete medium to replenish the culture volume to 500 µL, mix well, culture at 37°C with 5% of CO2 for 96 h and count, treat target cells (CCRF-CEM) with Mitomycin again, repeat the above steps, and draw an amplification curve.

Main samples and reagents:
Mitomycin C, MCE, Cat. No. HY-113061; and
Target cell: 8226.

### Experimental results:

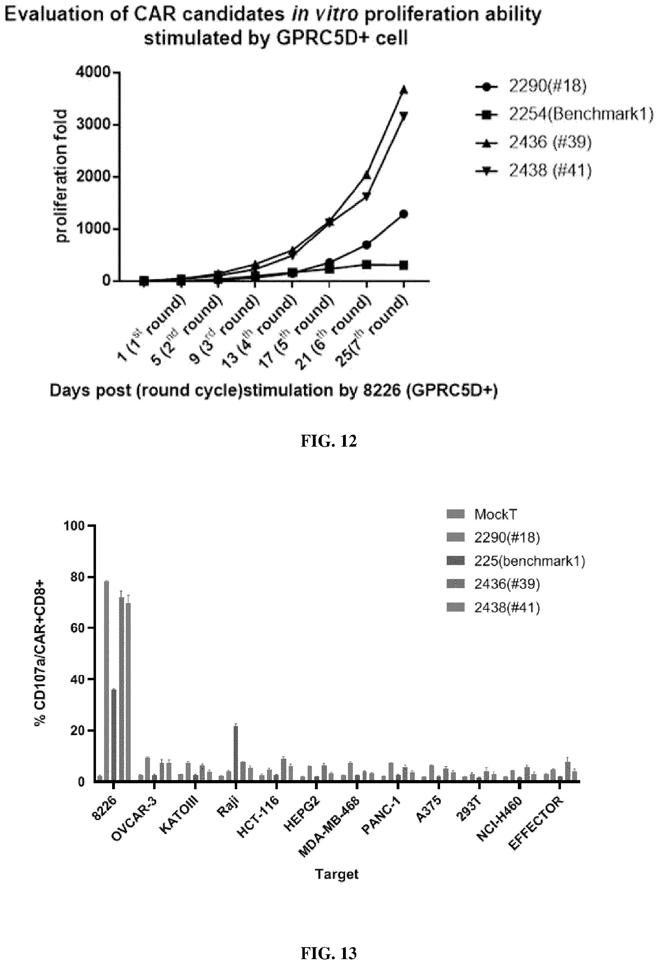

As shown in Table 6 and FIG. 12, four groups of CAR-T cell samples can be effectively amplified after repetitive stimulations. After target cells were stimulated 7 times, the amplification ability of Clone18, 39, and41 CAR-T cells was stronger than the amplification of Benchmark1. The proliferation ability of CAR-T cells after being stimulated by target cells was closely related to the long-term prognosis of patients. Therefore, CAR clones 2290 (#18), 2436 (#39), and 2438 (#41) were considered to have stronger potential for long-term proliferation and tumor cell elimination.

**Table 6 In vitro proliferation of various CAR-T cells after multiple stimulations of target cells (8226)**

| Proliferation fold | 1^{st} round stimulation | 2^{nd} round stimulation | 3^{rd} round stimulation | 4^{th} round stimulation | 5^{th} round stimulation | 6^{th} round stimulation | 7^{th} round stimulation |
|---|---|---|---|---|---|---|---|
| 2254 (Benchmark1) | 7.15 | 41.89 | 101.26 | 172.01 | 239.50 | 320.87 | 312.64 |
| 2290 (#18) | 6.66 | 31.30 | 70.12 | 157.76 | 356.70 | 698.61 | 1292.66 |
| 2436 (#39) | 10.12 | 51.17 | 146.44 | 329.99 | 594.86 | 1155.33 | 2045.32 |
| 2438 (#41) | 10.60 | 46.29 | 111.16 | 230.11 | 492.90 | 1114.22 | 1617.85 |

### Example 12. Determination of performance of organ-derived tumor cells to stimulate CAR cell degranulation

CD107a is a marker for intracellular microvesicles. CD107a on the cell membrane increases after granzyme-loaded microvesicles fuse with the cell membrane, and when its recovery is blocked by monesin (purchased from BioLegend), it can quantitatively reflect the strength of microvesicle release. When CAR-T cells are stimulated by target antigens on target cells, granzymes are released, and the activation of T cells by target cells can be determined by flow cytometric detection of the increase in CD107a.

Brief experimental steps of CD107a degranulation:
1) Centrifuge the CAR-T cells to be detected and target cells respectively at a room temperature and 300 g for 5 min, discard the supernatant, and then, resuspend the cells with a 1640 culture medium + 10%FBS to 2×10⁶ cells/mL;
2) Add 100 µL of the CAR-T cells to be detected and 100 µL of the target cells to 96-well plates respectively, and mix well;
3) Add 1.5 µL of PE/Cy7 anti-human CD107a antibody and 0.2 µL of monensin to each well of cells, then place in a cell incubator at 37°C, and incubate with 5% of CO2 for 4 h;
4) After completion of incubation, centrifuge at 4°C and 300 g for 5 min, then discard the supernatant, and wash the cells with 200 µL of PBS twice;
5) Resuspend the cells with 100 µL of PBS, add 1.5 µL of BV421 anti-human CD8 and 1.5 µL of APC anti-human EGFR antibodies respectively, mix well, and then, incubate on ice in the dark for 20 min; and
6) After completion of incubation, wash the cells with 200 µL of PBS 3 times, resuspend the cells with 100 µL of PBS, and then, detect by a flow cytometer.

### Main samples and reagents:

Target cells 8226, 293T, A375, HepG2, Nalm6, NCHI460, OVCAR-3, Raji, HCT116, Panc-1, MDA-MB-468, KATOIII;
Fetal bovine serum (FBS), Gibco, Cat. No.10099141;
Monensin, BioLegend, Cat. No. 420701;
PE/Cy7 mouse anti-human CD107a, BD, Cat. No. 561348;
BV421 mouse anti-human CD8, BD, Cat. No. 301036; and
APC anti-human EGFR Antibody, Clone AY13, BioLegend, Cat. No.35290.

### Experimental results:

CAR-T cells were obtained by lentiviral transduction, and the CAR-T cells were cultured in vitro for 9-12 days before a CD107a degranulation assay. The CAR-T cells to be detected were incubated with target cells, monensin and CD107a antibodies for 4 h, and the cell density of the CAR-T cells and target cells was both 2×10⁶ cells/mL. The samples were then labeled with CD8 antibodies and EGFR antibodies and then subjected to flow cytometric detection. By analysis in the Flowjo software, a live cell gate (P1) was selected in a scatter plot to remove cell debris; the cells in the P1 gate were analyzed, and a single scattered cell gate (P2)was selected; then, CD8-positive cells (P3) were further selected in the P2 gate; and finally, in the P3 gate, the proportion of CD107a-positive cells in EGFR antibody-stained positive cells (i.e., CAR-positive cells) was analyzed. The analysis results were as shown in FIG. 13. The degranulation level of the GPRC5D CAR-T of Clones 18, 39, and 41 was significantly increased only after co-incubation with GPRC5D-positive cells, while there was no significant increase in the degranulation level after co-incubation with cell lines of organ-derived tumor cells. This indicates that GPRC5D CAR-T cells were not easily activated by normal tissue and organ cells and have higher safety.

The above-mentioned in vitro experiments showed that the CAR-T cells of candidate clones 18, 39, and 41 can effectively recognize GPRC5D antigens on tumor cells and produce effective killing. In vitro negative cell verification showed no non-specific activation and killing activity, and the expected clinical safety was good. Repetitive stimulation assays showed that the three candidate clones can be efficiently amplified under in vitro target antigen stimulation, and have the potential to maintain long-term effective killing of tumor cells.

### Example 13 Anti-tumor efficacy assay on NCG tumor-bearing mice

To further verify the anti-tumor efficacy of the three candidate clones in mice, we expressed the firefly luciferase (ffLuc)-expressing tumor cell line H929-Luc and identified that it can normally express the firefly luciferase, while H929 itself is a natural cell line with high GPRC5D expression. H929-luc cells were inoculated into immunodeficient NCG mice via the tail vein. After tumors were formed in the mice, candidate clone CAR-T cells were inoculated to verify their anti-tumor effects.

### Brief test processes:

a) Establish a firefly luciferase (ffLuc) overexpressing cell line H929-luc, and identify that it can normally express the firefly luciferase.
b) After the above cells were cultured to the logarithmic growth phase, collect the cells.
c) Purchase 48 NCG mice, raise the mice for 1 week, inject H929-luc cells into the tail vein at 5×10⁶cells/200 µL of PBS per mouse, and observe the general conditions of the mice every day after the injection.
d) On the tenth day, divide the mice into groups, and inject CAR-T cells (4E+06 CAR+cells/mouse), control non-transfected Mock-T cells (2E+07 cells/mouse) and PBS buffer respectively into the tail vein.
e) Regularly observe images, weigh body weights, observe and record the general conditions of the mice, and regularly determine the proliferation of T cells in mouse blood.

### Experimental results:

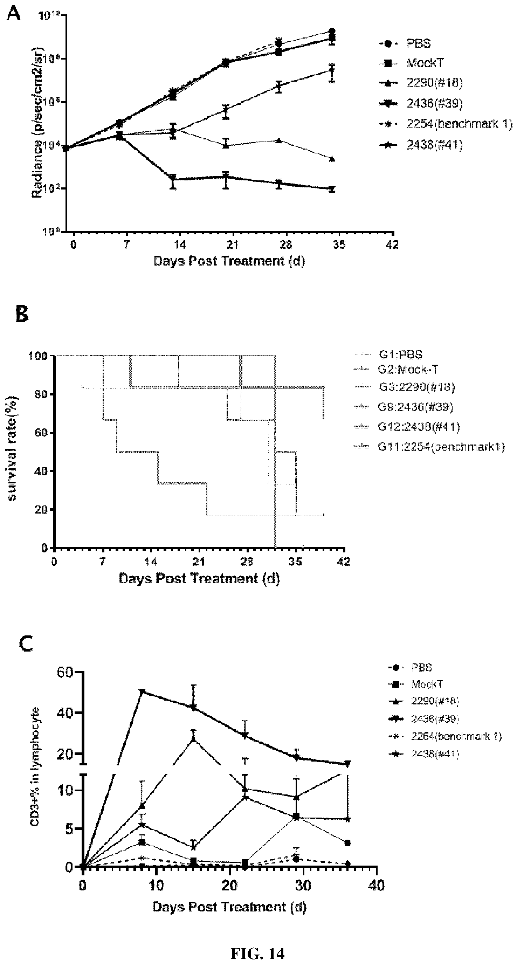

The experimental drug efficacy in the mice was as shown in FIG. 14. As shown in FIG. 14A , the tumor burden of the PBS, mockT, and benmark1 continues to increase after CAR cell reinfusion, and the tumor burden of Clone 41 was slightly lower than that of the PBS and mockT, but it has no obvious inhibiting effect on tumor growth. The tumor burden of Clones 39 and 18 can be significantly inhibited. However, Clone 18 mice gradually died 7 days after the reinfusion, indicating that it was highly toxic to mice and its expected clinical safety was poor. Only one mouse was left for endpoint photon number evaluation, so its inhibiting effect was unclear. Clone 39 has a significant tumor inhibiting effect and a high survival rate of mice (FIG. 14A and FIG. 14B). FIG. 14C shows proliferation conditions of T cells in mouse blood. The proliferation of mockT and bencmark1 T cells in vivo is not obvious. Clone 39 can maintain continuous and effective amplification in vivo, which is significantly better than Clone 41 and Clone 18. In summary, Clone 39 has the best anti-tumor efficacy in vivo and better safety in mice, so Clone 39 is a better candidate clone for later clinical development.

Nucleotide and amino acid sequences of the Benchmark1 antibody mentioned herein are as follows:
Nucleotide sequence (SEQ ID NO:40) of Benchmark1 GGATCCCAGGTGCAGCTGGTGCAGAGCGGCGCCGAGGTGAAGAAGCCCGGCGCCAGCGTGAA 
GGTGAGCTGCAAGGCCAGCGGCTACAGCTTCACCGGCTACACCATGAACTGGGTGCGCCAGGCCCC
CGGCCAGGGCCTGGAGTGGATGGGCCTGATCAACCCCTACAACAGCGACACCAACTACGCCCAGA
AGCTGCAGGGCCGCGTGACCATGACCACCGACACCAGCACCAGCACCGCCTACATGGAGCTGCGC
AGCCTGCGCAGCGACGACACCGCCGTGTACTACTGCGCCCGCGTGGCCCTGCGCGTGGCCCTGGAC
TACTGGGGCCAGGGCACCCTGGTGACCGTGAGCAGCGGCGGCGGCAGCGGCGGCGGCAG
CGGCGGCGGCGGCAGCGACATCCAGATGACCCAGAGCCCCAGCAGCCTGAGCGCCAGCGTGGGCG
ACCGCGTGACCATCACCTGCAAGGCCAGCCAGAACGTGGCCACCCACGTGGGCTGGTACCAGCAG
AAGCCCGGCAAGGCCCCCAAGCGCCTGATCTACAGCGCCAGCTACCGCTACAGCGGCGTGCCCAG
CCGCTTCAGCGGCAGCGGCAGCGGCACCGAGTTCACCCTGACCATCAGCAACCTGCAGCCCGAGG
ACTTCGCCACCTACTACTGCCAGCAGTACAACCGCTACCCCTACACCTTCGGCCAGGGCACCAAGC
TGGAGATCAAGTCTAGA
Amino acid sequence (SEQ ID NO:41) of Benchmark1

GSQVQLVQSGAEVKKPGASVKVSCKASGYSFTGYTMNWVRQAPGQGLEWMGLINPYNSDTNYA
QKLQGRVTMTTDTSTSTAYMELRSLRSDDTAVYYCARVALRVALDYWGQGTLVTVSSGGGGSGGGG
SGGGGSDIQMTQSPSSLSASVGDRVTITCKASQNVATHVGWYQQKPGKAPKRLIYSASYRYSGVPSRFS
GSGSGTEFTLTISNLQPEDFATYYCQQYNRYPYTFGQGTKLEIKSR

### References:

1. Shah U A, Mailankody S. Emerging immunotherapies in multiple myeloma. BMJ (online), 2020, 370:m3176
2. Soekojo C Y, Ooi M, Mel S D, et al. Immunotherapy in Multiple Myeloma. Cells, 2020, 9 (3):601.
3. Feng, D, Sun, J. Overview of anti-BCMA CAR-T immunotherapy for multiple myeloma and relapsed/refractory multiple myeloma. Scand J Immunol. 2020; 92: e12910.
4. https://ash.confex.com/ash/2022/webprogram/Paperl57988.html
5. https://www.fda.gov/drugs/resources-information-approved-drugs/fda-approves-teclistamab-cqyv-relapsed-or-refractory-multiple-myeloma
6. https://ash.confex.com/ash/2022/webprogram/Paper159707.html
7. Nikhil C. Munshi, Larry D. Anderson, Jr., Nina Sha et al. Idecabtagene Vicleucel in Relapsed and Refractory Multiple Myeloma. N Engl J Med 2021; 384:705-716
8. CARVYKTI™ Prescribing Information. Horsham, PA: Janssen Biotech, Inc.
9. Smith EL, et al. GPRC5D is a target for the immunotherapy of multiple myeloma with rationally designed CAR T cells. Sci Transl Med. 2019 Mar. 27;11(485)
10. Kodama T, Yu K, Nakai W, et al. Anti-GPRC5D/CD3 Bispecific T-Cell-Redirecting Antibody for the Treatment of Multiple Myeloma. Molecular Cancer Therapeutics, 2019, 18(9): molcanther.1216.2018.
11. Kodandaram Pillarisetti, et. al. A T-cell-redirecting bispecific G-protein-coupled receptor class 5 member D x CD3 antibody to treat multiple myeloma. Blood. 2020 Apr 9; 135(15): 1232-1243.
12. Sham Mailankody, et al. GPRC5D-Targeted CAR T Cells for Myeloma. N. Engl. J. Med. 2022;387:1196-206.

## Claims

1. A GPRC5D binding peptide, comprising a heavy chain variable region of an antibody molecule, wherein HCDR1, HCDR2 and HCDR3 in the heavy chain variable region are selected from one of the following combinations:
1) HCDR1 is GGSFSGYY (SEQ ID NO:1);
HCDR2 is INHSGST (SEQ ID NO:2);
the sequence of HCDR3 is ARARRYGGRTRFDP (SEQ ID NO:3);
2) HCDR1 is GFIFSSYG (SEQ ID NO:4);
the sequence of HCDR2 is ISSSGDYT (SEQ ID NO: 5);
the sequence of HCDR3 is ARMSFRRYDH (SEQ ID NO:6); and
3) HCDR1 is GFSFSGYI (SEQ ID NO:7);
the sequence of HCDR2 is TSSSGTET (SEQ ID NO: 8);
the sequence of HCDR3 is ARYYSKYGRSYHVDS (SEQ ID NO: 9).

2. The GPRC5D binding peptide of claim 1, wherein the GPRC5D binding peptide is an antibody molecule or an antigen binding fragment thereof.

3. The GPRC5D binding peptide of claim 1 or 2, wherein the heavy chain variable region comprises an amino acid sequence as shown in SEQ ID NO: 10, 11 or 12; or the heavy chain variable region comprises an amino acid sequence having at least 90%, 91%, 92%, 93%, 94%, 95%, 96%, 97%, 98%, or 99% sequence identity to the sequence as shown in SEQ ID NO: 10, 11 or 12 and is capable of specifically binding to GPRC5D.

4. The GPRC5D binding peptide of any one of claims 1-3, wherein the GPRC5D binding peptide is a single domain antibody molecule.

5. The GPRC5D binding peptide of any one of claims 1-4, wherein the GPRC5D binding peptide is a fully human antibody molecule.

6. The GPRC5D binding peptide of any one of claims 1-5, wherein the ability of the peptide to bind to cells expressing GPRC5D on the surface thereof is such that the EC50 value detected by FACS is not higher than 10 nM, 9 nM, 8 nM, 7 nM, 6 nM, or 5 nM.

7. A fusion protein comprising at least one of the GPRC5D binding peptide of any one of claims 1-6.

8. The fusion protein of claim 7, comprising at least two of the GPRC5D binding peptide according to any one of claims 1-6.

9. The fusion protein of any one of claims 7 or 8, wherein the HCDR1, HCDR2 and HCDR3 of the two GPRC5D binding peptides are selected from different combinations listed in claim 1, respectively.

10. The fusion protein of any one of claims 7-9, wherein
one of the two GPRC5D binding peptides comprises an amino acid sequence as shown in SEQ ID NO: 10, or comprises an amino acid sequence having at least 90%, 91%, 92%, 93%, 94%, 95%, 96%, 97%, 98%, or 99% sequence identity to the sequence as shown in SEQ ID NO: 10 and is capable of specifically binding to GPRC5D, and the other comprises an amino acid sequence as shown in SEQ ID NO:11, or comprises an amino acid sequence having at least 90%, 91%, 92%, 93%, 94%, 95%, 96%, 97%, 98%, or 99% sequence identity to the sequence as shown in SEQ ID NO:11 and is capable of specifically binding to GPRC5D;
one of the two GPRC5D binding peptides comprises an amino acid sequence as shown in SEQ ID NO: 10, or comprises an amino acid sequence having at least 90%, 91%, 92%, 93%, 94%, 95%, 96%, 97%, 98%, or 99% sequence identity to the sequence as shown in SEQ ID NO: 10 and is capable of specifically binding to GPRC5D, and the other comprises an amino acid sequence as shown in SEQ ID NO:12, or comprises an amino acid sequence having at least 90%, 91%, 92%, 93%, 94%, 95%, 96%, 97%, 98%, or 99% sequence identity to the sequence as shown in SEQ ID NO:12 and is capable of specifically binding to GPRC5D; or
one of the two GPRC5D binding peptides comprises an amino acid sequence as shown in SEQ ID NO:11, or comprises an amino acid sequence having at least 90%, 91%, 92%, 93%, 94%, 95%, 96%, 97%, 98%, or 99% sequence identity to the sequence as shown in SEQ ID NO:11 and is capable of specifically binding to GPRC5D, and the other comprises an amino acid sequence as shown in SEQ ID NO:12, or comprises an amino acid sequence having at least 90%, 91%, 92%, 93%, 94%, 95%, 96%, 97%, 98%, or 99% sequence identity to the sequence as shown in SEQ ID NO:12 and is capable of specifically binding to GPRC5D.

11. The fusion protein of any one of claims 7-10, wherein the fusion protein comprises at least three of the GPRC5D binding peptides, and the HCDR1, HCDR2 and HCDR3 of the three GPRC5D binding peptides are the three combinations listed in claim 1, respectively.

12. The fusion protein according to any one of claims 7-11, wherein the first of the three GPRC5D binding peptides of the fusion protein comprises an amino acid sequence as shown in SEQ ID NO: 10, or comprises an amino acid sequence having at least 90%, 91%, 92%, 93%, 94%, 95%, 96%, 97%, 98%, or 99% sequence identity to the sequence as shown in SEQ ID NO: 10 and is capable of specifically binding to GPRC5D; the second comprises an amino acid sequence as shown in SEQ ID NO:11, or comprises an amino acid sequence having at least 90%, 91%, 92%, 93%, 94%, 95%, 96%, 97%, 98%, or 99% sequence identity to the sequence as shown in SEQ ID NO:11 and is capable of specifically binding to GPRC5D; and the third comprises an amino acid sequence as shown in SEQ ID NO:12, or comprises an amino acid sequence having at least 90%, 91%, 92%, 93%, 94%, 95%, 96%, 97%, 98%, or 99% sequence identity to the sequence as shown in SEQ ID NO:12 and is capable of specifically binding to GPRC5D.

13. The fusion protein of any one of claims 7-12, wherein the fusion protein further comprises an Fc fragment.

14. The fusion protein of any one of claims 7-13, wherein the fusion protein further comprises another binding peptide capable of specifically binding to an antigen different from GPRC5D.

15. The fusion protein of any one of claims 7-14, wherein the another binding peptide is a single domain antibody or a single chain antibody.

16. The fusion protein of any one of claims 7-15, wherein the fusion protein further comprises a detectable tag or a purification tag.

17. The fusion protein of any one of claims 7-16, wherein the detectable tag has enzymatic activity.

18. A nucleic acid molecule encoding the GPRC5D binding peptide of any one of claims 1-6 or the fusion protein of any one of claims 7-17.

19. The nucleic acid molecule of claim 18, wherein the nucleic acid molecule comprises a nucleotide sequence as shown in SEQ ID NO:13, 14 or 15.

20. An expression vector comprising the nucleic acid molecule of claim 18 or 19.

21. A host cell, comprising the nucleic acid molecule of claim 18 or 19 or the expression vector of claim 20.

22. A multispecific antibody molecule, comprising at least a first functional portion and a second functional portion, wherein the first functional portion comprises the GPRC5D binding peptide of any one of claims 1-6; and the second functional portion has binding specificity different from that of the first functional portion.

23. The multispecific antibody molecule of claim 22, wherein the second functional portion has binding specificity for an immune cell.

24. The multispecific antibody molecule of claim 22 or 23, wherein the second functional portion has binding specificity for T cell.

25. The multispecific antibody molecule of any one of claims 22-24, wherein the second functional portion has binding specificity for CD3.

26. An immunoconjugate, comprising the GPRC5D binding peptide of any one of claims 1-6 linked to a therapeutic agent.

27. The immunoconjugate of claim 26, wherein the therapeutic agent is a drug.

28. The immunoconjugate of claim 26 or 27, wherein the therapeutic agent is a cytotoxin.

29. The immunoconjugate of any one of claims 26-28, wherein the therapeutic agent is a radioactive isotope.

30. A pharmaceutical composition, comprising:
1) the GPRC5D binding peptide of any one of claims 1-6;
the fusion protein of any one of claims 7-17;
the multispecific antibody molecule of any one of claims 22-25; or
the immunoconjugate of any one of claims 26-29; and
2) a pharmaceutically acceptable carrier.

31. Use of the GPRC5D binding peptide of any one of claims 1-6, the fusion protein of any one of claims 7-17, the multispecific antibody molecule of any one of claims 22-25, or the immunoconjugate of any one of claims 26-29 in the preparation of a drug for treating a GPRC5D-related disorder.

32. The use of claim 31, wherein the GPRC5D-related disorder is a cancer or an autoimmune disease, and the cancer is preferably a plasma cell malignant disease, such as multiple myeloma, or a B cell malignant disease, such as Hodgkin's lymphoma or non-Hodgkin's lymphoma.

33. A method for treating a GPRC5D-related disorder, comprising: administering an effective amount of the GPRC5D binding peptide of any one of claims 1-7, the fusion protein of any one of claims 7-17, the multispecific antibody molecule of any one of claims 22-25, the immunoconjugate of any one of claims 26-29, or the pharmaceutical composition of claim 30 to a subject in need thereof.

34. The method of claim 33, wherein the GPRC5D-related disorder is a cancer or an autoimmune disease.

35. The method of claim 34, wherein the cancer is a plasma cell malignant disease or a B cell malignant disease.

36. The method of claim 35, wherein the plasma cell malignant disease is multiple myeloma, and the B cell malignant disease is Hodgkin's lymphoma or non-Hodgkin's lymphoma.

37. A method for detecting the content of GPRC5D in a biological sample, comprising:
1) contacting the biological sample with the GPRC5D binding peptide of any one of claims 1-6, the fusion protein of any one of claims 7-17, or the multispecific antibody molecule of any one of claims 22-25, so as to form a binding complex with the GPRC5D; and
2) determining the content of GPRC5D in the biological sample based on the amount of the binding complex generated in step 1).

38. A method for detecting the content of GPRC5D in a biological sample, comprising:
1) coupling the GPRC5D binding peptide of any one of claims 1-6 to a detectable tag;
2) contacting the GPRC5D binding peptide carrying the detectable tag with the biological sample; and
3) determining the content of GPRC5D in the biological sample by detecting the amount of the detectable tag.

39. A detection kit, comprising the GPRC5D binding peptide of any one of claims 1-6, the fusion protein of any one of claims 7-17, or the multispecific antibody molecule of any one of claims 22-25.

40. A drug kit, comprising the GPRC5D binding peptide of any one of claims 1-6, the fusion protein of any one of claims 7-17, or the multispecific antibody molecule of any one of claims 22-25.

41. A method for identifying a GPRC5D-related disorder in a subject, comprising:
1) using the detection kit of claim 39 to determine the content of GPRC5D in a biological sample from the subject; and
2) comparing with the normal content of GPRC5D in a population to determine the presence or status of the GPRC5D-related disorder.

42. The method of claim 41, wherein the GPRC5D-related disorder is a cancer or an autoimmune disease.

43. The method of claim 42, wherein the cancer is a plasma cell malignant disease or a B cell malignant disease.

44. The method of claim 43, wherein the plasma cell malignant disease is multiple myeloma, and the B cell malignant disease is Hodgkin's lymphoma or non-Hodgkin's lymphoma.

45. A method for determining the therapeutic effect of a drug for treating a GPRC5D-related disorder in a subject, comprising:
1) using the method of any one of claims 42-44 to identify the status of the GPRC5D-related disorder in the subject;
2) treating the subject with the drug; and
3) repeating step 1) to determine the change in the status of the GPRC5D-related disorder in the subject, and determining the therapeutic effect of the drug based on the change.

46. The method of claim 45, wherein the GPRC5D-related disorder is a cancer or an autoimmune disease, and the cancer is preferably a plasma cell malignant disease, such as multiple myeloma, or a B cell malignant disease, such as Hodgkin's lymphoma or non-Hodgkin's lymphoma.

47. The method of claim 46, wherein the cancer is a plasma cell malignant disease or a B cell malignant disease.

48. The method of claim 47, wherein the plasma cell malignant disease is multiple myeloma, and the B cell malignant disease is Hodgkin's lymphoma or non-Hodgkin's lymphoma.

49. The pharmaceutical composition of claim 30 for use in treating a GPRC5D-related disorder.

50. A chimeric antigen receptor (CAR), of which extracellular antigen binding domain comprises one or more antibody molecule(s) or antigen binding fragment(s) thereof targeting GPRC5D, wherein HCDR1, HCDR2 and HCDR3 of the heavy chain variable region of the antibody molecule are selected from one of the following combinations:
1) the sequence of HCDR1 is GGSFSGYY (SEQ ID NO:1);
the sequence of HCDR2 is INHSGST (SEQ ID NO: 2);
the sequence of HCDR3 is ARARRYGGRTRFDP (SEQ ID NO:3);
2) the sequence of HCDR1 is GFIFSSYG (SEQ ID NO:4);
the sequence of HCDR2 is ISSSGDYT (SEQ ID NO: 5);
the sequence of HCDR3 is ARMSFRRYDH (SEQ ID NO:6); and
3) the sequence of HCDR1 is GFSFSGYI (SEQ ID NO:7);
the sequence of HCDR2 is TSSSGTET (SEQ ID NO: 8);
the sequence of HCDR3 is ARYYSKYGRSYHVDS (SEQ ID NO: 9).

51. The CAR of claim 50, wherein the extracellular antigen binding domain comprises two of the antibody molecules or antigen binding fragments thereof connected in series, and preferably, the two antibody molecules or antigen binding fragments thereof are connected by a linker peptide.

52. The CAR of claim 51, wherein the two antibody molecules or antigen binding fragments thereof are the same or different.

53. The CAR of any one of claims 50-52, wherein the antibody molecule is a single domain antibody, and preferably, the antibody molecule is a fully human single domain antibody.

54. The CAR of any one of claims 50-53, wherein the heavy chain variable region comprises an amino acid sequence as shown in SEQ ID NO: 10, 11 or 12; or the heavy chain variable region comprises an amino acid sequence having at least 90%, 91%, 92%, 93%, 94%, 95%, 96%, 97%, 98%, or 99% sequence identity to the sequence as shown in SEQ ID NO: 10, 11 or 12 and is capable of specifically binding to GPRC5D.

55. The CAR of any one of claims 50-54, wherein the CAR comprises the extracellular antigen binding domain, a transmembrane domain and an intracellular signaling domain sequentially from N-terminus to C-terminus.

56. The CAR of any one of claims 50-55, wherein the CAR further comprises a signal peptide at the N-terminus.

57. The CAR of any one of claims 50-56, wherein the signal peptide comprises an amino acid sequence as shown in SEQ ID NO:17.

58. The CAR of any one of claims 50-57, wherein the CAR further comprises a hinge region between the extracellular antigen binding domain and the transmembrane domain.

59. The CAR of any one of claims 50-58, wherein the hinge region comprises an amino acid sequence as shown in SEQ ID NO:19.

60. The CAR of any one of claims 50-59, wherein the transmembrane domain comprises an amino acid sequence as shown in SEQ ID NO:21.

61. The CAR of any one of claims 50-60, wherein the intracellular signaling domain comprises a 4-1BB intracellular domain and a CD3ζ intracellular domain.

62. The CAR of claims 50-61, wherein the 4-1BB intracellular domain comprises an amino acid sequence as shown in SEQ ID NO: 23, and/or the CD3ζ intracellular domain comprises an amino acid sequence as shown in SEQ ID NO: 25.

63. The CAR of any one of claims 50-62, wherein the CAR has an amino acid sequence as shown in SEQ ID NO: 35, 37 or 39; or the amino acid sequence of the CAR has at least 90%, 91%, 92%, 93%, 94%, 95%, 96%, 97%, 98%, or 99% sequence identity to the sequence as shown in SEQ ID NO: 35, 37 or 39.

64. The CAR of any one of claims 50-63, wherein the end of the CAR is further linked to a safety switch for CAR-T cell clearance; preferably, the safety switch is linked to the C-terminus of the CAR; and more preferably, the safety switch comprises a truncated EGFR molecule (tEGFR) or a fusion protein with a suicide capability.

65. The CAR of any one of claims 50-64, wherein the C-terminus of the CAR is further linked to a self-cleaving polypeptide and tEGFR; preferably, a CSF2RA signal peptide is further linked to the N-terminus of the tEGFR; and more preferably, the tEGFR comprises an amino acid sequence as shown in SEQ ID NO: 31, and/or the CSF2RA signal peptide comprises an amino acid sequence as shown in SEQ ID NO: 29.

66. The CAR of any one of claims 50-65, wherein the safety switch is linked to the CAR via a self-cleaving polypeptide, and preferably, the self-cleaving polypeptide comprises an amino acid sequence as shown in SEQ ID NO: 27 or 33.

67. The CAR according to any one of claims 50-66, wherein the CAR is a bispecific CAR, the extracellular antigen binding domain further comprises a second antibody molecule or an antigen binding fragment thereof targeting a second target, and the second target is selected from CD3, BCMA and a combination thereof.

68. An isolated nucleic acid molecule, encoding the CAR of any one of claims 50-67.

69. The nucleic acid molecule of claim 68, wherein the nucleic acid molecule comprises a nucleotide sequence as shown in any one of SEQ ID NOs: 13, 14, 15, 16, 18, 20, 22, 24, 26, 28, 30, 32, 34, 36, and 38.

70. An expression vector, comprising the nucleic acid molecule of claim 69.

71. The expression vector of claim 70, being selected from a plasmid, a retroviral vector and a lentiviral vector.

72. A host cell, comprising the CAR of any one of claims 50-67, the nucleic acid molecule of claim 68 or 69, or the expression vector of claim 70 or 71.

73. An engineered immune cell, expressing the CAR of any one of claims 50-67.

74. The engineered immune cell of claim 73, being an immune cell, preferably an autologous immune cell or an allogeneic immune cell, and more preferably, the immune cell is a T cell or a NK cell.

75. A pharmaceutical composition, comprising the cell of any one of claims 72-74 and a pharmaceutically acceptable carrier.

76. Use of the CAR of any one of claims 50-67, the nucleic acid molecule of claim 68 or 69, the expression vector of claim 70 or 71, or the cell of any one of claims 72-74 in the preparation of a drug for preventing or treating a GPRC5D-related disease.

77. The use of claim 76, wherein the GPRC5D-related disease is a cancer or an autoimmune disease, and the cancer is preferably a plasma cell malignant disease, such as multiple myeloma, or a B cell malignant disease, such as Hodgkin's lymphoma or non-Hodgkin's lymphoma.

78. A method for preventing or treating a GPRC5D-related disease, comprising: administering a therapeutically effective amount of the cell of any one of claims 72-74, or the pharmaceutical composition of claim 75 to a subject in need thereof.

79. The method of claim 78, further comprising: administering an EGFR antibody to the subject to inhibit the activity of the immune effector cell or the pharmaceutical composition.

80. The method of claim 78 or 79, wherein the GPRC5D-related disease is a cancer or an autoimmune disease, and the cancer is preferably a plasma cell malignant disease, such as multiple myeloma, or a B cell malignant disease, such as Hodgkin's lymphoma or non-Hodgkin's lymphoma.
